# EUROPEAN PATENT APPLICATION

(11) **EP 4 339 211 A1**
(43) Date of publication of application: **20.03.2024**
(21) Application number: 22807908.3
(22) Date of filing: 13.05.2022
(51) Int. Cl.: C07K 16/28, A61P 35/00, G01N 33/574, G01N 33/68, A61K 45/06, A61K 39/00

(54) **ANTI-CD300C MONOCLONAL ANTIBODY, AND BIOMARKER THEREOF FOR PREVENTING OR TREATING CANCER**

(30) Priority: 13.05.2021 KR 20210062311; 13.05.2021 KR 20210062312; 13.05.2021 KR 20210062313; 27.08.2021 KR 20210114297; 06.04.2022 KR 20220042680
(71) Applicant: CentricsBio, Inc., Seoul 05836 (KR)
(72) Inventor: JEON, Jae-Won, Suwon-si, Gyeonggi-do 16512 (KR); LEE, Suin, Gunpo-si, Gyeonggi-do 15801 (KR); KIM, Haneul, Namyangju-si, Gyeonggi-do 12221 (KR); LIM, Chang Ki, Hwaseong-si, Gyeonggi-do 18496 (KR)
(74) Representative: Regimbeau
(86) International application number: PCT/KR2022/006939
(87) International publication number: WO 2022/240261

(57) **Abstract**

The present disclosure relates to an anti-CD300c monoclonal antibody and its use for preventing or treating cancer. The anti-CD300c monoclonal antibody according to the present disclosure binds to a CD300c antigen with high specificity and also promotes anticancer immune responses. Thus, this anti-CD300c monoclonal antibody is expected to be effectively used against growth, development, metastasis, and the like of various cancers.

## Description

### [Technical Field]

The present disclosure relates to an anti-CD300c antibody or an antigen-binding fragment thereof, and a biomarker, a composition, a method, and a kit for preventing or treating cancer, each of which comprises the same.

### [Background Art]

Cancer is one of the diseases that account for the largest share of the causes of death in modern people. This disease is caused by changes in normal cells due to genetic mutations that result from various causes, and refers to a malignant tumor that does not follow differentiation, proliferation, growth pattern, and the like of normal cells. Cancer is characterized by "uncontrolled cell growth". This abnormal cell growth causes formation of a mass of cells called a tumor, which infiltrates the surrounding tissues and, in severe cases, may metastasize to other organs of the body. Cancer is an intractable chronic disease that is not fundamentally cured in many cases even if it is treated with surgery, radiotherapy, chemotherapy, and the like, causes pain to patients, and ultimately leads to death. In particular, in recent years, the global cancer incidence rate is increasing by 5% or higher every year due to increased elderly population, environmental deterioration, and the like. According to the WHO report, it is estimated that within the next 25 years the number of cancer patients will increase to 30 million, of which 20 million will die from cancer.

Cancer drug treatments, that is, cancer chemotherapies, are generally cytotoxic compounds, and treat cancer by attacking and killing cancer cells. However, these chemotherapies exhibit high adverse effects since they damage not only cancer cells but also normal cells. Thus, targeted cancer chemotherapies have been developed to decrease adverse effects. These targeted cancer chemotherapies were able to exhibit decreased adverse effects, but had a limitation in that resistance occurs with a high probability. Therefore, in recent years, interest in cancer immunotherapies, which use the body's immune system to decrease problems due to toxicity and resistance, is rapidly increasing. As an example of such cancer immunotherapies, immune checkpoint inhibitors have been developed which specifically bind to PD-L1 on the surface of cancer cells and inhibit its binding to PD-1 on T cells so that T cells are activated and attack cancer cells. However, even these immune checkpoint inhibitors are not effective in various types of cancer. Therefore, there is a need to develop novel cancer immune therapeutics that exhibit an equivalent therapeutic effect in various cancers.

### [PRIOR ART LITERATURE]

### [Patent Literature]

(Patent Literature 1) Korean Patent Laid-Open Publication No. 10-2018-0099557

### [Disclosure]

### [Technical Problem]

An object of the present disclosure is to solve all of the above-mentioned problems.

One object of the present disclosure is to provide an anti-CD300c antibody for preventing or treating cancer.

Another object of the present disclosure is to provide an anticancer therapy using an anti-CD300c antibody.

Yet another object of the present disclosure is to provide a pharmaceutical composition for therapy using an anti-CD300c antibody for preventing or treating cancer.

Still yet another object of the present disclosure is to provide a method for preventing or treating cancer which uses an anti-CD300c antibody.

Still another object of the present disclosure is to provide a kit for therapy for using an anti-CD300c antibody for preventing or treating cancer.

The object of the present disclosure is not limited to the objects as mentioned above. The object of the present disclosure will become clearer from the following description, and will be realized by the means as described in the claims and combinations thereof.

### [Technical Solution]

Representative configurations of the present disclosure for achieving the above-mentioned objects are as follows.

According to an aspect of the present disclosure, there is provided an antibody (for example, monoclonal antibody) or an antigen-binding fragment thereof which specifically binds to CD300c.

According to another aspect of the present disclosure, there are provided an anti-CD300c antibody or an antigen-binding fragment thereof, and a use thereof for preventing or treating cancer.

According to yet another aspect of the present disclosure, there is provided a use of an anti-CD300c antibody or an antigen-binding fragment thereof for the manufacture of a medicament for preventing or treating cancer.

According to still yet another aspect of the present disclosure, there is provided an anticancer therapy, comprising, as an active ingredient, an anti-CD300c antibody or an antigen-binding fragment thereof.

According to still yet another aspect of the present disclosure, there is provided a pharmaceutical composition for preventing or treating cancer, comprising, as an active ingredient, an anti-CD300c antibody or an antigen-binding fragment thereof.

According to still yet another aspect of the present disclosure, there is provided a method for preventing or treating cancer, comprising administering an anti-CD300c antibody or an antigen-binding fragment thereof to a subject in need thereof.

According to still yet another aspect of the present disclosure, there is provided a kit for preventing or treating cancer, comprising a composition, which comprises an effective amount of an anti-CD300c antibody or an antigen-binding fragment thereof, and an instruction on how to use the antibody or an antigen-binding fragment thereof.

### [Advantageous Effects]

The anti-CD300c monoclonal antibody according to the present disclosure specifically binds, with high binding affinity, to CD300c expressed on the surface of various cancers, which activates T cells and at the same time promotes differentiation into M1 macrophages so that proliferation of cancer cells can be effectively inhibited. Thus, the anti-CD300c monoclonal antibody can be effectively used as an immunotherapy for various cancers. In addition, the anti-CD300c monoclonal antibody according to the present disclosure can exhibit a further increased therapeutic effect through co-administration with a conventional cancer immunotherapy, and also has inter-species cross-reactivity that allows the antibody to be widely applied to various mammals. In addition, it is expected that in a case where resistant cancer cells showing the ability to resist apoptosis are treated with the anti-CD300c monoclonal antibody of the present disclosure, this antibody is able to remarkably weaken resistance of the cancer cells, thereby showing excellent efficacy in preventing cancer recurrence. In addition, in general, cancer cells inhibit production of the pro-inflammatory cytokine IL-2 to evade the immune system. It was identified that the anti-CD300c monoclonal antibody activates the immune system by restoring production of IL-2 blocked by these cancer cells, which induces cancer cell death. Thus, it is expected that the anti-CD300c monoclonal antibody can be utilized as a more fundamental cancer immunotherapy.

### [Brief Description of Drawings]

FIGS. 1a to 1y respectively illustrate heavy chain and light chain variable region sequences (nucleotide and amino acid sequences) of 25 anti-CD300c monoclonal antibodies according to the present disclosure. In each drawing, the CDR regions (CDR1, CDR2, and CDR3) are sequentially indicated.

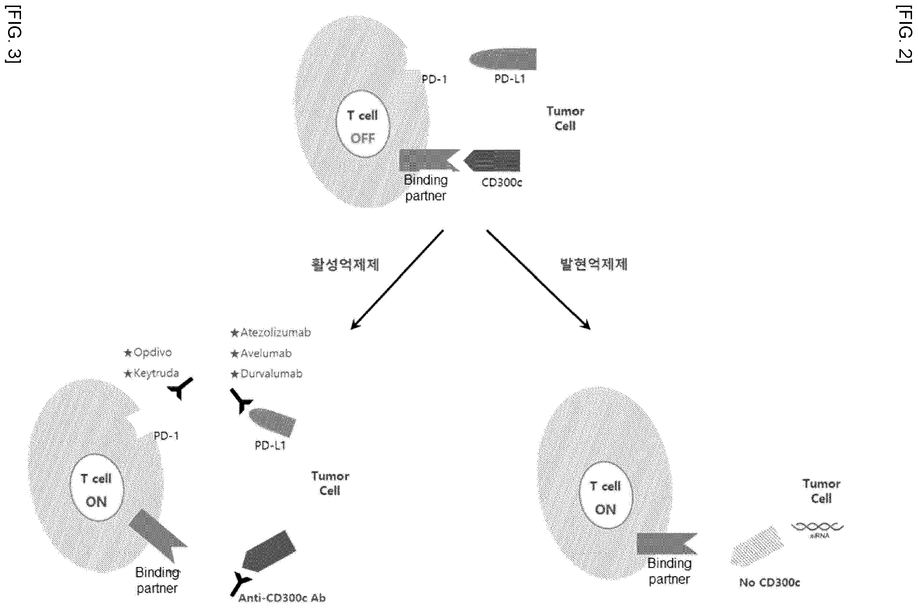

FIG. 2 illustrates a schematic diagram, briefly showing the mechanism by which the anti-CD300c monoclonal antibody and/or CD300c siRNA of the present disclosure exhibits an anticancer effect.
FIG. 3 illustrates a schematic diagram, briefly showing the mechanism by which the anti-CD300c monoclonal antibody of the present disclosure acts on monocytes, T cells, and cancer cells, respectively.
FIG. 4 illustrates results obtained by performing SDS-PAGE on the anti-CD300c monoclonal antibodies under a non-reducing condition, according to Example 1.4.
FIG. 5 illustrates results obtained by performing SDS-PAGE on the anti-CD300c monoclonal antibodies under a reducing condition, according to Example 1.4.
FIG. 6 illustrates results obtained by comparing the expression of CD300c in normal cells, immune cells, and a cancer cell line, according to Experimental Example 1.1.
FIGS. 7a and 7b illustrate results obtained by identifying the expression of CD300c in cancer tissue (FIG. 7a) and immune cells (FIG. 7b), according to Experimental Example 1.2.
FIGS. 8a and 8b illustrate results obtained by identifying the expression of CD300c in tonsil tissue (FIG. 8a) and cancer tissue (FIG. 8b), according to Experimental Example 1.3.
FIG. 9 illustrates results obtained by identifying the binding affinity, to a CD300c antigen, of the anti-CD300c monoclonal antibody, according to Experimental Example 2.1.
FIG. 10 illustrates sigmoidal curves according to the results of FACS binding in Experimental Example 2.2.
FIG. 11 illustrates results of the binding ELISA, according to Experimental Example 2.3.
FIG. 12 illustrates results of the surface plasmon resonance, according to Experimental Example 2.4.
FIG. 13 illustrates results of the binding ELISA, according to Experimental Example 2.5.
FIG. 14 illustrates results of the binding ELISA, according to Experimental Example 2.6.
FIG. 15 illustrates results obtained by comparing overall survival depending on the CD300c expression level in various cancer patients, in connection with Experimental Example 2.7.
FIG. 16 illustrates results obtained by identifying an anticancer effect of the anti-CD300c monoclonal antibody through T cell activation, according to Experimental Example 3.1.
FIGS. 17 and 18 illustrate results obtained by identifying the effect of the anti-CD300c monoclonal antibody on differentiation into M1 macrophages, according to Experimental Example 3.2.
FIGS. 19 and 20 illustrate results obtained by identifying the concentration-dependent effect of the anti-CD300c monoclonal antibody on differentiation into M1 macrophages, according to Experimental Example 3.3.
FIG. 21 illustrates results obtained by identifying the effect of the anti-CD300c monoclonal antibody on differentiation into M1 macrophages, according to Experimental Example 3.4.
FIG. 22 illustrates results obtained by identifying again whether the anti-CD300c monoclonal antibody promotes differentiation of human monocytes into M1 macrophages, according to Experimental Example 3.5.
FIGS. 23 to 25 illustrate results obtained by identifying whether the anti-CD300c monoclonal antibody can induce re-differentiation from M2 macrophages into M1 macrophages, according to Experimental Example 3.6.
FIG. 26 illustrates results obtained by identifying capacity of the anti-CD300c monoclonal antibody for causing differentiation and redifferentiation(repolarization) into M1 macrophages, according to Experimental Example 3.7.
FIGS. 27 and 28 illustrate results obtained by identifying effects of the monoclonal antibodies, which target CD300c, on growth of cancer cells, according to Experimental Example 4.1.
FIG. 29 illustrates results obtained by identifying cancer cell growth inhibitory effects of the anti-CD300c monoclonal antibody depending on its concentrations, according to Experimental Example 4.2.
FIG. 30 illustrates results obtained by identifying results obtained by identifying whether the anti-CD300c monoclonal antibody can promote differentiation from mouse macrophages into M1 macrophages, according to Experimental Example 4.3.
FIG. 31 illustrates results obtained by identifying whether the anti-CD300c monoclonal antibody exhibits an anticancer effect, according to Experimental Example 4.4.
FIGS. 32 to 35 illustrate results obtained by comparing capacity for causing differentiation into M1 macrophages between the anti-CD300c monoclonal antibody and conventional cancer immunotherapies, according to Experimental Example 5.1.
FIG. 36 illustrates results obtained by comparing capacity for causing differentiation from M0 macrophages into M1 macrophages between the anti-CD300c monoclonal antibody and a conventional cancer immunotherapy, according to Experimental Example 5.2.
FIG. 37 illustrates results obtained by comparing capacity for causing differentiation into M1 macrophages between the anti-CD300c monoclonal antibody and a conventional cancer immunotherapy, according to Experimental Example 5.3.
FIGS. 38 and 39 illustrate results obtained by comparing cancer cell growth inhibitory effects between the anti-CD300c monoclonal antibody and a conventional cancer immunotherapy, according to Experimental Example 5.4.
FIG. 40 illustrates results obtained by identifying effects *in vivo* of the anti-CD300c monoclonal antibody on tumor-associated macrophages, according to Experimental Example 6.1.
FIG. 41 illustrates results obtained by identifying effects *in vivo* of the anti-CD300c monoclonal antibody on CD8+ T cells, according to Experimental Example 6.2.
FIG. 42 illustrates results obtained by identifying whether the anti-CD300c monoclonal antibody increases the number of CD8+ T cells in a tumor-specific manner, according to Experimental Example 6.3.
FIG. 43 illustrates results obtained by identifying effects *in vivo* of the anti-CD300c monoclonal antibody on an increase in activity of CD8+ T cells, according to Experimental Example 6.4.
FIG. 44 illustrates results obtained by identifying effects *in vivo* of the anti-CD300c monoclonal antibody on an increase of cytotoxic T cells relative to regulatory T cells, according to Experimental Example 6.5.
FIG. 45 illustrates results obtained by identifying effects of the anti-CD300c monoclonal antibody on cytotoxic T cells, regulatory T cells, and tumor-associated macrophages, according to Experimental Example 6.6.
FIG. 46 illustrates results obtained by identifying anticancer effects *in vivo* of the anti-CD300c monoclonal antibody, according to Experimental Example 6.7.
FIG. 47 illustrates Nanostring Immune profiling results obtained in a case where a solid cancer model was treated with CL7, according to Example 2.1.
FIG. 48 illustrates changes in expression of various immune cell- and tumor microenvironment-related markers obtained in a case where a solid cancer model was treated with CL7, according to Example 2.1. * indicates a marker whose expression level was statistically significantly changed as compared with before treatment with CL7.
FIG. 49 illustrates changes in expression of immune checkpoint markers identified based on the Nanostring Immune profiling results obtained in Example 2.1, according to Example 2.2. * indicates a marker whose expression level was statistically significantly changed as compared with before treatment with CL7.
FIG. 50 illustrates results of differentiation of monocytes into M1 macrophages (whether or not M1 macrophages are increased) by treatment with the anti-CD300c monoclonal antibody and a cancer immunotherapy alone or in combination, according to Experimental Example 7.1.
FIG. 51 illustrates results of differentiation of monocytes into M1 macrophages (which indicate whether or not M1 macrophage markers increase) by treatment with the anti-CD300c monoclonal antibody, according to Experimental Example 7.2.
FIG. 52 illustrates results of differentiation of monocytes into M1 macrophages (which indicates whether or not M1 macrophage markers increase) by co-treatment with the anti-CD300c monoclonal antibody and a cancer immunotherapy, according to Experimental Example 7.2.
FIGS. 53 to 55 illustrate results obtained by identifying signal transduction of MAPK (FIG. 53), NF-kB (FIG. 54), and IkB (FIG. 55), which are signals of M1 macrophage differentiation, caused by co-treatment with the anti-CD300c monoclonal antibody and a cancer immunotherapy, according to Experimental Example 7.4.
FIG. 56 illustrates results obtained by identifying changes in apoptosis signal caused by co-treatment with the anti-CD300c monoclonal antibody and a cancer immunotherapy, according to Experimental Example 8.1.
FIGS. 57 and 58 illustrate results obtained by identifying cancer cell growth inhibitory effects caused by co-treatment with the anti-CD300c monoclonal antibody and a cancer immunotherapy, according to Experimental Example 8.2.
FIG. 59 schematically illustrates the experimental method used in Experimental Example 9.1.
FIG. 60 illustrates cancer growth inhibitory effects *in vivo* observed in a case where mice transplanted with a colorectal cancer cell line were administered with the anti-CD300c monoclonal antibody and an anti-PD-1 antibody alone or in combination, according to Experimental Example 9.1.
FIG. 61 illustrates results obtained by identifying whether the anti-CD300c monoclonal antibody increases M1 macrophages in cancer tissues of a mouse model, according to Experimental Example 9.3.
FIG. 62 illustrates results obtained by identifying whether the anti-CD300c monoclonal antibody promotes CD8+ T cell immunity in a mouse tumor model, according to Experimental Example 9.4.
FIG. 63 schematically illustrates the experimental method used in Experimental Example 10.1.
FIG. 64 illustrates results obtained by identifying whether the anti-CD300c monoclonal antibody is effective in other carcinomas in addition to a CT26 colorectal cancer mouse model, according to Experimental Example 10.1.
FIG. 65 illustrates results obtained by identifying effects *in vivo,* of administration of the anti-CD300c monoclonal antibody and (a) cancer immunotherapy(ies) alone or in combination (including double and triple co-administration), on CD8+ T cells in a B16F10 melanoma model, according to Experimental Example 10.2.
FIG. 66 illustrates results obtained by identifying effects *in vivo,* of the anti-CD300c monoclonal antibody and (a) cancer immunotherapy(ies) alone or in combination (including double and triple co-administration), on regulatory T cells in a B16F10 melanoma model, according to Experimental Example 10.3.
FIG. 67 illustrates results obtained by identifying effects *in vivo,* of the anti-CD300c monoclonal antibody and (a) cancer immunotherapy(ies) alone or in combination (including double and triple co-administration), on macrophages in a B16F10 melanoma model, according to Experimental Example 10.4.
FIGS. 68a and 68b illustrate results obtained by identifying anticancer effects in *vivo* caused by co-administration of the anti-CD300c monoclonal antibody and (a) cancer immunotherapy(ies), according to Experimental Example 11. FIG. 68a illustrates tumor volume decrease rates, and FIG. 68b illustrates complete remission rates.
FIG. 69 illustrates results obtained by identifying effects of improving a long-term survival rate caused by co-administration of the anti-CD300c monoclonal antibody and (a) cancer immunotherapy(ies), according to Experimental Example 12.
FIG. 70 illustrates results obtained by identifying effects *in vivo* of preventing cancer recurrence caused by co-administration of the anti-CD300c monoclonal antibody and a cancer immunotherapy, according to Experimental Example 13.
FIG. 71 illustrates results obtained by identifying immune memory effects caused by co-administration of the anti-CD300c monoclonal antibody and a cancer immunotherapy, according to Experimental Example 14.
FIGS. 72a and 72b illustrate results obtained by identifying whether administration of the anti-CD300c monoclonal antibody and (a) cancer immunotherapy(ies) alone or in combination (including double and triple co-administration) can promote differentiation from monocytes to M1 macrophages, according to Experimental Example 15.
FIGS. 73a and 73b illustrate results obtained by identifying whether the anti-CD300c monoclonal antibody can inhibit cancer cell growth by co-administration with a cancer immunotherapy, according to Experimental Example 16.

### [Best Mode for Carrying Out the Invention]

The following detailed description of the present disclosure will be described, with reference to specific drawings, for specific embodiments in which the present disclosure may be practiced. However, the present disclosure is not limited thereto, and the scope of the present disclosure is defined only by the appended claims, appropriately interpreted, along with the full range of equivalents to which the claims are entitled. It is to be understood that the various embodiments of the present disclosure, although different from each other, are not necessarily mutually exclusive. For example, a particular feature, structure, or characteristic described herein may vary from one embodiment to another or be implemented as a combination of embodiments without departing from the spirit and scope of the present disclosure. Technical and scientific terms used herein have the same meanings as commonly used in the art to which the present disclosure belongs, unless otherwise defined. For the purpose of interpreting this specification, the following definitions will apply, and the singular forms "a," "an," and "the" include plural referents and *vice versa* unless the context clearly dictates otherwise.

### Definition

As used herein, the term "about" means within an acceptable error range for each value which is known to one of ordinary skill in the art.

The term "antibody" is used broadly and includes monoclonal antibodies (including full length antibodies) of any isotype such as IgG, IgM, IgA, IgD, and IgE, polyclonal antibodies, multispecific antibodies (for example, bispecific antibodies), antibody fusions (for example, a fusion of an antibody with a (poly)peptide or a fusion of an antibody with a compound), and antibody fragments (including antigen-binding fragments). As used herein, the prefix "anti-", when in conjunction with an antigen, indicates that the given antibody is reactive with the given antigen. An antibody reactive with a specific antigen can be generated, without limitation, by synthetic and/or recombinant methods such as selection of libraries of recombinant antibodies in phage or similar vectors, or by immunizing an animal with the antigen or an antigen-encoding nucleic acid. A typical IgG antibody is comprised of two identical heavy chains and two identical light chains that are joined by disulfide bonds. Each heavy and light chain contains a constant region and a variable region. Heavy chain variable regions (HVRs) and light chain variable regions (LVRs) contain three segments, referred to as "complementarity determining regions" ("CDRs") or "hypervariable regions", respectively, which are primarily responsible for binding an epitope of an antigen. They are usually referred to as CDR1, CDR2, and CDR3, numbered sequentially from the N-terminus. The more highly conserved portions of the variable regions outside of the CDRs are called the "framework regions" ("FRs"). An antibody herein may be, for example, an animal antibody, a chimeric antibody, a humanized antibody, or a human antibody.

The term "humanization (also called reshaping or CDR-grafting) includes a well-established technique for reducing the immunogenicity of monoclonal antibodies from xenogeneic sources (commonly rodent) and for improving their affinity or effector function (ADCC, complement activation, C1q binding).

The term "monoclonal antibody" as used herein refers to an antibody obtained from a population of substantially homogeneous antibodies, that is, the individual antibodies comprising the population are identical except for possible naturally occurring mutations and/or post-translation modifications (for example, isomerizations, amidations) that may be present in minor amounts. Monoclonal antibodies are highly specific, being directed against a single antigenic site. The monoclonal antibody is obtained from a substantially homogeneous population of antibodies, displays the nature of an antibody, and is not to be construed as requiring production of the antibody by any particular method. For example, monoclonal antibodies to be used in accordance with the present disclosure may be made by a variety of techniques, including but not limited to the hybridoma method, recombinant DNA methods, phage-display methods, and methods utilizing transgenic animals containing all or part of the human immunoglobulin loci.

The term "antigen-binding fragment" refers to a portion of an antibody having specific binding ability to an antigen or a polypeptide comprising the same. The terms "antibody" and "antigen-binding fragment" may be used interchangeably except for a case where it is understood in the context that the "antibody" specifically excludes the "antigen-binding fragment," and the "antibody" may be interpreted as including the "antigen-binding fragment." Examples of the antigen-binding fragment include, but are not limited to, Fv, Fab, Fab', Fab'-SH, F(ab')2, diabodies, triabodies, tetrabodies, cross-Fab fragments, linear antibodies, single chain antibody molecules (for example, scFv), and multispecific antibodies formed of antibody fragments and single domain antibodies.

The term "cancer therapy" collectively refers to known agents used in conventional cancer treatment which act on various metabolic pathways of cells and exhibit cytotoxic or cytostatic effects on cancer cells. The cancer therapy includes chemotherapies, targeted chemotherapies, and immunotherapies.

The term "immunotherapy" (also referred to as "cancer immunotherapy") refers to a cancer therapy or an anticancer agent which activates immune cells to kill cancer cells.

The term "subject" is used interchangeably with "patient" and may be a mammal who is in need of prevention or treatment of cancer, such as primates (for example, humans), companion animals (for example, dogs and cats), livestock (for example, cows, pigs, horses, sheep, and goats), and laboratory animals (for example, rats, mice, and guinea pigs). In an embodiment of the present disclosure, the subject is a human.

The term "treatment" generally means obtaining a desired pharmacological and/or physiological effect. The effect may be therapeutic in terms of partially or completely curing a disease and/or adverse effect attributed to the disease. Desirable therapeutic effects include, but are not limited to, prevention of onset or recurrence of disease, alleviation of symptoms, diminishment of any direct or indirect pathological consequences of the disease, prevention of metastasis, decreasing the rate of disease progression, amelioration or slowing of the disease state, and remission or improved prognosis. Preferably, the "treatment" may refer to medical intervention of a disease or disorder that has already developed.

The term "prevention" relates to a prophylactic treatment, that is, to a measure or procedure, the purpose of which is to prevent, rather than to cure a disease. "Prevention" means that a desired pharmacological and/or physiological effect is obtained which is prophylactic in terms of completely or partially preventing a disease or symptom thereof.

The term "administration" means providing a substance (for example, an anti-CD300c antibody or an antigen-binding fragment thereof and another cancer therapy) to a subject to achieve a prophylactic or therapeutic purpose (for example, prevention or treatment of cancer).

The term "biological sample" encompasses a variety of sample types obtained from a subject and may be used in diagnostic or monitoring assays. The biological sample includes, but is not limited to, blood and other liquid samples of biological origin, and solid tissue samples such as a biopsy specimen or tissue cultures or cells derived therefrom and the progeny thereof. Thus, the biological sample encompasses a clinical sample, and also includes cells in culture, cell supernatants, cell lysates, serum, plasma, biological fluid, and tissue samples, in particular, tumor samples. The term "biological data" refers to any analytical data obtained using the biological sample.

The term "expression level" may be determined by measuring the expression level of at least one of mRNA and protein of a corresponding marker. For the method for measuring the expression level of mRNA or protein, any method known in the art may be used. For example, an agent for measuring the expression level of mRNA may be a pair of primers or a probe which specifically binds to the corresponding marker gene, and an agent for measuring the expression level of the protein may be an antibody, substrate, ligand, or cofactor which specifically binds to the corresponding marker. An assay method for measuring the mRNA level includes, but is not limited to, reverse transcriptase polymerase chain reaction, competitive reverse transcriptase polymerase chain reaction, real-time reverse transcriptase polymerase chain reaction, RNase protection assay, Northern blotting, or DNA chip assay. An assay method for measuring the protein level includes, but is not limited to, Western blotting, ELISA, radioimmunoassay, radial immunodiffusion assay, Ouchterlony immunodiffusion, rocket immunoelectrophoresis, immunohistochemical staining, immunoprecipitation assay, complement fixation assay, FACS, and protein chip assay.

The term "therapeutic responsiveness" refers to whether an individual suffering from or suspected of having cancer responds favorably or unfavorably to treatment with a therapeutically active ingredient (such as a CD300c antibody or an antigen-binding fragment thereof). The therapeutic responsiveness may be assessed by changes in immune system associated with tumor treatment which are observed after administration of the CD300c antibody or an antigen-binding fragment thereof.

### Anti-CD300c Antibody

According to an aspect of the present disclosure, there is provided an anti-CD300c antibody or an antigen-binding fragment thereof. The anti-CD300c antibody or an antigen-binding fragment thereof according to the present disclosure is an antigen-binding molecule that specifically binds to a CD300c protein. Preferably, the anti-CD300c antibody or an antigen-binding fragment thereof is a monoclonal antibody or an antigen-binding fragment thereof which specifically binds to a CD300c protein.

The term "CD300c protein" is used interchangeably with "CD300c" or "CD300c antigen" and is a protein encoded by CD300c gene. It is known that the CD300c protein exhibits significant sequence identity to B7 family proteins and is expressed on the membrane of antigen presenting cells. Inhibition of expression or activity of the CD300c protein may induce activation of T cells and/or promotion of differentiation into M1 macrophages.

In addition, the term "anti-CD300c antibody may be used interchangeably with a polypeptide that binds to the CD300c protein. The term "polypeptide" is intended to mean any polymer of amino acids linked to one another by peptide bonds, irrespective of its length. That is, the polypeptide as used herein also encompasses peptides and proteins.

In an embodiment, the anti-CD300c antibody or an antigen-binding fragment thereof is capable of specifically binding to an extracellular domain (ECD) of the CD300c protein. The extracellular domain of CD300c may be an extracellular domain of human CD300c protein. In addition, the extracellular domain of CD300c may include the amino acid sequence represented by SEQ ID NO: 402.

In an embodiment, it was identified that the expression level of a CD300c protein exhibits a very high correlation with survival of various cancer patients. Specifically, it was identified that relative to the average CD300c expression level of cancer patients, cancer patients with a high CD300c expression level had shorter survival than cancer patients with a low CD300c expression level. This means that inhibition of expression or activity of CD300c using the anti-CD300c antibody or an antigen-binding fragment thereof according to the present disclosure can result in a cancer therapeutic effect or an effect of increasing survival of cancer patients.

The anti-CD300c antibody or an antigen-binding fragment thereof according to the present disclosure can specifically bind to CD300c expressed on the surface of various cancer cells, and thus exhibit an anticancer effect. Binding of the anti-CD300c antibody to CD300c can activate T cells and at the same time promote differentiation into M1 macrophages to effectively inhibit proliferation of cancer cells, which allows the anti-CD300c antibody to be effectively used as an immunotherapy for various cancers. In addition, the anti-CD300c antibody according to the present disclosure can exhibit a further increased therapeutic effect through co-administration with a conventional cancer immunotherapy, and also has inter-species cross-reactivity (for example, between human and mouse antigens) that allows the antibody to be widely applied to various mammals. In addition, it is expected that in a case where resistant cancer cells showing the ability to resist apoptosis are treated with the anti-CD300c antibody of the present disclosure, this antibody is able to remarkably weaken resistance of the cancer cells, thereby showing excellent efficacy in preventing cancer recurrence. In addition, in general, cancer cells inhibit production of the proinflammatory cytokine IL-2 to evade the immune system. It was identified that the anti-CD300c antibody activates the immune system by restoring production of IL-2 blocked by these cancer cells, which induces cancer cell death. Thus, it is expected that the anti-CD300c antibody can be utilized as a more fundamental cancer immunotherapy. For the details on the CD300c protein or the anti-CD300c antibody, reference may also be made to Korean Patent Laid-Open Publication No. 10-2019-0136949, which is incorporated herein by reference in its entirety for all purposes.

In an embodiment, the anti-CD300c monoclonal antibody or an antigen-binding fragment thereof may comprise:
(i) a heavy chain variable region that comprises CDR1 comprising or consisting of an amino acid sequence selected from the group consisting of SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115, SEQ ID NO: 127, SEQ ID NO: 139, SEQ ID NO: 151, SEQ ID NO: 163, SEQ ID NO: 175, SEQ ID NO: 187, SEQ ID NO: 199, SEQ ID NO: 211, SEQ ID NO: 223, SEQ ID NO: 235, SEQ ID NO: 247, SEQ ID NO: 259, SEQ ID NO: 271, SEQ ID NO: 283, and SEQ ID NO: 295;
   CDR2 comprising or consisting of an amino acid sequence selected from the group consisting of SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116, SEQ ID NO: 128, SEQ ID NO: 140, SEQ ID NO: 152, SEQ ID NO: 164, SEQ ID NO: 176, SEQ ID NO: 188, SEQ ID NO: 200, SEQ ID NO: 212, SEQ ID NO: 224, SEQ ID NO: 236, SEQ ID NO: 248, SEQ ID NO: 260, SEQ ID NO: 272, SEQ ID NO: 284, and SEQ ID NO: 296; and
   CDR3 comprising or consisting of an amino acid sequence selected from the group consisting of SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117, SEQ ID NO: 129, SEQ ID NO: 141, SEQ ID NO: 153, SEQ ID NO: 165, SEQ ID NO: 177, SEQ ID NO: 189, SEQ ID NO: 201, SEQ ID NO: 213, SEQ ID NO: 225, SEQ ID NO: 237, SEQ ID NO: 249, SEQ ID NO: 261, SEQ ID NO: 273, SEQ ID NO: 285, and SEQ ID NO: 297; and
(ii) a light chain variable region that comprises CDR1 comprising or consisting of an amino acid sequence selected from the group consisting of SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 142, SEQ ID NO: 154, SEQ ID NO: 166, SEQ ID NO: 178, SEQ ID NO: 190, SEQ ID NO: 202, SEQ ID NO: 214, SEQ ID NO: 226, SEQ ID NO: 238, SEQ ID NO: 250, SEQ ID NO: 262, SEQ ID NO: 274, SEQ ID NO: 286, and SEQ ID NO: 298;
   CDR2 comprising or consisting of an amino acid sequence selected from the group consisting of SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119, SEQ ID NO: 131, SEQ ID NO: 143, SEQ ID NO: 155, SEQ ID NO: 167, SEQ ID NO: 179, SEQ ID NO: 191, SEQ ID NO: 203, SEQ ID NO: 215, SEQ ID NO: 227, SEQ ID NO: 239, SEQ ID NO: 251, SEQ ID NO: 263, SEQ ID NO: 275, SEQ ID NO: 287, and SEQ ID NO: 299; and
   CDR3 comprising or consisting of an amino acid sequence selected from the group consisting of SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120, SEQ ID NO: 132, SEQ ID NO: 144, SEQ ID NO: 156, SEQ ID NO: 168, SEQ ID NO: 180, SEQ ID NO: 192, SEQ ID NO: 204, SEQ ID NO: 216, SEQ ID NO: 228, SEQ ID NO: 240, SEQ ID NO: 252, SEQ ID NO: 264, SEQ ID NO: 276, SEQ ID NO: 288, and SEQ ID NO: 300.

In another embodiment, (i) the heavy chain variable region may comprise:
CDR1 comprising or consisting of an amino acid sequence selected from the group consisting of SEQ ID NO: 7, SEQ ID NO: 19, SEQ ID NO: 43, SEQ ID NO: 55, SEQ ID NO: 67, SEQ ID NO: 79, SEQ ID NO: 103, SEQ ID NO: 115, SEQ ID NO: 127, SEQ ID NO: 139, SEQ ID NO: 151, SEQ ID NO: 163, SEQ ID NO: 199, and SEQ ID NO: 211;
CDR2 comprising or consisting of an amino acid sequence selected from the group consisting of SEQ ID NO: 8, SEQ ID NO: 20, SEQ ID NO: 44, SEQ ID NO: 56, SEQ ID NO: 68, SEQ ID NO: 80, SEQ ID NO: 104, SEQ ID NO: 116, SEQ ID NO: 128, SEQ ID NO: 140, SEQ ID NO: 152, SEQ ID NO: 164, SEQ ID NO: 200, and SEQ ID NO: 212; and
CDR3 comprising or consisting of an amino acid sequence selected from the group consisting of SEQ ID NO: 9, SEQ ID NO: 21, SEQ ID NO: 45, SEQ ID NO: 57, SEQ ID NO: 69, SEQ ID NO: 81, SEQ ID NO: 105, SEQ ID NO: 117, SEQ ID NO: 129, SEQ ID NO: 141, SEQ ID NO: 153, SEQ ID NO: 165, SEQ ID NO: 201, and SEQ ID NO: 213; and
(ii) the light chain variable region may comprise:
   CDR1 comprising or consisting of an amino acid sequence selected from the group consisting of SEQ ID NO: 10, SEQ ID NO: 22, SEQ ID NO: 46, SEQ ID NO: 58, SEQ ID NO: 70, SEQ ID NO: 82, SEQ ID NO: 106, SEQ ID NO: 118, SEQ ID NO: 130, SEQ ID NO: 142, SEQ ID NO: 154, SEQ ID NO: 166, SEQ ID NO: 202, and SEQ ID NO: 214;
   CDR2 comprising or consisting of an amino acid sequence selected from the group consisting of SEQ ID NO: 11, SEQ ID NO: 23, SEQ ID NO: 47, SEQ ID NO: 59, SEQ ID NO: 71, SEQ ID NO: 83, SEQ ID NO: 107, SEQ ID NO: 119, SEQ ID NO: 131, SEQ ID NO: 143, SEQ ID NO: 155, SEQ ID NO: 167, SEQ ID NO: 203, and SEQ ID NO: 215; and
   CDR3 comprising or consisting of an amino acid sequence selected from the group consisting of SEQ ID NO: 12, SEQ ID NO: 24, SEQ ID NO: 48, SEQ ID NO: 60, SEQ ID NO: 72, SEQ ID NO: 84, SEQ ID NO: 108, SEQ ID NO: 120, SEQ ID NO: 132, SEQ ID NO: 144, SEQ ID NO: 156, SEQ ID NO: 168, SEQ ID NO: 204, and SEQ ID NO: 216.

In yet another embodiment, (i) the heavy chain variable region may comprise:
CDR1 comprising or consisting of an amino acid sequence selected from the group consisting of SEQ ID NO: 43, SEQ ID NO: 79, SEQ ID NO: 115, and SEQ ID NO: 211;
CDR2 comprising or consisting of an amino acid sequence selected from the group consisting of SEQ ID NO: 44, SEQ ID NO: 80, SEQ ID NO: 116, and SEQ ID NO: 212; and
CDR3 comprising or consisting of an amino acid sequence selected from the group consisting of SEQ ID NO: 45, SEQ ID NO: 81, SEQ ID NO: 117, and SEQ ID NO: 213; and
(ii) the light chain variable region may comprise:
   CDR1 comprising or consisting of an amino acid sequence selected from the group consisting of SEQ ID NO: 46, SEQ ID NO: 82, SEQ ID NO: 118, and SEQ ID NO: 214;
   CDR2 comprising or consisting of an amino acid sequence selected from the group consisting of SEQ ID NO: 47, SEQ ID NO: 83, SEQ ID NO: 119, and SEQ ID NO: 215; and
   CDR3 comprising or consisting of an amino acid sequence selected from the group consisting of SEQ ID NO: 48, SEQ ID NO: 84, SEQ ID NO: 120, and SEQ ID NO: 216.

In still yet another embodiment, the heavy chain variable region may comprise CDR1 comprising or consisting of the amino acid sequence represented by SEQ ID NO: 43, CDR2 comprising or consisting of the amino acid sequence represented by SEQ ID NO: 44, and CDR3 comprising or consisting of the amino acid sequence represented by SEQ ID NO: 45; and the light chain variable region may include CDR1 comprising or consisting of the amino acid sequence represented by SEQ ID NO: 46, CDR2 comprising or consisting of the amino acid sequence represented by SEQ ID NO: 47, and CDR3 comprising or consisting of the amino acid sequence represented by SEQ ID NO: 48.

In still yet another embodiment, the heavy chain variable region may comprise CDR1 comprising or consisting of the amino acid sequence represented by SEQ ID NO: 79, CDR2 comprising or consisting of the amino acid sequence represented by SEQ ID NO: 80, and CDR3 comprising or consisting of the amino acid sequence represented by SEQ ID NO: 81, and the light chain variable region may include CDR1 comprising or consisting of the amino acid sequence represented by SEQ ID NO: 82, CDR2 comprising or consisting of the amino acid sequence represented by SEQ ID NO: 83, and CDR3 comprising or consisting of the amino acid sequence represented by SEQ ID NO: 84.

In still yet another embodiment, the heavy chain variable region may comprise CDR1 comprising or consisting of the amino acid sequence represented by SEQ ID NO: 115, CDR2 comprising or consisting of the amino acid sequence represented by SEQ ID NO: 116, and CDR3 comprising or consisting of the amino acid sequence represented by SEQ ID NO: 117, and the light chain variable region may include CDR1 comprising or consisting of the amino acid sequence represented by SEQ ID NO: 118, CDR2 comprising or consisting of the amino acid sequence represented by SEQ ID NO: 119, and CDR3 comprising or consisting of the amino acid sequence represented by SEQ ID NO: 120.

In still yet another embodiment, the heavy chain variable region may comprise CDR1 comprising or consisting of the amino acid sequence represented by SEQ ID NO: 211, CDR2 comprising or consisting of the amino acid sequence represented by SEQ ID NO: 212, and CDR3 comprising or consisting of the amino acid sequence represented by SEQ ID NO: 213, and the light chain variable region may include CDR1 comprising or consisting of the amino acid sequence represented by SEQ ID NO: 214, comprising or CDR2 consisting of the amino acid sequence represented by SEQ ID NO: 215, and CDR3 comprising or consisting of the amino acid sequence represented by SEQ ID NO: 216.

In still yet another embodiment, the heavy chain variable region may comprise an amino acid sequence selected from the group consisting of SEQ ID Nos: 303, 307, 311, 315, 319, 323, 327, 331, 335, 339, 343, 347, 351, 355, 359, 363, 367, 371, 375, 379, 383, 387, 391, 395, and 399; and the light chain variable region may comprise an amino acid sequence selected from the group consisting of SEQ ID Nos: 304, 308, 312, 316, 320, 324, 328, 332, 336, 340, 344, 348, 352, 356, 360, 364, 368, 372, 376, 380, 384, 388, 392, 396, and 400.

In still yet another embodiment, the heavy chain variable region may comprise an amino acid sequence selected from the group consisting of SEQ ID Nos: 315, 327, 339, and 371; and the light chain variable region may comprise an amino acid sequence selected from the group consisting of SEQ ID Nos: 316, 328, 340, and 372. Preferably, the heavy chain variable region may comprise the amino acid sequence represented by SEQ ID No: 315 and the light chain variable region may comprise the amino acid sequence represented by of SEQ ID No: 316; or the heavy chain variable region may comprise the amino acid sequence represented by SEQ ID No: 327 and the light chain variable region may comprise the amino acid sequence represented by of SEQ ID No: 328; or the heavy chain variable region may comprise the amino acid sequence represented by SEQ ID No: 339 and the light chain variable region may comprise the amino acid sequence represented by of SEQ ID No: 340; or the heavy chain variable region may comprise the amino acid sequence represented by SEQ ID No: 371 and the light chain variable region may comprise the amino acid sequence represented by of SEQ ID No: 372.

In another aspect, there is provided an anti-CD300c monoclonal antibody or an antigen-binding fragment thereof, comprising a heavy chain variable region that includes CDR1 to CDR3 comprising or consisting of amino acid sequences, respectively, represented by Formulas (1) to (3), and a light chain variable region that includes CDR1 to CDR3 comprising or consisting of amino acid sequences, respectively, represented by Formulas (4) to (6) (each amino acid sequence is shown in N→C direction):
FTFX1X2X3X4MX5WVR (1) (SEQ ID NO: 403) in the above formula,
   X1 = G or S
   X2 = S, R, or D
   X3 = N or Y
   X4 = Y, A, G, or H
   X5 = S or H
X1ISX2SGX3X4TYYAX5 (2) (SEQ ID NO: 404) in the above formula,
   X1 = T or A
   X2 = G or S
   X3 = T or G
   X4 = S or Y
   X5 = D or E
YCAX1X2X3X4X5X6X7X8X9W (3) (SEQ ID NO: 405) in the above formula,
   X1 = R or S
   X2 = G or S
   X3 = M, S, Y, or I
   X4 = W, Q, G, or R
   X5 = G or L
   X6 = M, I, or P
   X7 = D, F, or L
   X8 = V or D
   X9 = I, Y, or not present
CX1X2X3X4X5X6X7X8X9X10X11VX12W (4) (SEQ ID NO: 406) in the above formula,
   X1 = T or S
   X2 = G or R
   X3 = K, N, or S
   X4 = H, N, or S
   X5 = R, I, or G
   X6 = H, G, or I
   X7 = T, I, or S
   X8 = R, A, K, or not present
   X9 = R, S, G, or not present
   X10 = N or not present
   X11 = Y or not present
   X12 = N, H, or Q
X1X2X3X4RPSGVX5 (5) (SEQ ID NO: 407) in the above formula,
   X1 = L, S, R, or E
   X2 = D, K, or N
   X3 = S or N
   X4 = E, N, Q, or K
   X5 = P or R
YCX1X2X3X4X5X6X7X8X9X10VF (6) (SEQ ID NO: 408) in the above formula,
   X1 = Q, A, or S
   X2 = S or A
   X3 = Y or W
   X4 = D or A
   X5 = S, D, or G
   X6 = S, N, or T
   X7 = S, L, N, or K
   X8 = V, S, N, or G
   X9 = G, L, V, or not present
   X10 = P or not present.

In certain embodiments, the anti-CD300c antibody or an antigen-binding fragment may comprise sequences that have 80% or more, 90% or more, 91% or more, 92% or more, 93% or more, 94% or more, 95% or more, 96% or more, 97% or more, 98% or more, or 99% or more sequence identity to the above-mentioned CDR sequences or the sequences shown in Tables 3, 4, and 5.

In certain embodiments, amino acid sequence variants of the antibodies of the present disclosure are contemplated. For example, it may be desirable to improve the binding affinity and/or other biological properties of the antibody. Amino acid sequence variants of the antibody may be prepared by introducing appropriate modifications into the nucleotide sequence encoding the molecules, or by peptide synthesis. Such modifications include, for example, deletions from, and/or insertions into and/or substitutions of residues within the amino acid sequence of the antibody. Any combination of deletion, insertion, and substitution can be made to arrive at the final construct, provided that the final construct possesses the desired characteristics, for example, antigen-binding. Sites of interest for substitutional mutagenesis include heavy chain variable regions (HVRs) and framework regions (FRs). Conservative substitutions are provided in Table 1 under the heading "Preferred Substitutions" and further described below in reference to amino acid side chain classes (1) to (6). Amino acid substitutions may be introduced into the molecule of interest and the products screened for a desired activity, for example, retained/improved antigen binding, decreased immunogenicity, or improved ADCC or CDC.

**[Table 1]**

| Original residue | Exemplary substitutions | Preferred substitutions |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gin; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Ile |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

| | | |
|---|---|---|
| Amino acids may be grouped according to common side-chain properties: (1) hydrophobic: norleucine, Met, Ala, Val, Leu, Ile; (2) neutral hydrophilic: Cys, Ser, Thr, Asn, Gln; (3) acidic: Asp, Glu; (4) basic: His, Lys, Arg; (4) basic: His, Lys, Arg; (5) residues that influence chain orientation: Gly, Pro; (6) aromatic: Trp, Tyr, Phe. | | |

Non-conservative substitutions will entail exchanging a member of one of these classes for another class.

As used herein, the term "amino acid sequence variant" includes substantial variants wherein there are amino acid substitutions in one or more hypervariable region residues of a parent antigen binding molecule (for example, a humanized or human antibody). In general, the resulting variant(s) selected for further study will have modifications (for example, improvements) in certain biological properties (for example, increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, for example, using phage display-based affinity maturation techniques known in the art. Briefly, one or more HVR residues are mutated and the variant antigen binding molecules displayed on phage and screened for a particular biological activity (for example, binding affinity). In certain embodiments, substitutions, insertions, or deletions may occur within one or more HVRs so long as such alterations do not substantially reduce the ability of the antigen binding molecule to bind antigen. For example, conservative alterations (for example, conservative substitutions as provided herein) that do not substantially reduce binding affinity may be made in HVRs.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include antibodies with an N-terminal methionyl residue. Other insertional variants of the molecule include the fusion to the N- or C-terminus to a polypeptide which increases the serum half-life of the antibody. In addition, other insertional variants of the molecule may include fusions to the N-terminus or C-terminus of the polypeptide to facilitate passage of the blood-brain barrier (BBB).

In addition, there are provided variants of the antibody or an antigen-binding fragment thereof of the present disclosure which have improved affinity for the CD300c antigen. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of *E*. *coli* (Low et al., J. Mol. Biol., 250, 359-368, 1996), DNA shuffling (Patten et al., Curr. Opin. Biotechnol., 8, 724-733, 1997), phage display (Thompson et al., J. Mol. Biol., 256, 77-88, 1996), and sexual PCR (Crameri et al., Nature, 391, 288-291, 1998). Vaughan et al. (Science, 239, 1534-1536, 1988) discuss these methods of affinity maturation.

In an embodiment, the anti-CD300c monoclonal antibody or an antigen-binding fragment thereof may have inter-species cross-reactivity. Specifically, the anti-CD300c monoclonal antibody or an antigen-binding fragment thereof may exhibit cross-reactivity to both human and mouse CD300c antigens. Such cross-reactivity is identified in Experimental Examples 4.1 to 4.4.

In another embodiment, the anti-CD300c monoclonal antibody or an antigen-binding fragment thereof may include a form of an antibody-drug conjugate, in which the anti-CD300c monoclonal antibody or an antigen-binding fragment thereof is bound to another drug, or may be provided in such a form.

As used herein, the term "antibody-drug conjugate" refers to a form in which the drug and the antibody are chemically linked to each other without degrading biological activity of the antibody and the drug. In the present disclosure, the antibody-drug conjugate denotes a form in which the drug is bound to an amino acid residue at the N-terminus of the heavy and/or light chain of the antibody, specifically, a form in which the drug is bound to an α-amine group at the N-terminus of the heavy and/or light chain of the antibody.

The "drug" may mean any substance having a certain biological activity for a cell (for example, a cancer cell), which is a concept including DNA, RNA, or a peptide. The drug may be in a form which contains a reactive group capable of reacting and crosslinking with an α-amine group, and also includes a form which contains a reactive group capable of reacting and crosslinking with an α-amine group and to which a linker is linked.

The reactive group capable of reacting and crosslinking with the α-amine group are not particularly limited in terms of type as long as the reactive group can react and crosslink with an α-amine group at the N-terminus of a heavy chain or light chain of an antibody. The reactive group includes all types of groups known in the art which react with an amine group. The reactive group may, for example, be any one of isothiocyanate, isocyanate, acyl azide, NHS ester, sulfonyl chloride, aldehyde, glyoxal, epoxide, oxirane, carbonate, aryl halide, imidoester, carbodiimide, anhydride, and fluorophenyl ester, but is not limited thereto.

The drug is included regardless of the type of drug that can treat the disease targeted by the anti-CD300c antibody or antigen-binding fragment thereof according to the present disclosure, but may preferably be an anticancer agent.

### Nucleic Acid, Vector, Host Cell, and Production Method

The anti-CD300c monoclonal antibody or an antigen-binding fragment thereof of the present disclosure can be produced by any antibody production technique known in the art.

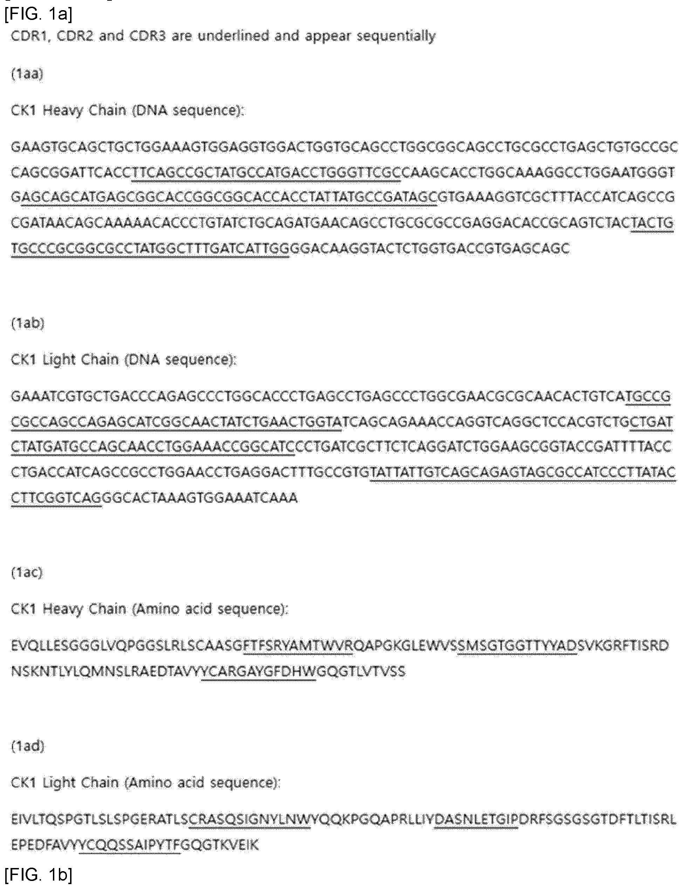

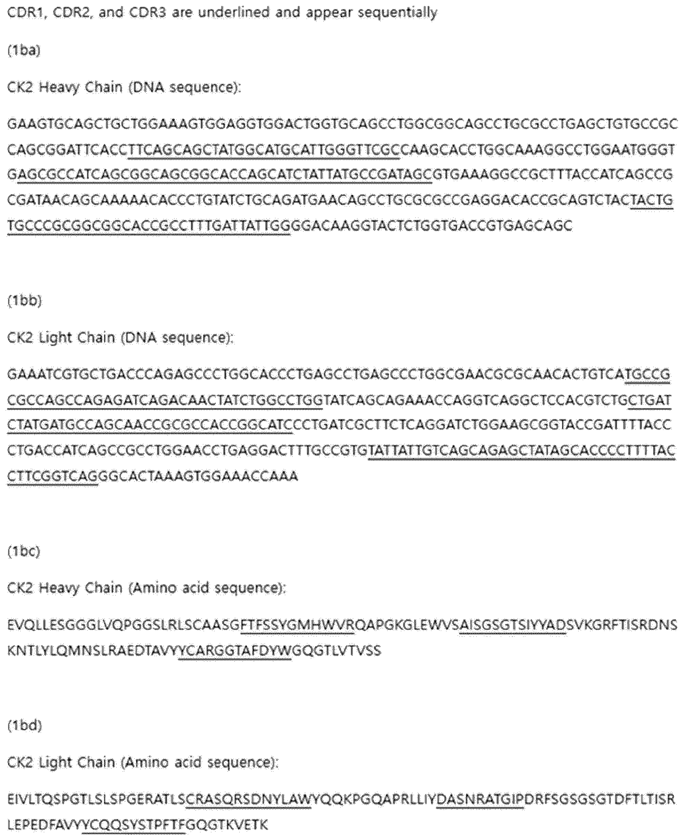

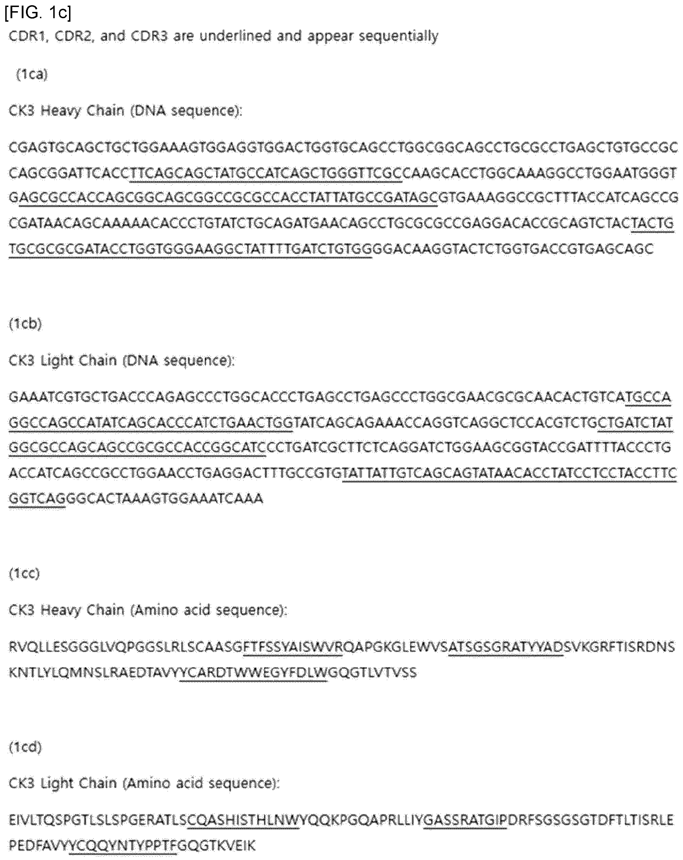

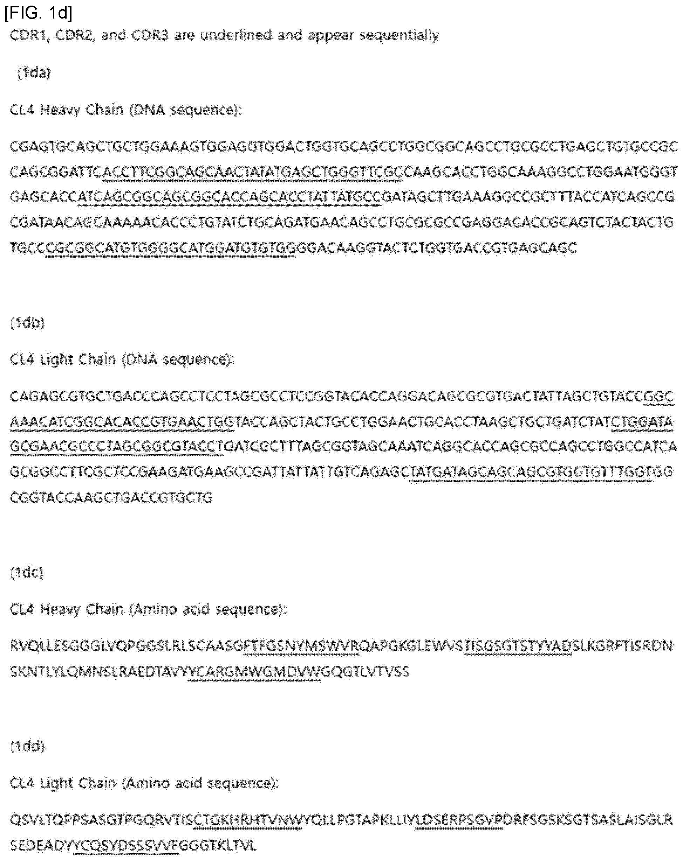

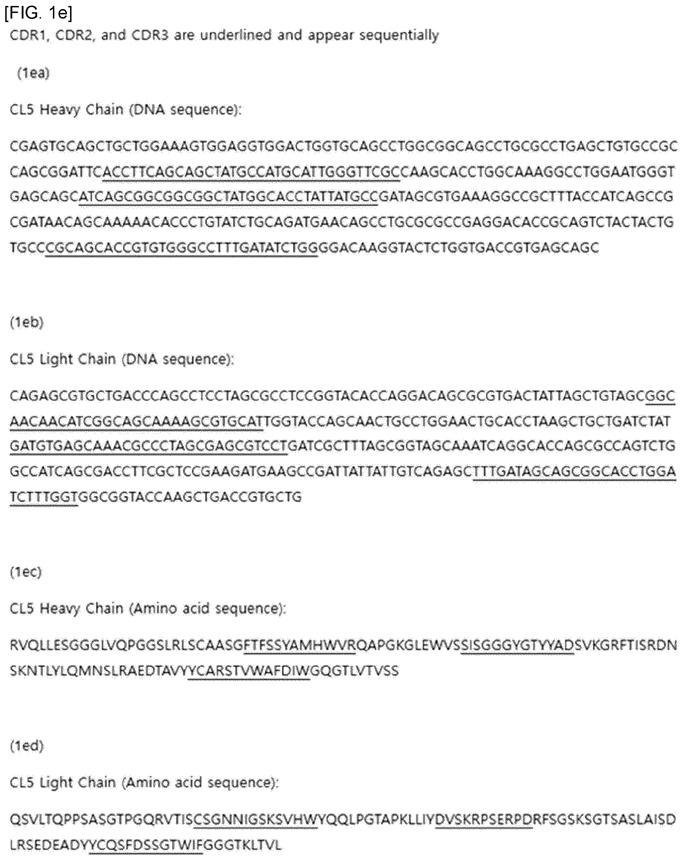

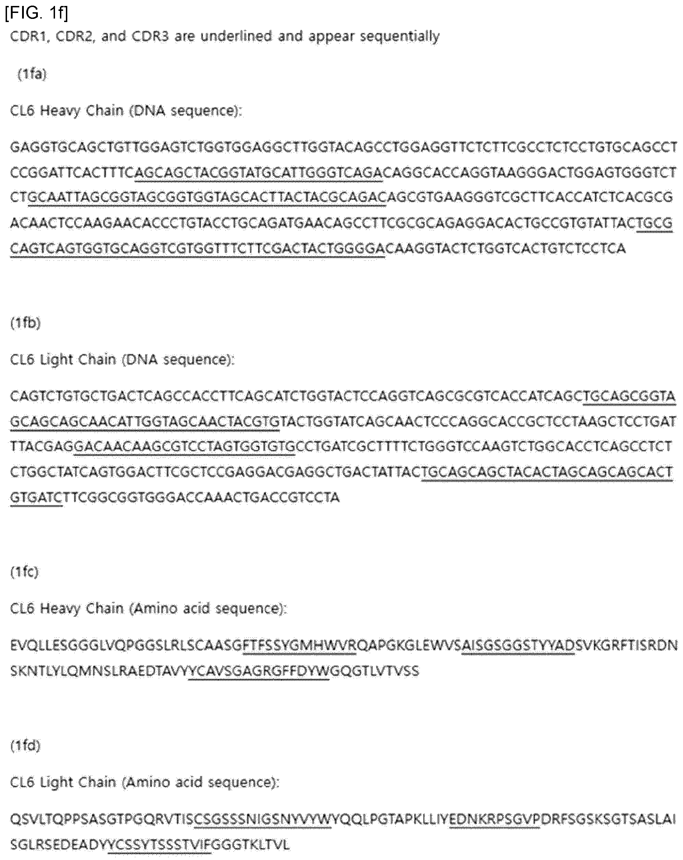

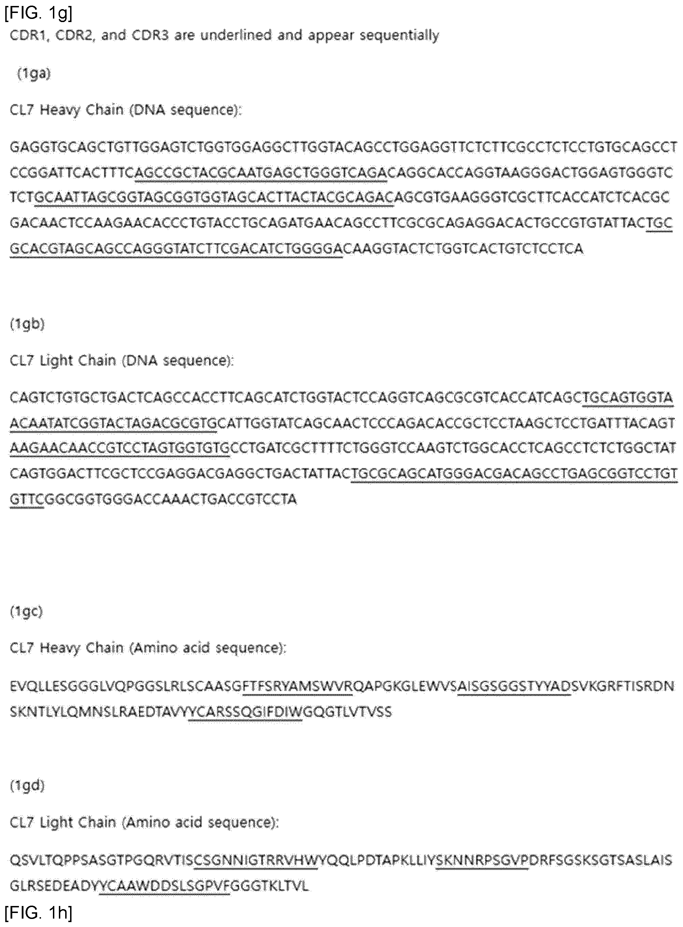

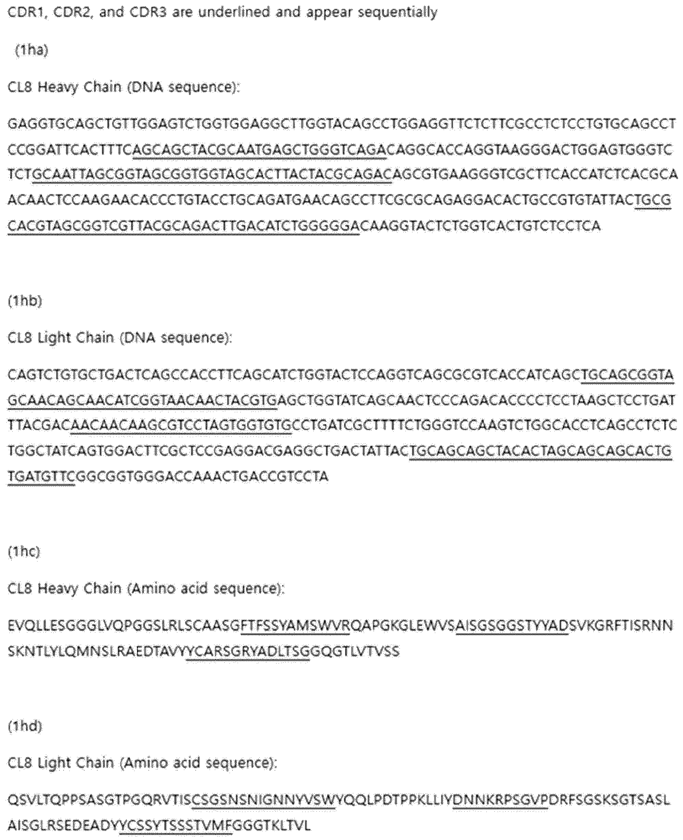

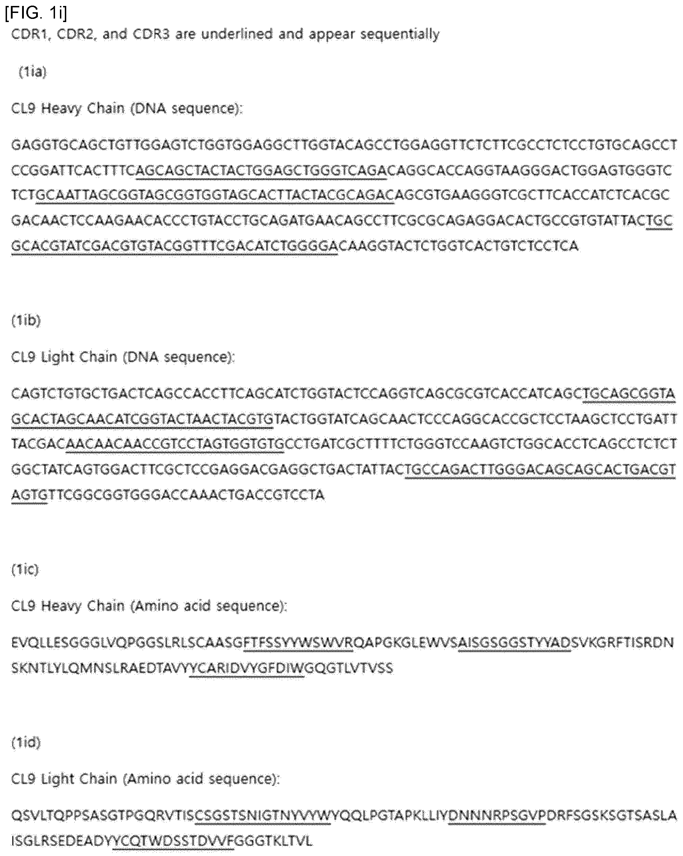

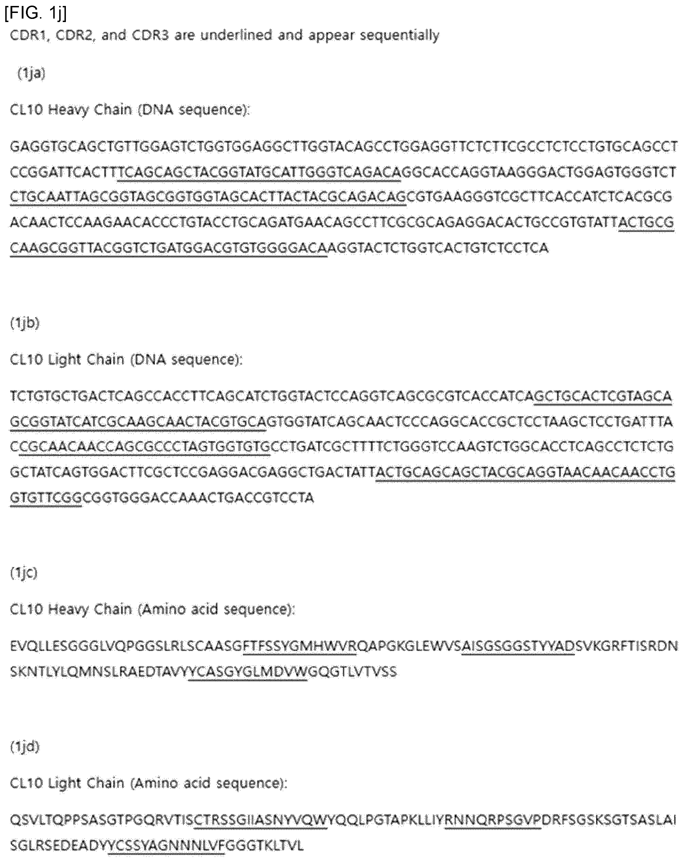

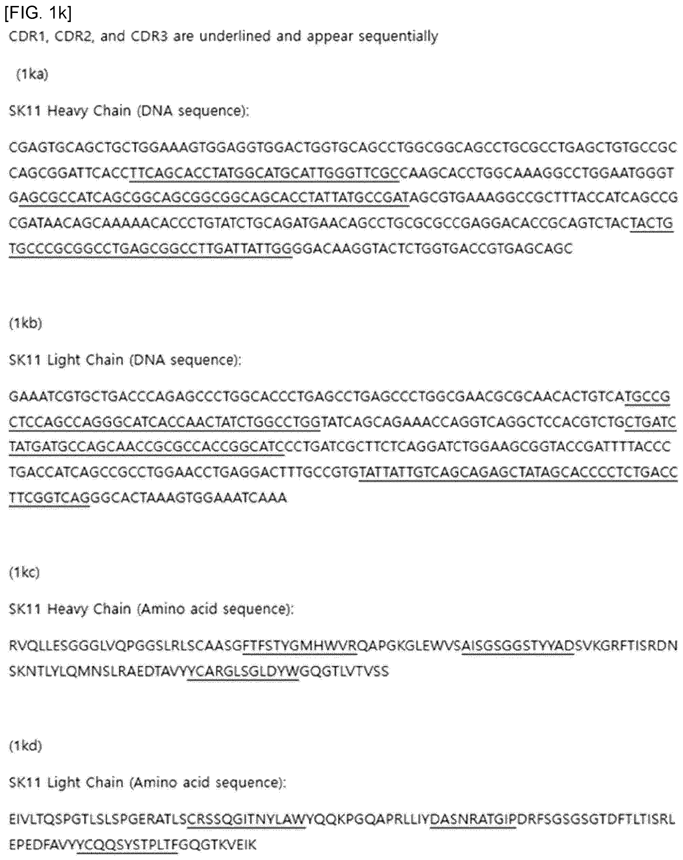

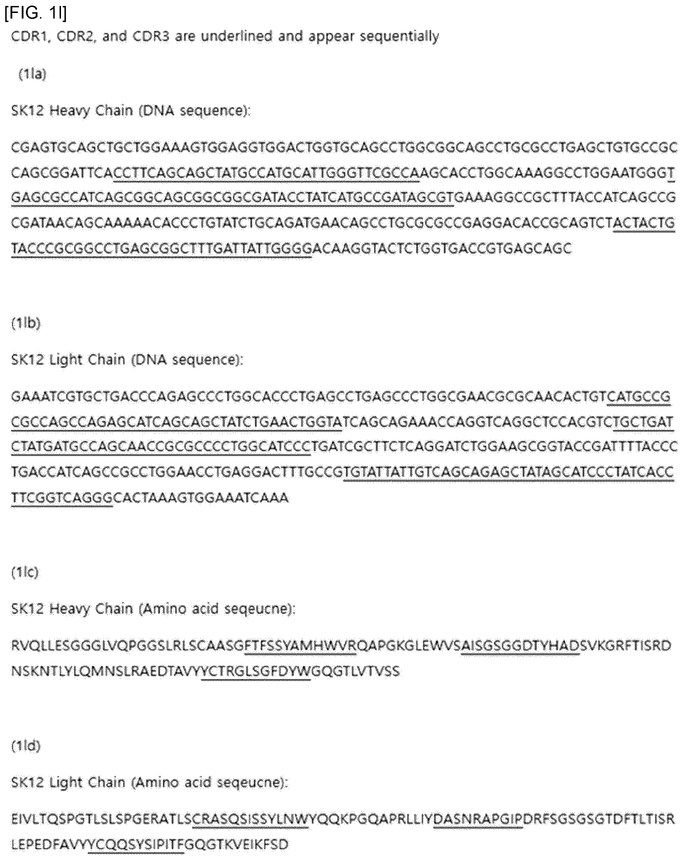

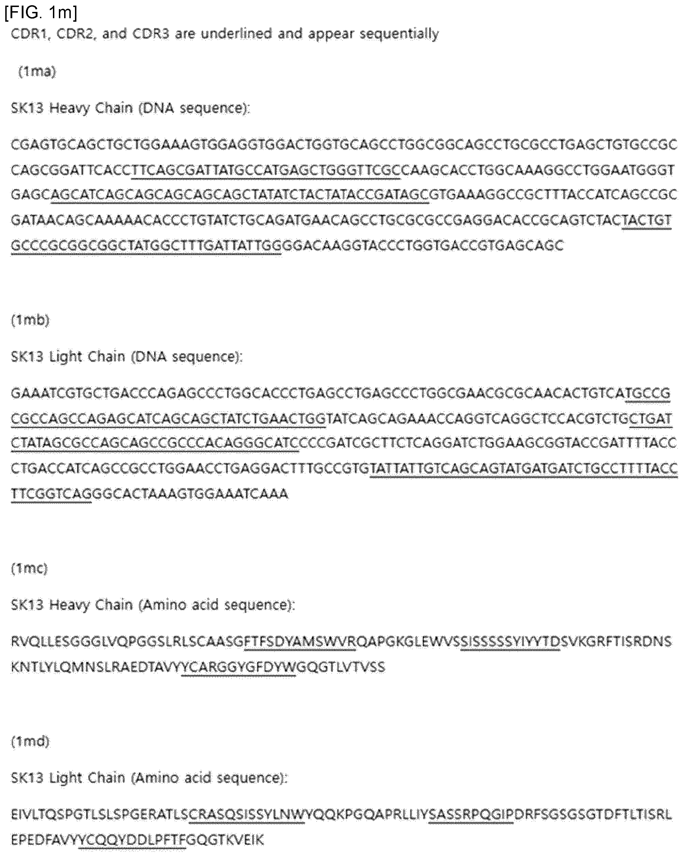

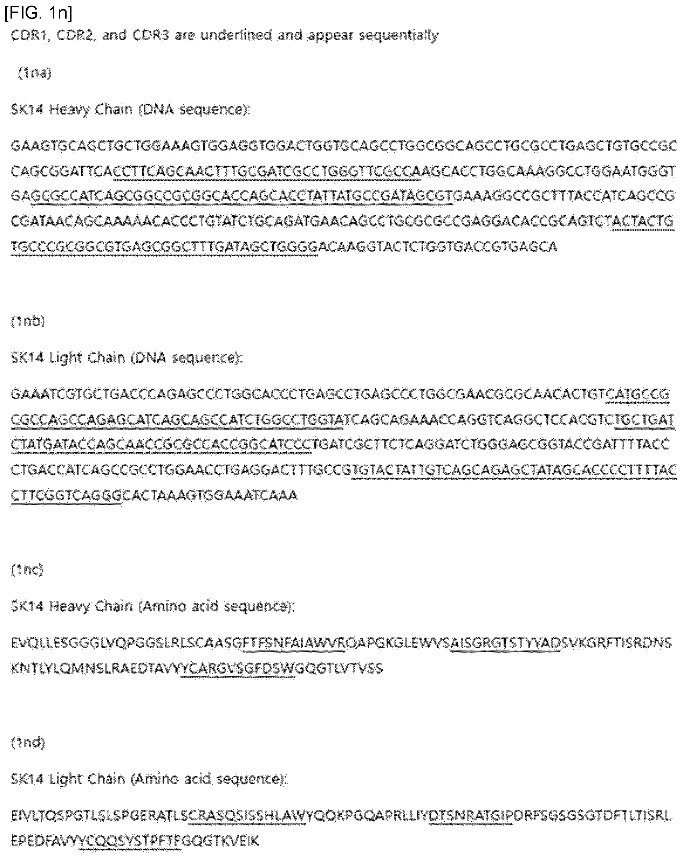

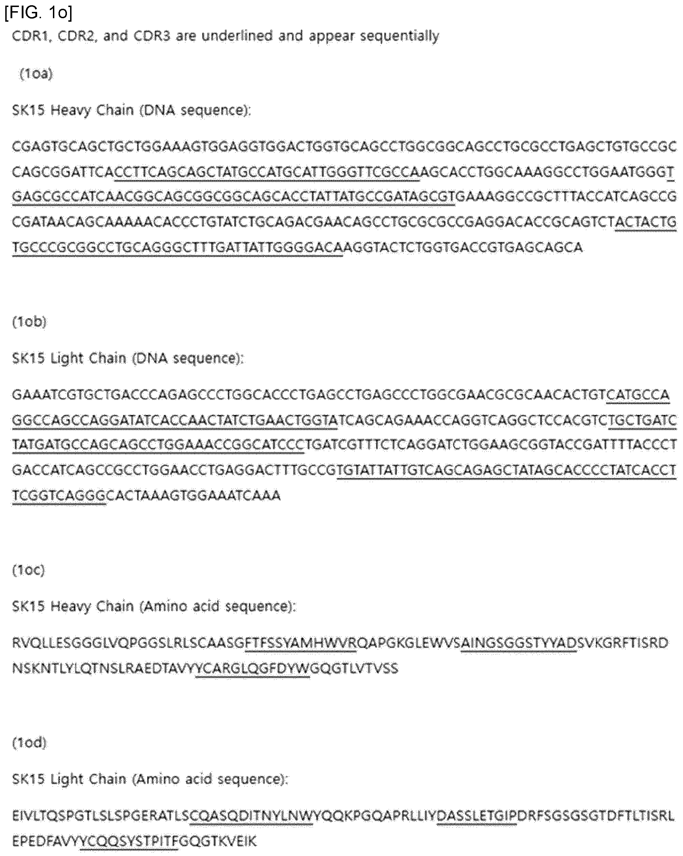

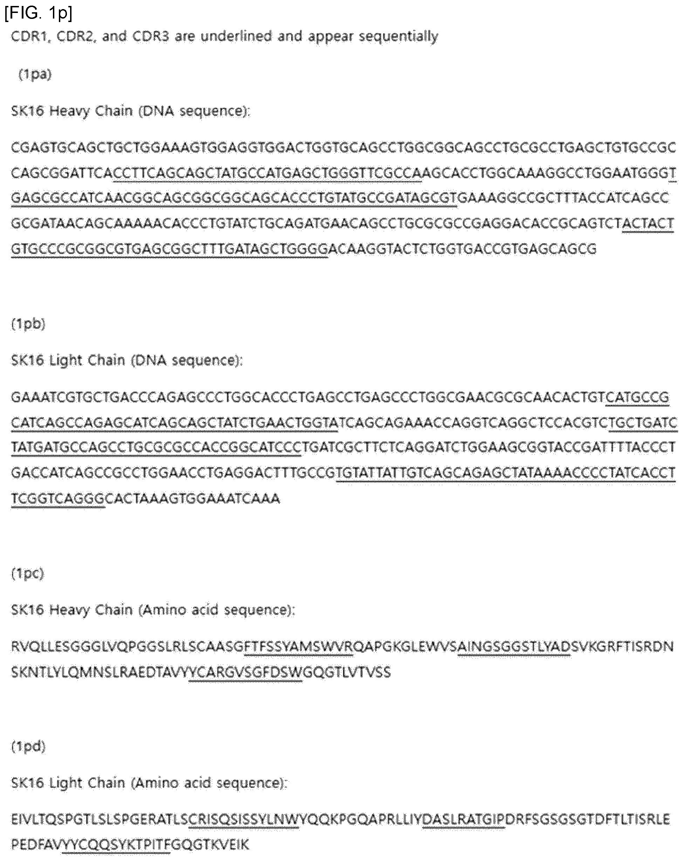

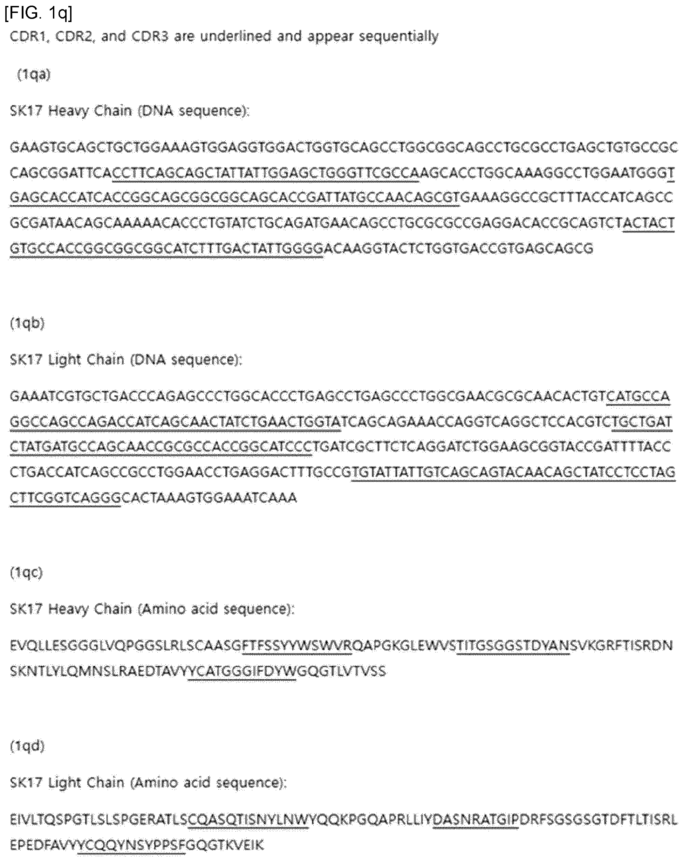

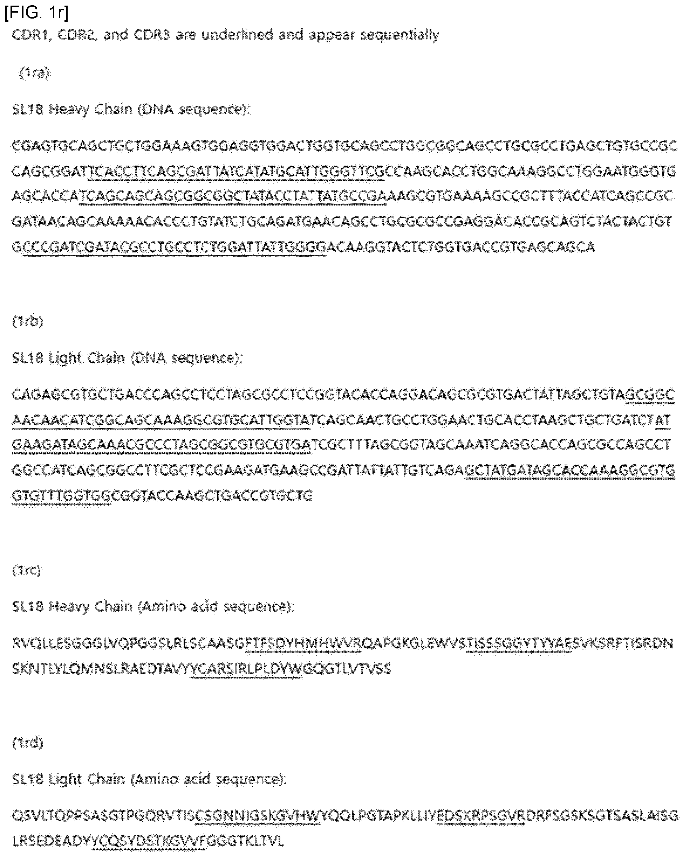

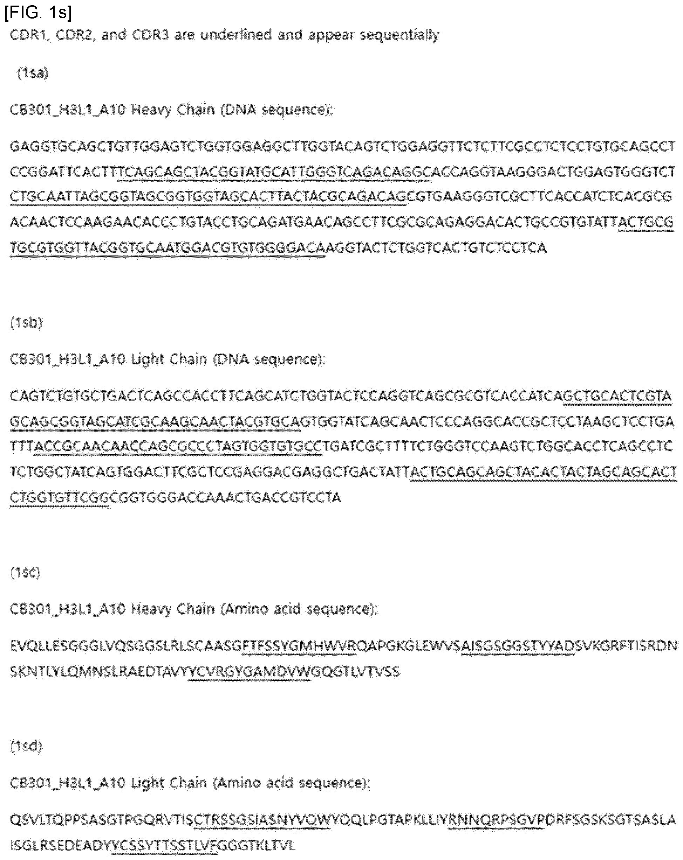

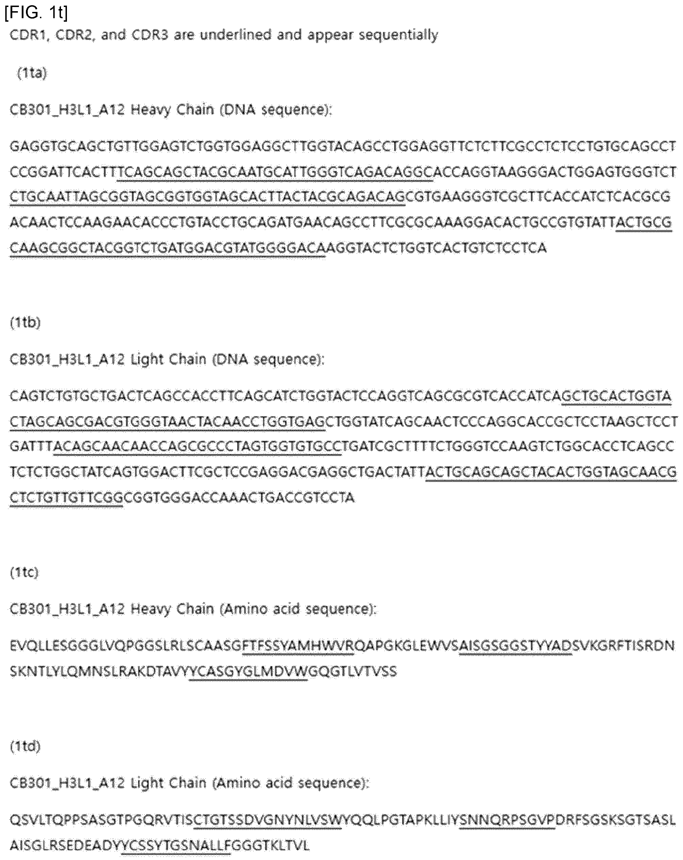

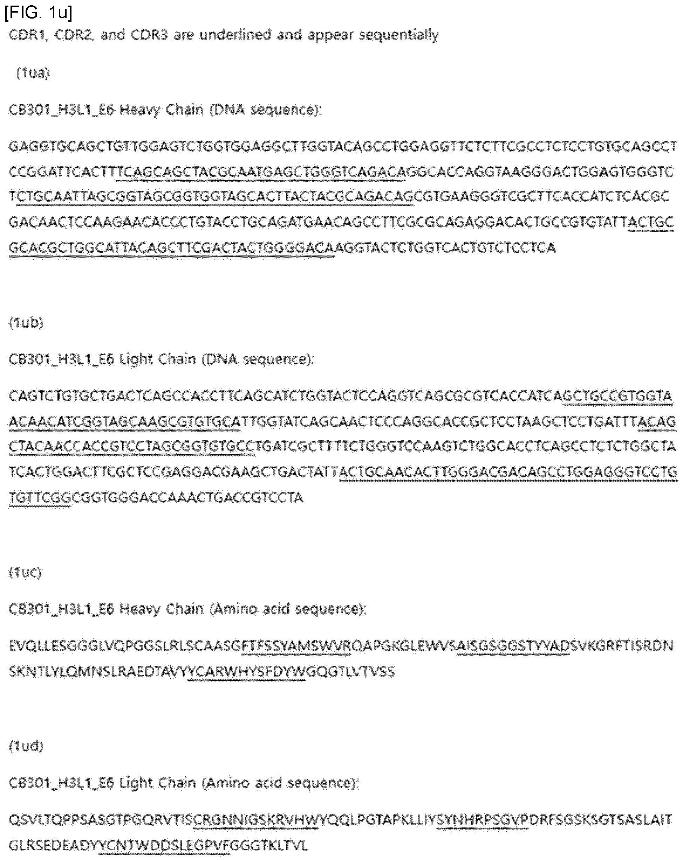

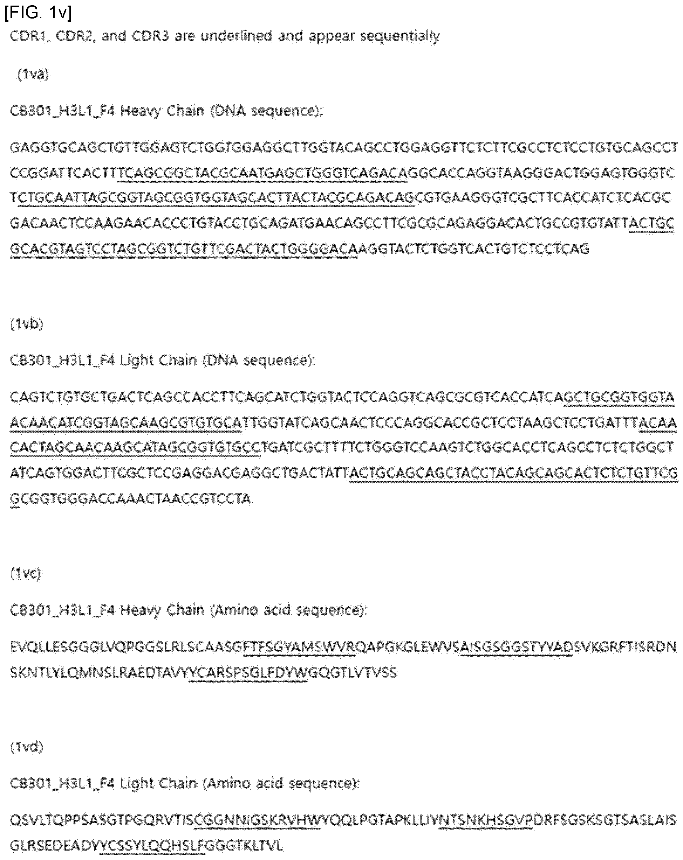

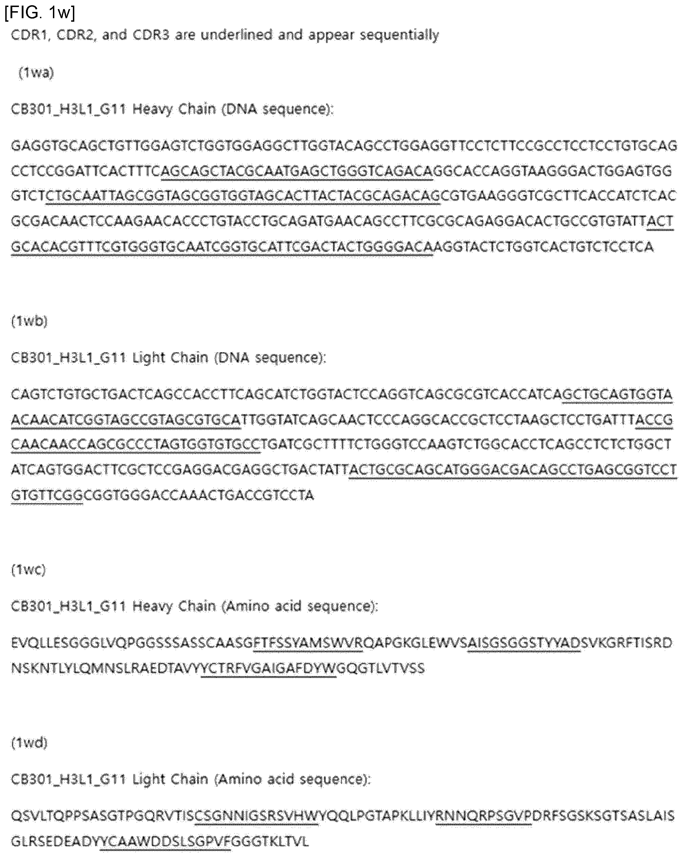

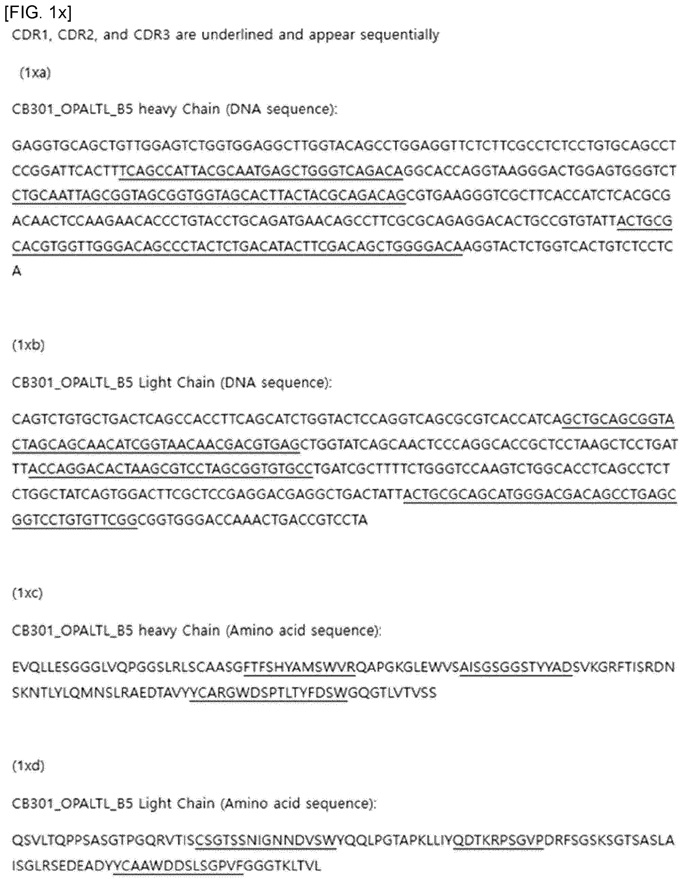

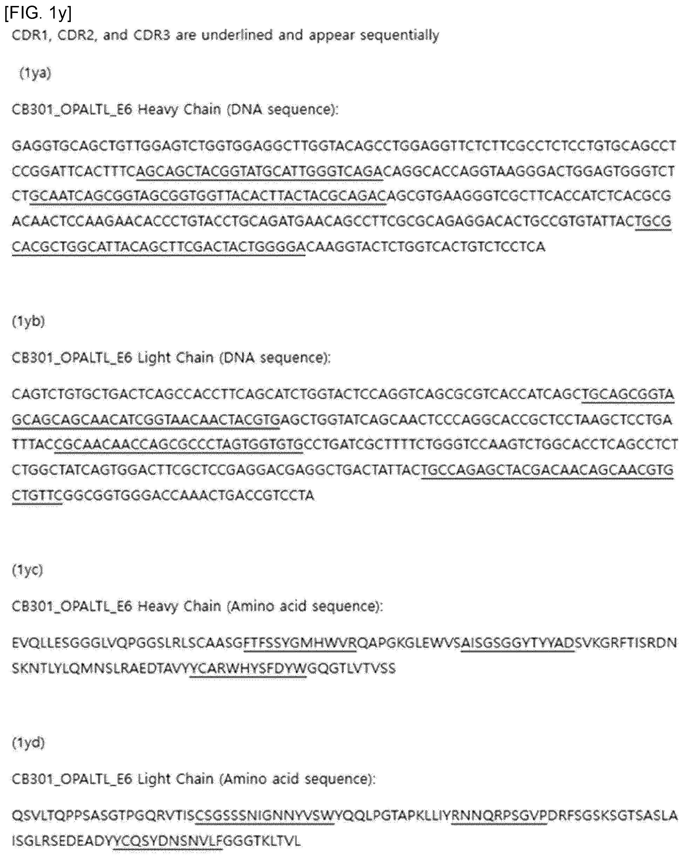

According to yet another aspect of the present disclosure, there is provided a nucleic acid molecule (for example, a polynucleotide) encoding the anti-CD300c monoclonal antibody or an antigen-binding fragment thereof. Such a nucleic acid molecule may encode an amino acid sequence, which comprises a heavy chain variable region or heavy chain CDR regions, and/or an amino acid sequence, which comprises a light chain variable region or light chain CDR regions, of the anti-CD300c monoclonal antibody. For sequences of the nucleic acid molecules encoding the heavy/light chain variable region and the CDR regions of the anti-CD300c monoclonal antibody according to the present disclosure, reference may be made to Tables 3 to 5 and FIG. 1.

The term "nucleic acid molecule" encompasses DNA (gDNA and cDNA) and RNA molecules. In the nucleic acid molecule, a nucleotide, which is a basic unit, also includes an analogue in which a sugar or base part is modified, as well as a natural nucleotide. The sequences of nucleic acid molecules encoding the heavy chain and light chain variable regions of the present disclosure may be modified. The modification includes additions, deletions, or non-conservative or conservative substitutions of nucleotides. The nucleic acid molecule of the present disclosure is interpreted to also include a nucleotide sequence having substantial identity to the nucleotide sequence as described above. The substantial identity refers to a nucleotide sequence exhibiting at least 80% homology, at least 90% homology in one specific example, at least 95% homology in another specific example, or at least 98% homology in yet another specific example when the nucleotide sequence of the present disclosure is aligned to correspond to any other sequence as much as possible, and the aligned sequence is analyzed using an algorithm commonly used in the art.

According to still yet another aspect of the present disclosure, there is provided at least one vector (for example, an expression vector) comprising the nucleic acid.

The term "vector" refers to a nucleic acid molecule capable of transporting another nucleic acid to which it has been linked. One type of vector is a "plasmid," which refers to a circular double stranded DNA loop into which additional DNA segments can be inserted. Another type of vector is a viral vector, wherein virally-derived DNA or RNA sequences are present in the vector for packaging into a virus. Certain vectors are capable of autonomous replication in a host cell into which they are introduced (for example, bacterial vectors having a bacterial origin of replication and episomal mammalian vectors). Other vectors (for example, non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids. In the present specification, "plasmid" and "vector" may be used interchangeably as the plasmid is the most commonly used form of vector. However, the present disclosure is intended to include other forms of expression vectors, such as viral vectors (for example, replication defective retroviruses, adenoviruses, and adeno-associated viruses), which serve equivalent functions.

According to still yet another aspect of the present disclosure, there is provided a host cell comprising at least one nucleic acid molecule (for example, a polynucleotide) that encodes the monoclonal antibody of the present disclosure.

The host cell may be a cell transformed with the recombinant vector of the present disclosure. Any host cell known in the art to enable stable and continuous cloning or expression of the recombinant vector may be used. Suitable prokaryotic host cells may include *E*. *coli, Bacillus* species strains such as *Bacillus subtillis* or *Bacillus thuringiensis,* intestinal bacteria and strains such as *Salmonella typhymurum, Serratia marcescens,* and various *Pseudomonas* species. Suitable eukaryotic host cells to be transformed may include yeasts, such as *Saccharomyces cerevisiae,* insect cells, plant cells, and animal cells, for example, Sp2/0, Chinese hamster ovary (CHO) K1, CHO DG44, PER.C6, W138, BHK, COS-7, 293, HepG2, Huh7, 3T3, RIN, and MDCK cell lines. In addition, the "host cell" is used to refer to a cell which has been transformed, or a cell which is capable of being transformed with a nucleic acid sequence and then expressing a selected gene of interest. The term includes the progeny of the parent cell, whether or not the progeny is identical in morphology or in genetic make-up to the original parent, so long as the selected gene is present.

According to still yet another aspect of the present disclosure, there is provided a method for producing the anti-CD300c monoclonal antibody or an antigen-binding fragment thereof, comprising culturing the host cell.

Culture of host cells for production of the antibody or an antigen-binding fragment thereof may be performed under suitable media and culture conditions known in the art. A process for such culture can easily be adjusted by those skilled in the art depending on a selected host cell. The culture process is divided into a suspension culture and an adherent culture in terms of the type of cell growth, and is divided into batch, fed-batch and continuous culture in terms of the type of culture. Various culture processes are disclosed, for example, in "Biochemical Engineering" by James M. Lee, Prentice-Hall International Editions, pp 138-176.

To collect the antibody, any method known in the art for purification of immunoglobulins may be used and examples thereof may include chromatography (ion exchange, affinity (for example, protein A), size exclusion, and the like), centrifugation, differential solubility, or other standard techniques for purification of proteins.

### Combination with Cancer Immunotherapy

The present inventors have found that the anti-CD300c antibody or an antigen-binding fragment thereof can exhibit an enhanced anticancer effect when used in combination with one or more other cancer immunotherapies. Accordingly, the anti-CD300c antibody or an antigen-binding fragment thereof of the present disclosure may be used in combination with one or more other cancer immunotherapies to prevent or treat cancer.

Cancer immunotherapies have a novel mechanism by which immune cells in the body are activated to kill cancer cells, and thus are advantageous in that they can be widely used for most cancers without specific genetic mutations. In addition, the immunotherapies have fewer adverse effects in that they treat cancer by strengthening the patient's own immune system, and have effects of improving the patient's quality of life and significantly extending the survival. These immunotherapies include immune checkpoint inhibitors, and may be manufactured by known methods or commercially available products. Examples of the immunotherapy include, but are not limited to, anti-PD-1, anti-PD-L1, anti-CTLA-4, anti-CD47, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT antibodies. In addition, examples of the immunotherapy include, but are not limited to, durvalumab (IMFINZI^{®}), atezolizumab (TECENTRIQ^{®}), avelumab (BAVENCIO^{®}), pembrolizumab (KEYTRUDA^{®}), nivolumab (OPDIVO^{®}), αCD47, cemiplimab (LIBTAYO^{®}), magrolimab (Hu5F9-G4), and ipilimumab (YERVOY^{®}).

In an embodiment, the immunotherapy may include at least any one selected from the group consisting of anti-PD-1, anti-PD-L1, anti-CTLA-4, anti-CD47, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT antibodies. In one example, the immunotherapy may include at least any one selected from the group consisting of anti-PD-1, anti-PD-L1, anti-CTLA-4, and anti-CD47 antibodies.

In another embodiment, the immunotherapy may include at least any one selected from the group consisting of durvalumab (IMFINZI^{®}), atezolizumab (TECENTRIQ^{®}), pembrolizumab (KEYTRUDA^{®}), nivolumab (OPDIVO^{®}), αCD47, and ipilimumab (YERVOY^{®}).

### Method for Prevention or Treatment of Cancer

According to still yet another aspect of the present disclosure, there is provided a method for preventing or treating cancer, improving or decreasing severity of at least one symptom or sign of cancer, inhibiting metastasis, or inhibiting growth of cancer using the anti-CD300c antibody or an antigen-binding fragment thereof according to the present disclosure. As used herein, "preventing or treating cancer" may include inhibiting proliferation, survival, metastasis, recurrence, or therapy resistance of cancer. Such a method may comprise administering the anti-CD300c antibody or an antigen-binding fragment thereof according to the present disclosure to a subject in need of prevention or treatment of cancer.

As used herein, the term "cancer" refers to a physiological condition that is typically characterized by unregulated cell growth in mammals. The cancer to be prevented or treated in the present disclosure may include, depending on the site of occurrence, colorectal cancer, small intestine cancer, rectal cancer, colon cancer, thyroid cancer, endocrine adenocarcinoma, oral cancer, tongue cancer, pharyngeal cancer, laryngeal cancer, esophageal cancer, cervical cancer, uterine cancer, fallopian tube cancer, ovarian cancer, brain cancer, head and neck cancer, lung cancer, lymph gland cancer, gallbladder cancer, bladder cancer, kidney cancer, liver cancer, pancreatic cancer, prostate cancer, skin cancer (or melanoma), breast cancer, stomach cancer, bone cancer, blood cancer, and the like. However, any cancer can be included therein as long as it expresses a CD300c protein on the surface of cancer cells. In an embodiment, the cancer may include at least any one selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer. In another embodiment, the cancer may be a solid cancer.

In an embodiment, the method may further comprise determining an expression level of a CD300c protein based on the subject's biological sample or data, prior to administration of the anti-CD300c antibody or an antigen-binding fragment thereof.

In addition, the method may comprise a determining that the subject is suitable for treatment with the anti-CD300c antibody or an antigen-binding fragment thereof in a case where the expression level of the CD300c protein determined using the subject's biological sample or data is higher than a predetermined level. Specifically, the method may comprise determining that the subject is suitable for treatment with the anti-CD300c antibody or an antigen-binding fragment thereof in a case where the expression level of the CD300c protein determined using the subject's biological sample or data is statistically significantly higher (for example, at least 10% higher) than that of a control (for example, the expression level in normal people who do not have cancer or the average expression level in cancer patients). However, a difference in the expression level of the CD300c protein as presented above is merely exemplary. The difference may be, but is not limited to, 20% or higher, 30% or higher, 40% or higher, 50% or higher, 60% or higher, or 70% or higher. Preferably, the control may be the average expression level in allogeneic cancer patients.

In an embodiment of the present disclosure, using data of patients with kidney cancer (530 patients), pancreatic cancer (177 patients), and liver cancer (370 patients) obtained from The Cancer Genome Atlas (TCGA) database, comparison of overall survival depending on the CD300c expression level was performed. As a result, relative to the average CD300c expression level for each cancer type, the cancer patients with a higher CD300c expression level had shorter overall survival as compared with those with a lower CD300c expression level. Therefore, it may be desirable to consult the expression level of a CD300c protein in a subject in order to increase a therapeutic response rate to the anti-CD300c antibody in the subject.

In another embodiment, the method may further comprise administering one or more immunotherapies. In a case where (i) the anti-CD300c antibody or an antigen-binding fragment thereof is used in combination with (ii) one or more immunotherapies, (i) and (ii) may be administered simultaneously or sequentially.

"Administered sequentially" means that one ingredient is administered first and the other ingredient is administered immediately or at a predetermined interval after the first administration, wherein the ingredients may be administered in any order. That is, the anti-CD300c antibody or an antigen-binding fragment thereof may be administered first and one or more immunotherapies may be administered immediately or at a predetermined interval after the first administration, or *vice versa.* In addition, any of the one or more immunotherapies may be administered first, followed by the anti-CD300c antibody or an antigen-binding fragment thereof, and then the other of the one or more immunotherapies."

In another embodiment, the anti-CD300c antibody or an antigen-binding fragment thereof may be administered in combination with two or more immunotherapies. In one example, it was identified that the highest cancer cell proliferation inhibitory effect was observed in a case where the anti-CD300c antibody or an antigen-binding fragment thereof was used in combination with two immunotherapies (for example, an anti-PD-L1 antibody and an anti-PD-1 antibody, or an anti-PD-1 antibody and an anti-CTLA-4 antibody).

Each of the antibody or an antigen-binding fragment thereof according to the present disclosure and optionally one or more additional immunotherapies may be administered in several ways depending on whether local or systemic treatment is desired and the area to be treated. Methods of administering these ingredients to a subject may vary depending on the purpose of administration, the site of the disease, the subject's condition, and the like. The route of administration may be oral, parenteral, inhalation, local or topical (for example, intralesional administration). For example, parenteral administration may include, but is not limited to, intravenous, subcutaneous, intraperitoneal, intrapulmonary, intraarterial, intramuscular, rectal, vaginal, intraarticular, intraprostatic, intranasal, intraocular, intravesical, intrathecal, or intraventricular administration (for example, intracerebroventricular administration). In addition, when used in combination, the anti-CD300c antibody and the additional immunotherapy may be administered by the same route or may be administered by different routes.

In the method, an effective amount of each of the anti-CD300c antibody or an antigen-binding fragment thereof according to the present disclosure and optionally one or more additional cancer therapies may vary depending on the age, sex, and body weight of an individual (patient). In general, administration may be performed in an amount of about 0.01 mg to about 100 mg, or about 5 mg to about 50 mg, per kg of body. The amount may be administrated once a day or several times a day in divided doses. However, the effective amount may be increased or decreased depending on the route and period of administration, severity of disease, sex, body weight, age, and the like. Thus, the scope of the present disclosure is not limited thereto.

The method according to the present disclosure may comprise a identifying an expression level of a CD300c protein from the subject in advance. Depending on the expression level, determination may be made on whether to administer the anti-CD300c antibody or an antigen-binding fragment thereof.

In another embodiment, the method according to the present disclosure may comprise a measuring changes in expression level of a specific marker after administration of the anti-CD300c antibody or an antigen-binding fragment thereof to a subject, so as to select an additional (at least one) immunotherapy suitable for use in combination with the anti-CD300c antibody or an antigen-binding fragment thereof.

Specifically, the method may further comprise a determining the expression level of one or more markers selected from the following markers, using a biological sample or data from the subject to which the anti-CD300c antibody or an antigen-binding fragment thereof has been received:
Bst2, Cd40, Cd70, Cd86, Ccl8, Xcl1, Ccr7, Cd80, Cd206, Msr1, Arg1, Vegfa, Pdgfrb, Col4a1, Hif1a, Vcam1, Icam1, Gzma, Gzmb, Icos, Cd69, Ifng, Tnf, Cd1d1, Cd1d2, Cd38, Cxcr6, Xcr1, Tbx21, Stat1, Stat4, Cxcr3, IL-12b, IL-4, IL-6, IL-13, PD-1, PD-L1, CTLA-4, Lag3, Tim3, Ox40, Gitr, Hvem, CD27, CD28, Cma1, Timd4, Bcl6, Cxcl5, and Ccl21a.

See Table 2 for description of the markers.

**[Table 2]**

| No. | Name | Full name | Brief description |
|---|---|---|---|
| 1 | Bst2 | bone marrow stromal antigen 2 | This is also known as Tetherin or CD137, and is a lipid raft associated protein encoded by the BST2 gene. |
| 2 | CD40 | Cluster of differentiation 40 | This is a co-stimulatory protein found in antigen-presenting cells and is required for their activation. The binding of CD154 (CD40L) on TH cells to CD40 activates antigen presenting cells and induces a variety of downstream effects. |
| 3 | CD70 | Cluster of differentiation 70 | This is a protein encoded by the CD70 gene in humans. CD70 is a ligand for CD27. |
| 4 | CD86 | Cluster of differentiation 86 | This is also known as B7-2, and is a protein constitutively expressed in dendritic cells, Langerhans cells, macrophages, B-cells (including |
| | | | memory B-cells), and in other antigen-presenting cells. |
| 5 | Ccl8 | Chemokine (C-C motif) ligand 8 | This is also known as monocyte chemoattractant protein 2 (MCP2) and is a protein encoded by the CCL8 gene in humans. |
| 6 | Xcl1 | X-C Motif Chemokine Ligand 1 | This is a small cytokine belonging to the C chemokine family that is also known as lymphotactin. |
| 7 | Ccr7 | C-C chemokine receptor type 7 | This is a protein encoded by the CCR7 gene in humans. The following two ligands have been identified for this receptor: chemokine (C-C motif) ligand19 (CCL19/ELC) and (C-C motif) ligand 21 (CCL21). |
| 8 | CD80 | Cluster of differentiation 80 | This is a B7, type 1 membrane protein in the immunoglobulin superfamily, with an extracellular immunoglobulin constant-like domain and a variable-like domain required for receptor binding. It is closely related to CD86, another B7 protein (B7-2), and often works in tandem, binding to the same receptors to prime T cells. |
| 9 | CD20 6 | Cluster of differentiation 206 | CD206 is also known as the mannose receptor. This is a 206 C-type lectin primarily present on the surface of macrophages, immature dendritic cells, and liver sinusoidal endothelial cells, but is also expressed on the surface of skin cells such as human dermal fibroblasts and keratinocytes. |
| 10 | Msr1 | Macrophage scavenger receptor 1 | This is a protein encoded by the scavenger MSR1 gene in humans. MSR1 is receptor 1 also called cluster of differentiation 204 (CD204). |
| 11 | Arg1 | Arginase 1 | The human ARG1 gene encodes a protein arginase. |
| 12 | Vegfa | Vascular endothelial growth factor A | This is a protein encoded by the VEGFA gene in humans. |
| 13 | Pdgfrb | Platelet-derived growth factor receptor beta | This is a protein encoded by the PDGFRB gene in humans. |
| 14 | Col4a 1 | Collagen, type IV, alpha 1 | This is a protein encoded by the COL4A1 gene on chromosome in humans. |
| 15 | Hif1a | Hypoxia-inducible factor 1-alpha | This is also known as HIF-1-alpha and is a subunit of a hetero-dimeric transcription factor hypoxia-inducible factor 1 (HIF-1) encoded by the HIF1A gene. |
| 16 | Vcam 1 | Vascular cell adhesion protein 1 | This is also known as vascular cell adhesion molecule 1 (VCAM-1) protein 1 or cluster of differentiation 106 (CD106) and is a protein encoded by the VCAM1 gene in humans. |
| 17 | Icam1 | Intercellular Adhesion Molecule 1 | This is also known as CD54 Adhesion (Cluster of Differentiation 54) and is a protein encoded by the ICAM1 gene in humans. |
| 18 | Gzma | Granzyme A | This is encoded by the Gzma gene and is present in cytotoxic T lymphocyte granules. |
| 19 | Gzmb | Granzyme B | This is encoded by the Gzmb gene and is expressed by cytotoxic T lymphocytes (CTL) and natural killer (NK) cells. |
| 20 | Icos | Inducible T-cell Co-Stimulator | This is an immune checkpoint protein encoded by the ICOS gene in humans. |
| 21 | Cd69 | Cluster of Differentiation 69 | This is a human transmembrane C-Type lectin protein encoded by the CD69 gene. It is an early activation marker expressed in hematopoietic stem cells, T cells, and the like. |
| 22 | Ifng | Interferon gamma | This is a dimerized soluble that is the only member of the type II class of interferons. |
| 23 | Tnf | Tumor Necrosis Factor | This is encoded by the Tnf gene and is a multifunctional pro-inflammatory cytokine that belongs to the tumor necrosis factor (TNF) superfamily and is secreted primarily by macrophages. |
| 24 | Cd1d1 | CD1d1 antigen | This enables T cell receptor binding activity and endogenous lipid antigen binding activity, and is involved in differentiation of natural killer T (NKT) cells and regulation of immature T cell proliferation. |
| 25 | Cd1d2 | CD1d2 antigen | This encodes an MHC class I-like molecule involved in presentation of lipid antigens to T cells, and is involved in activation of natural killer T cells. |
| 26 | Cd38 | Cluster of Differentiation 38 | This is a glycoprotein found on the surface of many immune cells (white blood cells), including CD4+, CD8+, B lymphocytes, and natural killer cells. |
| 27 | Cxcr6 | C-X-C chemokine receptor type 6 | This is also called CD 186 and has been identified as an entry coreceptor used by HIV-1 and SIV to enter target cells, in conjunction with CD4. |
| 28 | Xcr1 | X-C motif chemokine receptor 1 | This is a chemokine receptor belonging to the G protein-coupled receptor superfamily. |
| 29 | Tbx21 | T-box transcription factor TBX21 | This is also called T-box transcription expressed in T cells (T-bet) and is a protein encoded TBX21 by the TBX21 gene in humans. |
| 30 | Stat1 | Signal transducer and activator of transcription 1 | This is a transcription factor encoded by the activator of STAT1 gene in humans. It is a member of the STAT protein family. |
| 31 | Stat4 | Signal transducer and activator of transcription 4 | This is a transcription factor belonging to the STAT protein family. |
| 32 | Cxcr3 | C-X-C Motif Chemokine Receptor 3 | This is a Gαᵢ protein-coupled receptor in the CXC chemokine receptor family. |
| 33 | IL-12b | Subunit beta of interleukin 12 | This is also known as IL-12B, natural killer cell stimulatory factor 2, cytotoxic lymphocyte maturation factor p40, or interleukin-12 subunit p40, and is a protein encoded by the IL12B gene in humans. |
| 34 | IL4 | Interleukin 4 | This is a cytokine that induces differentiation of naive helper T cells (Th0 cells) to Th2 cells. Upon activation by IL-4, Th2 cells subsequently produce additional IL-4 in a positive feedback loop. |
| 35 | IL6 | Interleukin 6 | This is an interleukin that acts as both a pro-inflammatory cytokine and an anti-inflammatory myokine. In humans, it is encoded by the IL6 gene. |
| 36 | IL13 | Interleukin 13 | This is a protein encoded by the IL13 gene in humans. |
| 37 | PD-1 | Programmed cell death protein 1 | This is also known as cluster of differentiation 279 (CD279), and is a protein that has a role in regulating the immune system's response to the cells of the human body by down-regulating the immune system and promoting self-tolerance by suppressing T cell inflammatory activity. It prevents autoimmune diseases, but it can also prevent the immune system from killing cancer cells. |
| 38 | PD-L1 | Programmed death-ligand 1 | This is also known as cluster of death-differentiation 274 (CD274) or B7 homolog 1 (B7-H1), and is a protein encoded by the CD274 gene in humans. |
| 39 | CTLA-4 | cytotoxic T-lymphocyte-associated protein 4 | This is also known as cluster of differentiation 152 (CD152), and is a protein receptor that functions as an immune checkpoint and downregulates immune responses. |
| 40 | Lag3 | Lymphocyte-activation gene 3 | This is a protein encoded by the LAG3 gene in humans. |
| 41 | Tim3 | immunoglobulin and mucin-domain containing-3 | This is also known as hepatitis A virus cellular receptor 2 (HAVCR2), and is a protein encoded by the HAVCR2 gene in humans. |
| 42 | Ox40 | (CD134, TNFRSF4) | This is a member of the TNFR-superfamily of receptors which is not constitutively expressed on resting naive T cells, unlike CD28. |
| 43 | Gitr | glucocorticoid-induced tumor necrosis factor receptor | This is a protein encoded by the TNFRSF18 gene in humans. |
| 44 | Hvem | Herpesvirus entry mediator | This is a human cell surface receptor of the TNF-receptor superfamily. |
| 45 | CD27 | Cluster of differentiation 27 | This is a member of the tumor necrosis factor receptor superfamily. |
| 46 | CD28 | Cluster of differentiation 28 | This is one of the proteins expressed on T cells that provide co-stimulatory signals required for T cell activation and survival. |
| 47 | Cma1 | Chymase 1 | This is an enzyme encoded by the CMA1 gene in humans. |
| 48 | Timd4 | T-cell immunoglobulin and mucin domain containing 4 | This is also known as T-cell membrane protein 4 (TIM-4) and is a protein encoded by the TIMD4 gene in humans. |
| 49 | Bcl6 | B-cell lymphoma 6 protein | This is a protein encoded by the BCL6 gene in humans. Like BCL2, BCL3, BCL5, BCL7A, BCL9, and BCL10, it has clinical significance in lymphoma. |
| 50 | Cxcl5 | C-X-C Motif Chemokine Ligand 5 | This is a protein encoded by the CXCL5 gene in humans. |
| 51 | Ccl21 a | Chemokine (C-C motif) ligand 21 | This is a small cytokine belonging to the CC chemokine family. |

In yet another embodiment, the method may further comprise selecting an additional immunotherapy based on the determined expression level of the marker. Here, the marker may include, but is not limited to, PD-1, PD-L1, CTLA-4, Lag3, Tim3, Icos, Ox40, Gitr, Hvem, CD27, and CD28. In still yet another embodiment, the marker may include at least one selected from the group consisting of PD-1, PD-L1, CTLA-4, Lag3, Tim3, Icos, Ox40, Gitr, Hvem, CD27, and CD28. Preferably, the marker may include at least one selected from the group consisting of PD-1, PD-L1, CTLA-4, Lag3, and Tim3. In addition, the marker may include at least one selected from the group consisting of Icos, Ox40, Gitr, Hvem, CD27, and CD28.

Changes in expression levels of these markers mean changes in tumor/immune-related markers, which affect tumor suppressive effects, observed in a case where administration of the anti-CD300c antibody or an antigen-binding fragment thereof of the present disclosure is administered to an individual. For example, the changes in expression levels of the markers may include changes in expression pattern of protein markers or immune checkpoint protein markers related to activity of immune cells (for example, dendritic cells, macrophages, T cells, NKT cells), tumor microenvironment (TME) protein markers that affect tumor proliferation, and markers related to Th1 and Th2 responses. For specific examples of the markers, reference is made to the description as shown above. Through these changes in expression pattern, it is possible to predict the probability that a patient will respond to a drug or to select a cancer therapy, including another immune checkpoint inhibitor, which can maximize an anticancer effect. In addition, utilization of the markers makes it possible to determine whether a patient can be treated with the antibody. In addition, it is possible to monitor therapeutic effects of a drug. In addition, it is possible to provide information for treatment methods, including drug doses, usages, combination therapies, and the like.

According to an embodiment of the present disclosure, it was identified that an enhanced antitumor effect was exhibited in a case where the anti-CD300c antibody or an antigen-binding fragment thereof of the present disclosure was used in combination with another immune checkpoint inhibitor (at least one of durvalumab (IMFINZIO) that is an anti-PD-L1 antibody, nivolumab (OPDIVOO) that is an anti-PD-1 antibody, pembrolizumab (KEYTRUDA^{®}) that is an anti-PD-1 antibody, an anti-CTLA-4 antibody, and an anti-CD47 antibody (αCD47)) which is selected by changes in expression levels of the markers in an individual observed after administration of the anti-CD300c antibody or an antigen-binding fragment thereof.

In still yet another embodiment, the method may further comprise determining therapeutic responsiveness of the anti-CD300c antibody or an antigen-binding fragment thereof based on the determined expression level of the marker. The marker may include, but is not limited to, vegfa, pdgfrb, Col4a1, Hif1a, Bst2, CCL8, Xcl1, CCR7, CD80, Tbx21, Stat1, Stat4, Ifng, Cxcr3, IL-6, Gzma, Icos, Cd69, Cd1d1, Cd38, Cxcr6, Ox40, Gitr, CD27, and CD28. Preferably, the marker may include at least one selected from the group consisting of vegfa, pdgfrb, Col4a1, Hif1a, Bst2, CCL8, Xcl1, CCR7, CD80, Tbx21, Stat1, Stat4, Ifng, Cxcr3, IL-6, Gzma, Icos, Cd69, Cd1d1, Cd38, and Cxcr6. In addition, the marker may include at least one selected from the group consisting of vegfa, pdgfrb, Col4a1, and Hif1a. Additionally, the marker may include at least one selected from the group consisting of Bst2, CCL8, and Xcl1. In still yet another embodiment, the marker may include CCR7, CD80, or a combination thereof. In addition, the marker may include at least one selected from the group consisting of Tbx21, Stat1, Stat4, Ifng, Cxcr3, and IL-6.

In still yet another embodiment, the method may further comprise determining that the therapeutic responsiveness of the anti-CD300c antibody or an antigen-binding fragment thereof is good or excellent in a case where the expression level of at least one of the markers is decreased as compared with a subject having not received the anti-CD300c antibody or an antigen-binding fragment thereof. For example, according to the method, it may be determined that the therapeutic responsiveness of the anti-CD300c antibody or an antigen-binding fragment thereof is good or excellent in a case where the expression level of at least one marker selected from the group consisting of vegfa, pdgfrb, Col4a1, Hif1a, and IL-6 is decreased as compared with a subject having not received the anti-CD300c antibody or an antigen-binding fragment thereof. Here, the decrease in expression level means a statistically significant decrease. A decrease rate in expression level may include, but is not limited to, about 10% or higher, about 20% or higher, about 30% or higher, about 40% or higher, about 50% or higher, about 60% or higher, about 70% or higher, and about 100% or higher.

In addition, the method may further comprise determining that the therapeutic responsiveness of the anti-CD300c antibody or an antigen-binding fragment thereof is good or excellent in a case where the expression level of at least one of the markers is increased as compared with a subject having not received the anti-CD300c antibody or an antigen-binding fragment thereof. For example, according to the method, it may be determined that the therapeutic responsiveness of the anti-CD300c antibody or an antigen-binding fragment thereof is good or excellent in a case where the expression level of at least one marker selected from the group consisting of Bst2, CCL8, Xcl1, CCR7, CD80, Tbx21, Stat1, Stat4, Ifng, Cxcr3, Gzma, Icos, Cd69, Cd1d1, Cd38, Cxcr6, Ox40, Gitr, Cd27, and Cd28 is increased as compared with a subject having not received the anti-CD300c antibody or an antigen-binding fragment thereof. Here, the increase in expression level means a statistically significant increase. An increase rate in expression level may include, but is not limited to, about 10% or higher, about 20% or higher, about 30% or higher, about 40% or higher, about 50% or higher, about 60% or higher, about 70% or higher, and about 100% or higher.

### Pharmaceutical Composition

According to still yet another aspect of the present disclosure, there is provided a pharmaceutical composition for preventing or treating cancer, comprising, as an active ingredient, the anti-CD300c antibody or an antigen-binding fragment thereof.

The anti-CD300c antibody or an antigen-binding fragment thereof may be included in the composition in a prophylactically or therapeutically effective amount. The pharmaceutical composition may be administered to a subject to inhibit proliferation, survival, metastasis, recurrence, or therapy resistance of cancer.

In an embodiment, the pharmaceutical composition may further comprise at least one additional immunotherapy. Specifically, the anti-CD300c antibody or an antigen-binding fragment thereof and optionally the additional immunotherapy may be included in the same composition or may be included in separate compositions. When included in separate compositions, the anti-CD300c antibody or an antigen-binding fragment thereof and the additional immunotherapy may be formulated respectively, and may be administered simultaneously or sequentially.

To prepare the pharmaceutical composition of the present disclosure, the antibody or an antigen-binding fragment thereof and optionally the additional immunotherapy may be mixed with a pharmaceutically acceptable carrier and/or excipient. The pharmaceutical composition may be prepared in the form of a lyophilized preparation or an aqueous solution. For example, please refer to Remington's Pharmaceutical Sciences and US Pharmacopeia: National Formulary, Mack Publishing Company, Easton, Pa. (1984).

Acceptable carriers and/or excipients (including stabilizers) are non-toxic to recipients at the dosages and concentrations employed, and include, but are not limited to, buffers (such as phosphate, citrate, and other organic acids); antioxidants (such as ascorbic acid and methionine); preservatives (such as octadecyldimethylbenzyl ammonium chloride; hexamethonium chloride; benzalkonium chloride, benzethonium chloride; phenol, butyl or benzyl alcohol; alkyl parabens (such as methyl and propyl paraben); catechol; resorcinol; cyclohexanol; 3-pentanol; and m-cresol); low molecular weight (about less than about 10 residues) polypeptides; proteins (such as serum albumin, gelatin, and immunoglobulins); hydrophilic polymers (such as polyvinylpyrrolidone); amino acids (such as glycine, glutamine, asparagine, histidine, arginine, and lysine); monosaccharides, disaccharides, and other carbohydrates (such as glucose, mannose, and dextrins); chelating agents (such as EDTA); sugars (such as sucrose, mannitol, trehalose, and sorbitol); salt-forming counter-ions (such as sodium); metal complexes (such as Zn-protein complexes); and(or) non-ionic surfactants (such as TWEEN^{™}, PLURONICS^{™}, and polyethylene glycol (PEG)).

The pharmaceutical composition of the present disclosure may be formulated in a suitable form known in the art depending on the route of administration.

As used herein, the term "prophylactically or therapeutically effective amount" or "effective amount" refers to an amount of an active ingredient in a composition which is effective for preventing or treating cancer in a subject. This amount is also sufficient for preventing or treating cancer at a reasonable benefit/risk ratio applicable to medical treatment and does not cause adverse effects. A level of the effective amount may be determined depending on the patient's health status, type of disease, severity of disease, activity of the drug, sensitivity to the drug, method of administration, frequency of administration, route of administration and rate of excretion, duration of treatment, drugs used in combination or coincidentally therewith, and other factors well known in the medical field. Here, it is important to administer a minimum amount that allows the maximum effect to be achieved with minimal or no adverse effects in consideration of all of the above factors, which can be easily determined by those skilled in the art.

Specifically, the effective amount of each active ingredient in the pharmaceutical composition of the present disclosure may vary depending on the age, sex, and body weight of an individual (patient). In general, about 0.01 mg to about 100 mg, or about 5 mg to about 50 mg, per kg of body, may be administered once a day or several times a day in divided doses. However, the scope of the present disclosure is not limited thereto since the effective amount may be increased or decreased depending on the route and duration of administration, severity of disease, sex, body weight, age, and the like.

### Kits for Prevention or Treatment of Cancer

According to still yet another aspect of the present disclosure, there is provided a kit for preventing or treating cancer, comprising a composition that comprises the anti-CD300c antibody or an antigen-binding fragment thereof according to the present disclosure, and an instruction that directs use of the antibody or an antigen-binding fragment thereof. Here, the composition may contain a prophylactically or therapeutically effective amount of the anti-CD300c antibody or an antigen-binding fragment thereof.

In an embodiment, the instruction may include an instruction that directs combined use of the antibody or an antigen-binding fragment thereof and at least one additional cancer therapy.

In an embodiment, the instruction may include an instruction that contains a guideline on how to take or administer the active ingredient(s). For example, the instruction may include an instruction that directs measurement of an expression level of a CD300c protein using a biological sample or data obtained from the subject prior to administration of the antibody or an antigen-binding fragment thereof. Optionally, an instrument or device necessary for administering the active ingredient(s) may be included in the kit.

### Doses

An effective amount or effective non-toxic amount of each of the anti-CD300c antibody or an antigen-binding fragment thereof according to the present disclosure, and optionally the additional immunotherapy, can be determined by routine experimentation. For example, a therapeutically active amount of the antibody or the immunotherapy may vary depending on factors such as stage of disease, severity of disease, the subject's age, sex, medical complications, and body weight, and the ingredient's capacity to elicit a desired response in the subject, and dosage of the concurrently used cancer therapy. The dose and dosing regimen of each of the anti-CD300c antibody or an antigen-binding fragment thereof or the additional immunotherapy may be adjusted to provide an optimal therapeutic response. For example, several divided doses may be administered daily, weekly, every 2 weeks, every 3 weeks, every 4 weeks, or the like, and/or the doses may be proportionally decreased or increased depending on urgency of therapeutic situation.

### Methods and Kits for Providing Information for Prevention or Treatment of Cancer

According to still yet another aspect of the present disclosure, there is provided a method for providing information for prevention or treatment of cancer, comprising of determining an expression level of a CD300c protein using a biological sample or data obtained from a subject in need of prevention or treatment of cancer. The method may also be utilized for pre-screening for administration of the anti-CD300c antibody or an antigen-binding fragment thereof.

In an embodiment, the determining the expression level of the CD300c protein (marker) may include using a molecule that specifically binds to the CD300c protein, such as an antibody, a substrate, a ligand, or a cofactor, or a molecule that specifically binds to the mRNA of CD300c, such as a primer pair or a probe. Methods for determining the expression level using these reagents are well known in the art, including those as described above. In an embodiment, the method may comprise determining that the subject is suitable for treatment with the anti-CD300c antibody or an antigen-binding fragment thereof in a case where the expression level of the CD300c protein determined using the subject's biological sample or data is higher than a predetermined level. Specifically, the method may comprise determining that the subject is suitable for treatment with the anti-CD300c antibody or an antigen-binding fragment thereof in a case where the expression level of the CD300c protein determined using the subject's biological sample or data is statistically significantly higher (for example, at least 10% higher) than a control (for example, the expression level in normal people who do not have cancer or the average expression level in cancer patients). However, the difference in the expression level of the CD300c protein as presented above is merely exemplary. The difference may be, but is not limited to, 20% or higher, 30% or higher, 40% or higher, 50% or higher, 60% or higher, or 70% or higher. Preferably, the control may be the average expression level in allogeneic cancer patients.

In an embodiment of the present disclosure, using data of patients with kidney cancer (530 patients), pancreatic cancer (177 patients), and liver cancer (370 patients) obtained from The Cancer Genome Atlas (TCGA) database, comparison of overall survival depending on the CD300c expression level was performed. As a result, relative to the average CD300c expression level for each cancer type, the cancer patients with a higher CD300c expression level had shorter overall survival as compared with those with a lower CD300c expression level. Therefore, it may be desirable to consult the expression level of a CD300c protein in a subject in order to increase a therapeutic response rate to the anti-CD300c antibody in the subject.

In another embodiment, the information for prevention or treatment of cancer may include, but is not limited to, information on any one or more of therapeutic responsiveness of a therapeutic agent associated with a CD300c protein (for example, an anti-CD300c antibody or an antigen-binding fragment thereof), selection of a therapeutic agent, selection of a subject to be treated, prognosis of a subject, and survival of a subject. Preferably, the information for prevention or treatment of cancer may include cancer therapeutic responsiveness of the anti-CD300c antibody or an antigen-binding fragment thereof, survival of a subject, or both.

According to still yet another aspect of the present disclosure, there is provided a kit for providing information for prevention or treatment of cancer, comprising a substance for measuring an expression level of a CD300c protein using a biological sample or data obtained from a subject in need of prevention or treatment of cancer. The kit may comprise one or more other component compositions, solutions, or devices for carrying out the assay. The kit may be a kit for measuring an expression level of a protein marker, for example, an enzyme-linked immunosorbent assay (ELISA) kit. The kit may comprise other reagents known in the art as necessary for immunological detection of antibodies. The kit may further comprise a pharmaceutical composition that comprises, as an active ingredient, the anti-CD300c antibody or an antigen-binding fragment thereof.

### [DETAILED DESCRIPTION OF THE INVENTION]

Hereinafter, the present disclosure will be described in more detail by way of examples. However, the following examples are only for illustrating the present disclosure, and the scope of the present disclosure is not limited thereto.

### EXAMPLES

### I. Production of Anti-CD300c Monoclonal Antibody

### Example 1. Production of Anti-CD300c Monoclonal Antibody

### Example 1.1. Construction of Anti-CD300c Monoclonal Antibody Library

In order to select anti-CD300c monoclonal antibodies, biopanning was performed using a lambda phage library, a kappa phage library, a VH3VL1 phage library, and an OPALTL phage library. More specifically, a CD300c antigen was added at a concentration of 5 µg/mL to an immunotube, and reaction was allowed to proceed for 1 hour so that the antigen was adsorbed on the surface of the immunotube. 3% skim milk was added to suppress non-specific reactions. Then, 10¹² PFU of the antibody phage library dispersed in 3% skim milk was added to each immunotube for antigen binding. Washing was performed 3 times using Tris buffered saline-Tween 20 (TBST) solution to remove non-specifically bound phages, and then single-chain variable fragment (scFv) phage antibodies, which were specifically bound to the CD300c antigen, were eluted using 100 mM triethylamine solution. The eluted phages were neutralized using 1.0 M Tris-HCl buffer (pH 7.8). Then, the resultant was subjected to *E. coli* ER2537 and infection was allowed to proceed at 37°C for 1 hour. The infected *E. coli* was applied onto LB agar medium containing carbenicillin, and cultured at 37°C for 16 hours. Then, the formed *E. coli* colonies were suspended using 3 mL of a super broth (SB)-carbenicillin culture. Some of the suspension was stored at -80°C until use with the addition of 15% glycerol, and the remaining portion was reinoculated into SB-carbenicillin-2% glucose solution and cultured at 37°C. Then, the obtained culture was centrifuged, and biopanning was again repeated 3 times using the supernatant containing phage particles to obtain and concentrate antigen-specific antibodies.

After repeating the biopanning 3 times, *E. coli* containing the antibody gene was applied onto LB agar medium containing carbenicillin and cultured at 37°C for 16 hours. The formed *E. coli* colonies were inoculated again into SB-carbenicillin-2% glucose solution and cultured at 37°C. until the absorbance (at OD 600 nm) reached 0.5. Then, IPTG was added and further cultured at 30°C for 16 hours. Thereafter, periplasmic extraction was performed. From the results, a library pool of antibodies, which specifically bind to the CD300c antigen, was primarily obtained.

### Example 1.2. Selection of Anti-CD300c Monoclonal Antibody

In order to select anti-CD300c monoclonal antibodies that specifically bind, with high binding affinity, to a CD300c antigen, ELISA was performed using the library pool obtained in the same manner as in Example 1.1. More specifically, each of a CD300c antigen and a CD300a antigen in a coating buffer (0.1 M sodium carbonate, pH 9.0) was dispensed onto an ELISA plate at a concentration of 5 µg/mL per well, and then reaction was allowed to proceed at room temperature for 3 hours so that the antigen could bind to the plate. Washing was performed 3 times using phosphate buffered saline-Tween 20 (PBST) to remove unbound antigens, and then 350 µL of PBST supplemented with 2% bovine serum albumin (BSA) was added to each well. The reaction was allowed to proceed at room temperature for 1 hour, and washing was performed again using PBST. Then, 25 µg of a periplasmic extract containing scFv obtained in the same manner as in Example 1.1 was added thereto, and the reaction was allowed to proceed for 1 hour at room temperature for antigen binding. After 1 hour, washing was performed 3 times using PBST to remove unbound scFvs, and then 4 µg/mL of an antibody for detection was added. The reaction was allowed to proceed again at room temperature for 1 hour. Subsequently, the unbound antibodies for detection were removed using PBST. Then, anti-rabbit IgG to which HRP was bound was added and the reaction was allowed to proceed at room temperature for 1 hour. The unbound antibodies were removed again using PBST. Subsequently, 3,3',5,5'-tetramethylbenzidine (TMB) solution was added and the reaction was allowed to proceed for 10 minutes for development. Then, 2 N sulfuric acid solution was added to terminate the development, and the absorbance was measured at 450 nm to identify the antibodies that specifically bind to the CD300c antigen.

### Example 1.3. Identification of Anti-CD300c Monoclonal Antibody Sequences

The nucleotide sequences of the anti-CD300c monoclonal antibodies, which were selected using the same method as in Example 1.2, were identified. More specifically, for each of the selected antibody clones, plasmid DNA was extracted therefrom using a plasmid miniprep kit. Then, DNA sequencing was performed to analyze complementarity-determining region (CDR) sequences. As a result, 25 types of anti-CD300c monoclonal antibodies having different amino acid sequences were obtained. The heavy chain and light chain variable regions of these 25 anti-CD300c monoclonal antibodies are shown in Tables 3 and 4 below.

**Table 3**

| Antibody name | Source (phage library) | Heavy chain variable region (nucleotide) | Light chain variable region (nucleotide) | Heavy chain variable region (amino acid) | Light chain variable region (amino acid) |
|---|---|---|---|---|---|
| CK1 | Kappa | FIG. 1aa (SEQ ID: 301) | FIG. 1ab (SEQ ID: 302) | FIG. 1ac (SEQ ID: 303) | FIG. 1ad (SEQ ID: 304) |
| CK2 | Kappa | FIG. 1 ba(SEQ ID: 305) | FIG. 1bb (SEQ ID: 306) | FIG. 1bc (SEQ ID: 307) | FIG. 1bd (SEQ ID: 308) |
| CK3 | Kappa | FIG. 1ca(SEQ ID: 309) | FIG. 1cb (SEQ ID: 310) | FIG. 1cc (SEQ ID: 311) | FIG. 1cd (SEQ ID: 312) |
| CL4 | Lambda | FIG. 1da(SEQ ID: 313) | FIG. 1db (SEQ ID: 314) | FIG. 1dc (SEQ ID: 315) | FIG. 1dd (SEQ ID: 316) |
| CL5 | Lambda | FIG. | FIG. 1eb | FIG. 1ec | FIG. 1ed |
| | | 1ea(SEQ ID: 317) | (SEQ ID: 318) | (SEQ ID: 319) | (SEQ ID: 320) |
| CL6 | VH3VL1 | FIG. 1fa(SEQ ID: 321) | FIG. 1fb (SEQ ID: 322) | FIG. 1fc (SEQ ID: 323) | FIG. 1fd (SEQ ID: 324) |
| CL7 | VH3VL1 | FIG. 1ga (SEQ ID: 325) | FIG. 1gb (SEQ ID: 326) | FIG. 1gc (SEQ ID: 327) | FIG. 1gd (SEQ ID: 328) |
| CL8 | VH3VL1 | FIG. 1ha (SEQ ID: 329) | FIG. 1hb (SEQ ID: 330) | FIG. 1hc (SEQ ID: 331) | FIG. 1hd (SEQ ID: 332) |
| CL9 | VH3VL1 | FIG. 1ia (SEQ ID: 333) | FIG. 1ib (SEQ ID: 334) | FIG. 1 ic (SEQ ID: 335) | FIG. 1id (SEQ ID: 336) |
| CL10 | VH3VL1 | FIG. 1ja (SEQ ID: 337) | FIG. 1jb (SEQ ID: 338) | FIG. 1jc (SEQ ID: 339) | FIG. 1jd (SEQ ID: 340) |
| SK11 | Kappa | FIG. 1ka (SEQ ID: 341) | FIG. 1kb (SEQ ID: 342) | FIG. 1kc (SEQ ID: 343) | FIG. 1kd (SEQ ID: 344) |
| SK12 | Kappa | FIG. 1la (SEQ ID: 345) | FIG. 1lb (SEQ ID: 346) | FIG. 1lc (SEQ ID: 347) | FIG. 1ld (SEQ ID: 348) |
| SK13 | Kappa | FIG. 1ma (SEQ ID: 349) | FIG. 1mb (SEQ ID: 350) | FIG. 1mc (SEQ ID: 351) | FIG. 1md (SEQ ID: 352) |
| SK14 | Kappa | FIG. 1na (SEQ ID: 353) | FIG. 1nb (SEQ ID: 354) | FIG. 1nc (SEQ ID: 355) | FIG. 1nd (SEQ ID: 356) |
| SK15 | Kappa | FIG. 1oa (SEQ ID: 357) | FIG. 1ob (SEQ ID: 358) | FIG. 1oc (SEQ ID: 359) | FIG. 1od (SEQ ID: 360) |
| SK16 | Kappa | FIG. 1pa (SEQ ID: 361) | FIG. 1pb (SEQ ID: 362) | FIG. 1pc (SEQ ID: 363) | FIG. 1pd (SEQ ID: 364) |
| SK17 | Kappa | FIG. 1qa (SEQ ID: 365) | FIG. 1qb (SEQ ID: 366) | FIG. 1qc (SEQ ID: 367) | FIG. 1qd (SEQ ID: 368) |

**[Table 4]**

| Antibody name | Source (phage library) | Heavy chain variable region (nucleotid e) | Light chain variable region (nucleotid e) | Heavy chain variable region (amino acid) | Light chain variable region (amino acid) |
|---|---|---|---|---|---|
| SL18 | Lambda | FIG. 1ra (SEQ ID: 369) | FIG. 1rb (SEQ ID: 370) | FIG. 1rc (SEQ ID: 371) | FIG. 1rd (SEQ ID: 372) |
| CB301_H3L1 _A10 | VH3VL1 | FIG. 1sa (SEQ ID: 373) | FIG. 1sb (SEQ ID: 374) | FIG. 1sc (SEQ ID: 375) | FIG. 1sd (SEQ ID: 376) |
| CB301_H3L1 _A12 | VH3VL1 | FIG. 1ta (SEQ ID: 377) | FIG. 1tb (SEQ ID: 378) | FIG. 1tc (SEQ ID: 379) | FIG. 1td (SEQ ID: 380) |
| CB301_H3L1 _E6 | VH3VL1 | FIG. 1ua (SEQ ID: 381) | FIG. 1ub (SEQ ID: 382) | FIG. 1uc (SEQ ID: 383) | FIG. 1ud (SEQ ID: 384) |
| CB301_H3L1 _F4 | VH3VL1 | FIG. 1va (SEQ ID: 385) | FIG. 1vb (SEQ ID: 386) | FIG. 1vc (SEQ ID: 387) | FIG. 1vd (SEQ ID: 388) |
| CB301_H3L1 _G11 | VH3VL1 | FIG. 1wa (SEQ ID: 389) | FIG. 1 wb (SEQ ID: 390) | FIG. 1 wc (SEQ ID: 391) | FIG. 1 wd (SEQ ID: 392) |
| CB301_OPA LTL_B5 | OPALTL | FIG. 1xa (SEQ ID: 393) | FIG. 1xb (SEQ ID: 394) | FIG. 1xc (SEQ ID: 395) | FIG. 1xd (SEQ ID: 396) |
| CB301_OPA LTL_E6 | OPALTL | FIG. 1ya (SEQ ID: 397) | FIG. 1yb (SEQ ID: 398) | FIG. 1yc (SEQ ID: 399) | FIG. 1yd (SEQ ID: 400) |

In each of the drawings mentioned in Tables 3 and 4 above, the CDR regions (CDR1, CDR2, and CDR3) are underlined and appear sequentially (that is, CDR1 appears, followed by CDR2, and then CDR3). In addition, the CDR regions included in each drawing are represented by SEQ ID NOs as shown in Table 5:

**[Table 5]**

| Related drawing | Antibod y | Heavy chain/ Light chain | Amino acid/ nucleoti de | CDR1 | CDR2 | CDR3 |
|---|---|---|---|---|---|---|
| FIG. 1aa | CK1 | heavy chain | nucleoti de | SEQ ID: 1 | SEQ ID: 2 | SEQ ID: 3 |
| FIG. 1ab | | light chain | nucleoti de | SEQ ID: 4 | SEQ ID: 5 | SEQ ID: 6 |
| FIG. 1ac | | heavy chain | amino acid | SEQ ID: 7 | SEQ ID: 8 | SEQ ID: 9 |
| FIG. 1ad | | light chain | amino acid | SEQ ID: 10 | SEQ ID: 11 | SEQ ID: 12 |
| FIG. 1ba | CK2 | heavy chain | nucleoti de | SEQ ID: 13 | SEQ ID: 14 | SEQ ID: 15 |
| FIG. 1bb | | light chain | nucleoti de | SEQ ID: 16 | SEQ ID: 17 | SEQ ID: 18 |
| FIG. 1bc | | heavy chain | amino acid | SEQ ID: 19 | SEQ ID: 20 | SEQ ID: 21 |
| FIG. 1bd | | light chain | amino acid | SEQ ID: 22 | SEQ ID: 23 | SEQ ID: 24 |
| FIG. 1ca | CK3 | heavy chain | nucleoti de | SEQ ID: 25 | SEQ ID: 26 | SEQ ID: 27 |
| FIG. 1cb | | light chain | nucleoti de | SEQ ID: 28 | SEQ ID: 29 | SEQ ID: 30 |
| FIG. 1cc | | heavy chain | amino acid | SEQ ID: 31 | SEQ ID: 32 | SEQ ID: 33 |
| FIG. 1cd | | light chain | amino acid | SEQ ID: 34 | SEQ ID: 35 | SEQ ID: 36 |
| FIG. 1da | CL4 | heavy chain | nucleoti de | SEQ ID: 37 | SEQ ID: 38 | SEQ ID: 39 |
| FIG. 1db | | light chain | nucleoti de | SEQ ID: 40 | SEQ ID: 41 | SEQ ID: 42 |
| FIG. 1dc | | heavy chain | amino acid | SEQ ID: 43 | SEQ ID: 44 | SEQ ID: 45 |
| FIG. 1dd | | light chain | amino acid | SEQ ID: 46 | SEQ ID: 47 | SEQ ID: 48 |
| FIG. 1ea | CL5 | heavy chain | nucleoti de | SEQ ID: 49 | SEQ ID: 50 | SEQ ID: 51 |
| FIG. 1eb | | light chain | nucleoti de | SEQ ID: 52 | SEQ ID: 53 | SEQ ID: 54 |
| FIG. 1ec | | heavy chain | amino acid | SEQ ID: 55 | SEQ ID: 56 | SEQ ID: 57 |
| FIG. 1ed | | light chain | amino acid | SEQ ID: 58 | SEQ ID: 59 | SEQ ID: 60 |
| FIG. 1fa | CL6 | heavy chain | nucleoti de | SEQ ID: 61 | SEQ ID: 62 | SEQ ID: 63 |
| FIG. 1fb | | light chain | nucleoti de | SEQ ID: 64 | SEQ ID: 65 | SEQ ID: 66 |
| FIG. 1fc | | heavy chain | amino acid | SEQ ID: 67 | SEQ ID: 68 | SEQ ID: 69 |
| FIG. 1fd | | light chain | amino acid | SEQ ID: 70 | SEQ ID: 71 | SEQ ID: 72 |
| FIG. 1ga | CL7 | heavy chain | nucleoti de | SEQ ID: 73 | SEQ ID: 74 | SEQ ID: 75 |
| FIG. 1gb | | light chain | nucleoti de | SEQ ID: 76 | SEQ ID: 77 | SEQ ID: 78 |
| FIG. 1gc | | heavy chain | amino acid | SEQ ID: 79 | SEQ ID: 80 | SEQ ID: 81 |
| FIG. 1gd | | light chain | amino acid | SEQ ID: 82 | SEQ ID: 83 | SEQ ID: 84 |
| FIG. 1ha | CL8 | heavy chain | nucleoti de | SEQ ID: 85 | SEQ ID: 86 | SEQ ID: 87 |
| FIG. 1hb | | light chain | nucleoti de | SEQ ID: 88 | SEQ ID: 89 | SEQ ID: 90 |
| FIG. 1hc | | heavy chain | amino acid | SEQ ID: 91 | SEQ ID: 92 | SEQ ID: 93 |
| FIG. 1hd | | light chain | amino acid | SEQ ID: 94 | SEQ ID: 95 | SEQ ID: 96 |
| FIG. 1ia | CL9 | heavy chain | nucleoti de | SEQ ID: 97 | SEQ ID: 98 | SEQ ID: 99 |
| FIG. 1ib | | light chain | nucleoti de | SEQ ID: 100 | SEQ ID: 101 | SEQ ID: 102 |
| FIG. 1ic | | heavy chain | amino acid | SEQ ID: 103 | SEQ ID: 104 | SEQ ID: 105 |
| FIG. 1id | | light chain | amino acid | SEQ ID: 106 | SEQ ID: 107 | SEQ ID: 108 |
| FIG. 1ja | CL10 | heavy chain | nucleoti de | SEQ ID: 109 | SEQ ID: 110 | SEQ ID: 111 |
| FIG. 1jb | | light chain | nucleoti de | SEQ ID: 112 | SEQ ID: 113 | SEQ ID: 114 |
| FIG. 1jc | | heavy chain | amino acid | SEQ ID: 115 | SEQ ID: 116 | SEQ ID: 117 |
| FIG. 1jd | | light chain | amino acid | SEQ ID: 118 | SEQ ID: 119 | SEQ ID: 120 |
| FIG. 1ka | SK11 | heavy chain | nucleoti de | SEQ ID: 121 | SEQ ID: 122 | SEQ ID: 123 |
| FIG. 1kb | | light chain | nucleoti de | SEQ ID: 124 | SEQ ID: 125 | SEQ ID: 126 |
| FIG. 1kc | | heavy chain | amino acid | SEQ ID: 127 | SEQ ID: 128 | SEQ ID: 129 |
| FIG. 1kd | | light chain | amino acid | SEQ ID: 130 | SEQ ID: 131 | SEQ ID: 132 |
| FIG. 1la | SK12 | heavy chain | nucleoti de | SEQ ID: 133 | SEQ ID: 134 | SEQ ID: 135 |
| FIG. 1lb | | light chain | nucleoti de | SEQ ID: 136 | SEQ ID: 137 | SEQ ID: 138 |
| FIG. 1lc | | heavy chain | amino acid | SEQ ID: 139 | SEQ ID: 140 | SEQ ID: 141 |
| FIG. 1ld | | light chain | amino acid | SEQ ID: 142 | SEQ ID: 143 | SEQ ID: 144 |
| FIG. 1ma | SK13 | heavy chain | nucleoti de | SEQ ID: 145 | SEQ ID: 146 | SEQ ID: 147 |
| FIG. 1mb | | light chain | nucleoti de | SEQ ID: 148 | SEQ ID: 149 | SEQ ID: 150 |
| FIG. 1mc | | heavy chain | amino acid | SEQ ID: 151 | SEQ ID: 152 | SEQ ID: 153 |
| FIG. 1md | | light chain | amino acid | SEQ ID: 154 | SEQ ID: 155 | SEQ ID: 156 |
| FIG. 1na | SK14 | heavy chain | nucleoti de | SEQ ID: 157 | SEQ ID: 158 | SEQ ID: 159 |
| FIG. 1nb | | light chain | nucleoti de | SEQ ID: 160 | SEQ ID: 161 | SEQ ID: 162 |
| FIG. 1nc | | heavy chain | amino acid | SEQ ID: 163 | SEQ ID: 164 | SEQ ID: 165 |
| FIG. 1nd | | light chain | amino acid | SEQ ID: 166 | SEQ ID: 167 | SEQ ID: 168 |
| FIG. 1oa | SK15 | heavy chain | nucleoti de | SEQ ID: 169 | SEQ ID: 170 | SEQ ID: 171 |
| FIG. 1ob | | light chain | nucleoti de | SEQ ID: 172 | SEQ ID: 173 | SEQ ID: 174 |
| FIG. 1oc | | heavy chain | amino acid | SEQ ID: 175 | SEQ ID: 176 | SEQ ID: 177 |
| FIG. 1od | | light chain | amino acid | SEQ ID: 178 | SEQ ID: 179 | SEQ ID: 180 |
| FIG. 1pa | SK16 | heavy chain | nucleoti de | SEQ ID: 181 | SEQ ID: 182 | SEQ ID: 183 |
| FIG. 1pb | | light chain | nucleoti de | SEQ ID: 184 | SEQ ID: 185 | SEQ ID: 186 |
| FIG. 1pc | | heavy chain | amino acid | SEQ ID: 187 | SEQ ID: 188 | SEQ ID: 189 |
| FIG. 1pd | | light chain | amino acid | SEQ ID: 190 | SEQ ID: 191 | SEQ ID: 192 |
| FIG. 1qa | SK17 | heavy chain | nucleoti de | SEQ ID: 193 | SEQ ID: 194 | SEQ ID: 195 |
| FIG. 1qb | | light chain | nucleoti de | SEQ ID: 196 | SEQ ID: 197 | SEQ ID: 198 |
| FIG. 1qc | | heavy chain | amino acid | SEQ ID: 199 | SEQ ID: 200 | SEQ ID: 201 |
| FIG. 1qd | | light chain | amino acid | SEQ ID: 202 | SEQ ID: 203 | SEQ ID: 204 |
| FIG. 1ra | SL18 | heavy chain | nucleoti de | SEQ ID: 205 | SEQ ID: 206 | SEQ ID: 207 |
| FIG. 1rb | | light chain | nucleoti de | SEQ ID: 208 | SEQ ID: 209 | SEQ ID: 210 |
| FIG. 1rc | | heavy chain | amino acid | SEQ ID: 211 | SEQ ID: 212 | SEQ ID: 213 |
| FIG. 1rd | | light chain | amino acid | SEQ ID: 214 | SEQ ID: 215 | SEQ ID: 216 |
| FIG. 1sa | CB301_ H3L1_A 10 | heavy chain | nucleoti de | SEQ ID: 217 | SEQ ID: 218 | SEQ ID: 219 |
| FIG. 1sb | | light chain | nucleoti de | SEQ ID: 220 | SEQ ID: 221 | SEQ ID: 222 |
| FIG. 1sc | | heavy chain | amino acid | SEQ ID: 223 | SEQ ID: 224 | SEQ ID: 225 |
| FIG. 1sd | | light chain | amino acid | SEQ ID: 226 | SEQ ID: 227 | SEQ ID: 228 |
| FIG. 1ta | CB301_ H3L1_A 12 | heavy chain | nucleoti de | SEQ ID: 229 | SEQ ID: 230 | SEQ ID: 231 |
| FIG. 1tb | | light chain | nucleoti de | SEQ ID: 232 | SEQ ID: 233 | SEQ ID: 234 |
| FIG. 1tc | | heavy chain | amino acid | SEQ ID: 235 | SEQ ID: 236 | SEQ ID: 237 |
| FIG. 1td | | light chain | amino acid | SEQ ID: 238 | SEQ ID: 239 | SEQ ID: 240 |
| FIG. 1ua | CB301_ H3L1_E 6 | heavy chain | nucleoti de | SEQ ID: 241 | SEQ ID: 242 | SEQ ID: 243 |
| FIG. 1ub | | light chain | nucleoti de | SEQ ID: 244 | SEQ ID: 245 | SEQ ID: 246 |
| FIG. 1uc | | heavy chain | amino acid | SEQ ID: 247 | SEQ ID: 248 | SEQ ID: 249 |
| FIG. 1ud | | light chain | amino acid | SEQ ID: 250 | SEQ ID: 251 | SEQ ID: 252 |
| FIG. 1va | CB301_ H3L1_F 4 | heavy chain | nucleoti de | SEQ ID: 253 | SEQ ID: 254 | SEQ ID: 255 |
| FIG. 1vb | | light chain | nucleoti de | SEQ ID: 256 | SEQ ID: 257 | SEQ ID: 258 |
| FIG. 1vc | | heavy chain | amino acid | SEQ ID: 259 | SEQ ID: 260 | SEQ ID: 261 |
| FIG. 1vd | | light chain | amino acid | SEQ ID: 262 | SEQ ID: 263 | SEQ ID: 264 |
| FIG. 1wa | CB301_ H3L1_G 11 | heavy chain | nucleoti de | SEQ ID: 265 | SEQ ID: 266 | SEQ ID: 267 |
| FIG. 1wb | | light chain | nucleoti de | SEQ ID: 268 | SEQ ID: 269 | SEQ ID: 270 |
| FIG. 1wc | | heavy chain | amino acid | SEQ ID: 271 | SEQ ID: 272 | SEQ ID: 273 |
| FIG. 1wd | | light chain | amino acid | SEQ ID: 274 | SEQ ID: 275 | SEQ ID: 276 |
| FIG. 1xa | CB301_ OPALT L_B5 | heavy chain | nucleoti de | SEQ ID: 277 | SEQ ID: 278 | SEQ ID: 279 |
| FIG. 1xb | | light chain | nucleoti de | SEQ ID: 280 | SEQ ID: 281 | SEQ ID: 282 |
| FIG. 1xc | | heavy chain | amino acid | SEQ ID: 283 | SEQ ID: 284 | SEQ ID: 285 |
| FIG. 1xd | | light chain | amino acid | SEQ ID: 286 | SEQ ID: 287 | SEQ ID: 288 |
| FIG. 1ya | CB301_ OPALT L_E6 | heavy chain | nucleoti de | SEQ ID: 289 | SEQ ID: 290 | SEQ ID: 291 |
| FIG. 1yb | | light chain | nucleoti de | SEQ ID: 292 | SEQ ID: 293 | SEQ ID: 294 |
| FIG. 1yc | | heavy chain | amino acid | SEQ ID: 295 | SEQ ID: 296 | SEQ ID: 297 |
| FIG. 1yd | | light chain | amino acid | SEQ ID: 298 | SEQ ID: 299 | SEQ ID: 300 |

As described above, 25 types of anti-CD300c monoclonal antibodies were identified which specifically bind, with high binding affinity, to the CD300c antigen and can be used for the prevention or treatment of cancer.

### Example 1.4. Production and Purification of Anti-CD300c Monoclonal Antibody

Using each of the nucleotide sequences of the anti-CD300c monoclonal antibodies identified in Example 1.3, expression vectors capable of expressing each antibody were prepared into which the heavy chain and the light chain are separately inserted. More specifically, the expression vectors were prepared by inserting genes into the pCIW3.3 vectors using the analyzed CDR sequences so that the vectors can express the heavy and light chains, respectively. The prepared expression vectors for heavy and light chains were mixed with polyethylenimine (PEI) in a mass ratio of 1:1 and transfected into 293T cells to induce antibody expression. Then, on day 8, the culture was centrifuged to remove the cells. The resulting culture was obtained. The obtained culture was filtered, and then resuspended using a mixed solution of 0.1 M NaH₂PO₄ and 0.1 M Na₂HPO₄ (pH 7.0). The resuspended solution was purified through affinity chromatography using protein A beads (GE Healthcare), and finally eluted using an elution buffer (Thermofisher).

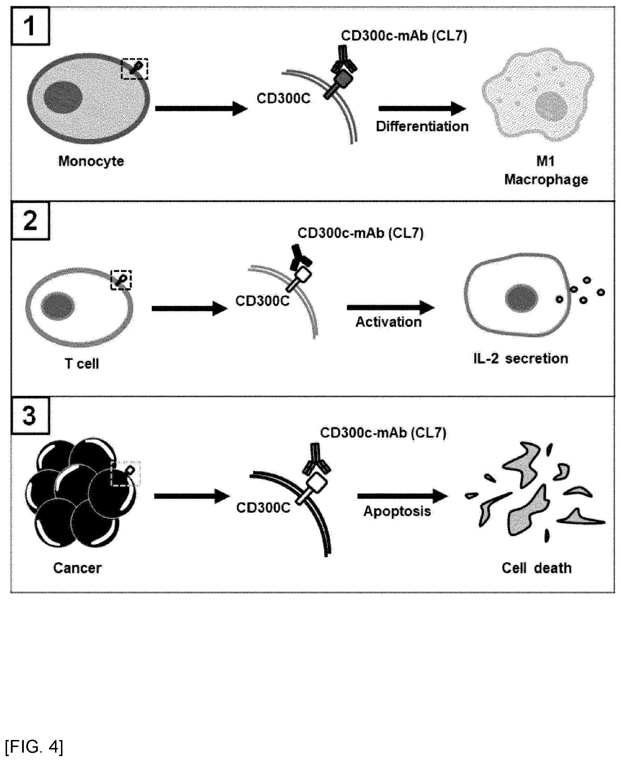

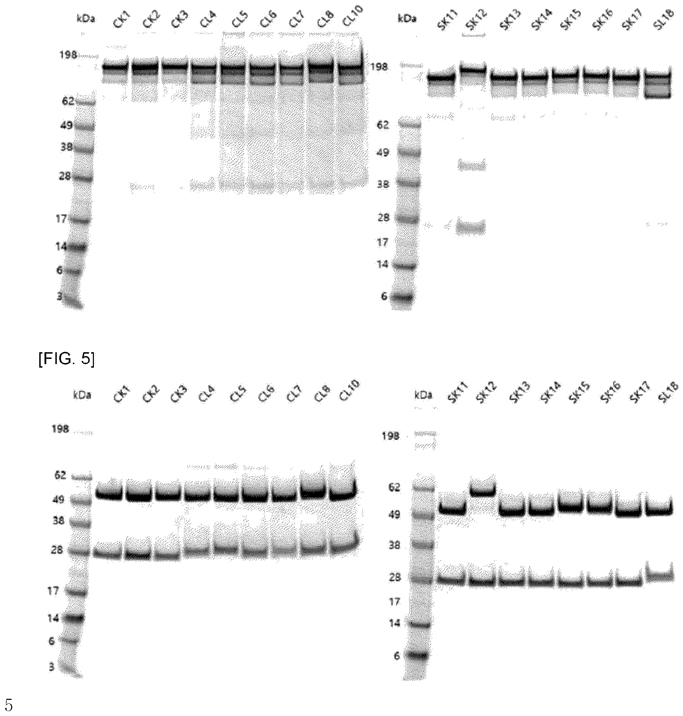

In order to identify the produced antibody, each of reducing sample buffer and non-reducing sample buffer was added to 5 µg of purified antibody, and electrophoresis was performed using pre-made SDS-PAGE (Invitrogen). Then, the proteins were stained using Coomassie Blue. The respective results under a non-reducing condition are illustrated in FIG. 4, and the respective results under a reducing condition are illustrated in FIG. 5.

As illustrated in FIGS. 4 and 5, it was identified that the anti-CD300c monoclonal antibodies having a high purity were produced and purified.

### II. Expression of CD300c in Cancer Cells and Binding of Anti-CD300c Monoclonal Antibody to CD300c Antigen

### Experimental Example 1. Expression of CD300c in Cancer Cells and Immune Cells

### Experimental Example 1.1. Identification of Expression of CD300c in Cancer Cell Line

To evaluate whether CD300c is expressed in various cancer cells, various cancer cell lines such as MKN45 (human gastric cancer cell line), IM95 (human gastric cancer cell line), HT-29 (human colorectal cancer cell line), A549 (human lung cancer cell line), HCT116 (human colorectal cancer cell line), MDA-MB-231 (human breast cancer cell line), and HepG2 (human liver cancer cell line) were cultured and expression of CD300c was evaluated at mRNA and protein levels. In addition, evaluation was also performed on THP-1 cells (human monocyte cell line) which are immune cells. Here, HEK293T (normal cell line) was used as a control.

Meanwhile, expression of the protein was identified by Western blot and flow cytometry (FACS) of fluorescently labeled cells. Specifically, each cultured cell line was fixed with 4% formaldehyde, and then blocked using 5% normal bovine serum albumin. Then, staining was performed with 0.5 µg of eFluor660-labeled anti-CD300c antibody (Invitrogen). Subsequently, the fluorescently labeled cells were identified using flow cytometry (FACS).

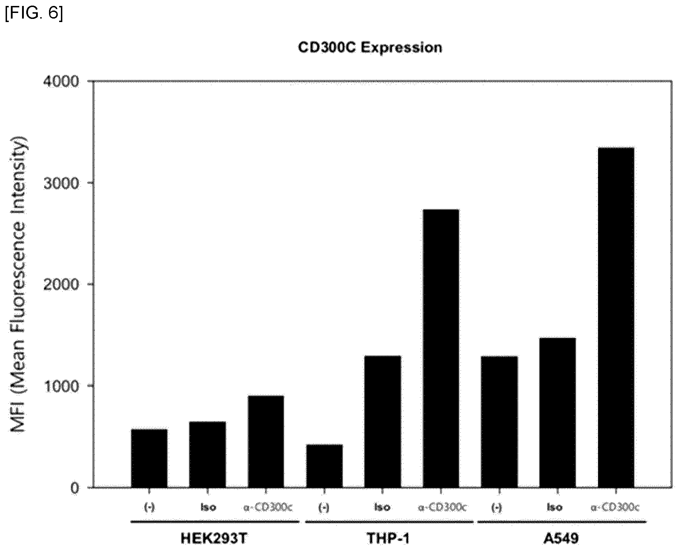

As a result, it was identified that the CD300c antigen was expressed at the mRNA and protein levels in various cancer cells such as colorectal cancer, lung cancer, and breast cancer, *etc.* In addition, as illustrated in FIG. 6, according to the analysis using flow cytometry (FACS), it was identified that significantly high expression of CD300c was observed in the human lung cancer cell line (A549) and the human monocyte cell line (THP-1) as compared with the normal cell line (HEK293T).

### Experimental Example 1.2. Identification of Expression of CD300c in Cancer Tissue and Immune Cells (I)

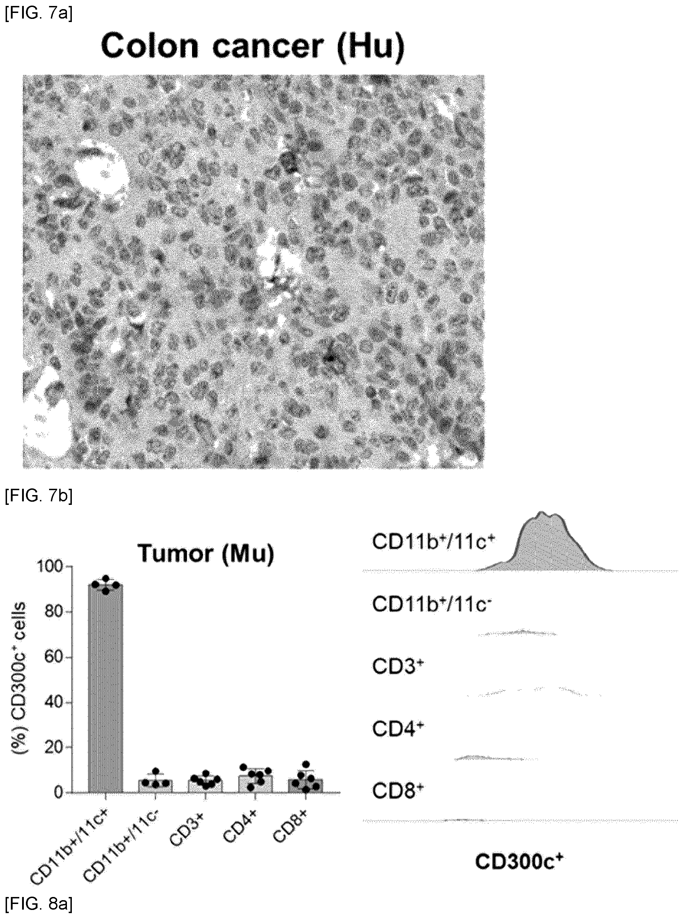

In order to identify whether CD300c is expressed in the patient's cancer tissue, tissue microarray was performed as follows. The colorectal cancer patient's tissue was fixed with formalin and embedded into a paraffin block. Then, the paraffin block was cut into sections with a diameter of 2.0 mm and a thickness of 3 to 5 um using a microtome. Then, the sections were attached to a slide in a certain direction and dried. The cancer tissue was stained with H&E staining, and then treated with an anti-CD300c antibody (Invitrogen) at 1:500 to stain CD300c. As a result, as illustrated in FIG. 7a, it was identified that CD300c was expressed in the patient's colorectal cancer tissue.

In order to identify whether CD300c is expressed not only in the patient's colorectal cancer tissue but also in immune cells within the cancer tissue, 2×10⁵ CT26 cells were transplanted into 8-week-old BALB/c mice by subcutaneous injection. On day 25 (D25) after tumor transplantation, the mice were sacrificed and tumor tissues were collected from 6 control mice that had not received the anti-CD300c antibody. The collected tumor tissue was incubated for 1 hour at 37°C in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with CD16/32 antibody (from Invitrogen), and the cells were stained with a staining solution for checking cell viability (from Invitrogen), and antibodies to the total macrophage markers F4/80 (from Abcam), CD11b (from Abcam), CD11c (from Abcam), CD3 (from Abcam), CD4 (from Thermofisher) and CD8 (from Thermofisher), and an anti-CD300c antibody (from Sino Biological). Then, the data were read with CytoFLEX flow cytometer and analyzed with FlowJo software.

As a result, as illustrated in FIG. 7b, it was identified that immune cells expressing CD300c were present in the mouse tumor tissue. These are immune cells that simultaneously express CD11b and CD11c markers, and examples thereof include dendritic cells and macrophages. From these results, it was identified that CD300c was expressed in both cancer tissue and immune cells.

### Experimental Example 1.3. Identification of Expression of CD300c in Cancer Tissue and Immune Cells (II)

In order to identify whether CD300c is expressed in human immune tissue and cancer cells, tissue microarray was performed as follows. Each of the normal tonsil tissue and the colorectal cancer patient's tissue was fixed with formalin and embedded into a paraffin block. Subsequently, in the normal tonsil tissue and the colorectal cancer patient's tissue, locations for performing tissue microarray were determined, and then each paraffin block was cut into sections with a diameter of 2.0 mm and a thickness of 3 to 5 um using a microtome. Then, the sections were attached to a slide in a certain direction and dried. The cancer tissue was stained with H&E staining, and then treated with an anti-CD300c antibody (Invitrogen) at 1:500 to stain CD300c.

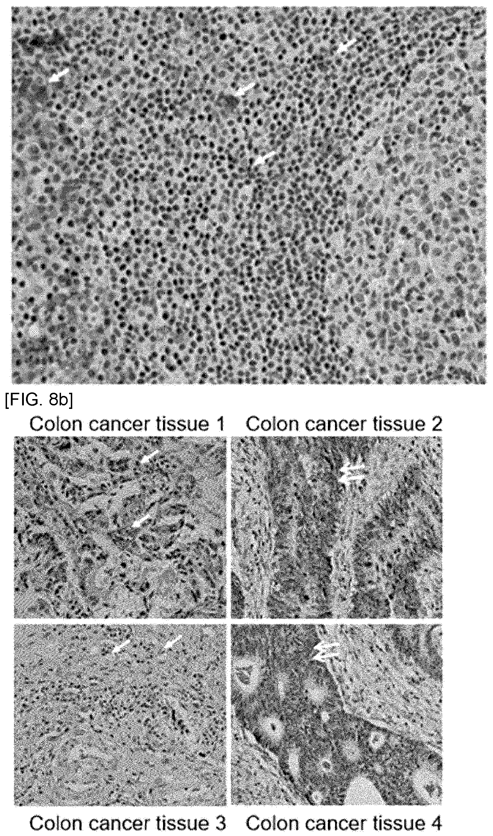

As a result, as illustrated in FIGS. 8a and 8b, it was identified that CD300c was expressed in both the normal tonsil tissue (FIG. 8a), which is an immune tissue, and the patient's colon cancer tissue (FIG. 8b). Since a large number of immune cells such as T cells and monocytes are distributed in the tonsils, the expression of CD300c in the tonsil tissue means that CD300c is expressed in immune cells. As in Experimental Example 1.2, it was identified in this experimental example that CD300c was expressed in the colorectal cancer patient's tissue; however, this experimental example is meaningful in that expression of CD300c was observed in tissues of all 4 colorectal cancer patients, indicating that CD300c was expressed in a large number of colon cancer tissues.

### Experimental Example 2. Identification of Recognition of CD300c Antigen by Anti-CD300c Monoclonal Antibody and its Binding to CD300c Antigen

### Experimental Example 2.1. Determination of Antigen-Binding Affinity of Anti-CD300c Monoclonal Antibody

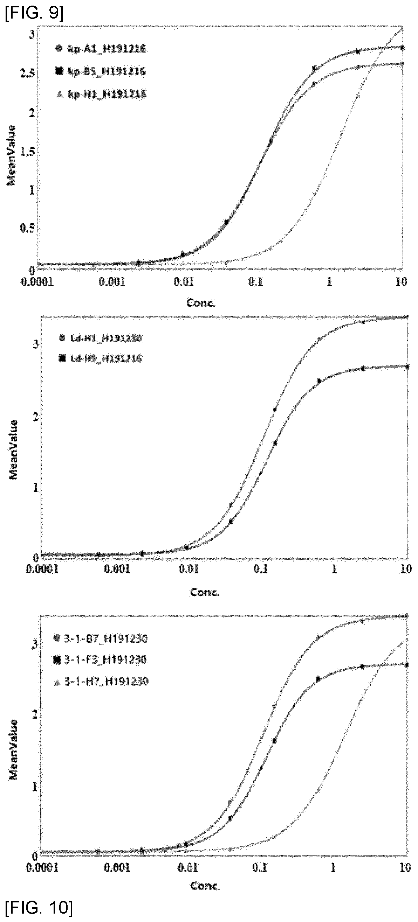

In order to identify the antigen-binding ability of the anti-CD300c monoclonal antibody produced in Example 1, binding ELISA was performed. Specifically, each of the CD300c antigen (11832-H08H, Sino Biological) or CD300a antigen (12449-H08H, Sino Biological) in a coating buffer (0.1 M sodium carbonate, pH 9.0) was dispensed onto an ELISA plate at a concentration of 8 µg/mL per well, and then reaction was allowed to proceed at room temperature for 3 hours so that the antigen could bind to the plate. Washing was performed 3 times using phosphate buffered saline-Tween 20 (PBST) to remove unbound antigens, and then 300 µL of PBST supplemented with 5% bovine serum albumin (BSA) was added to each well. The reaction was allowed to proceed at room temperature for 1 hour, and washing was performed again using PBST. Then, the anti-CD300c monoclonal antibody was diluted in quadruplicate and added thereto. The reaction was allowed to proceed for 1 hour at room temperature for antigen binding. After 1 hour, washing was performed 3 times using PBST to remove unbound anti-CD300c monoclonal antibody, and then 4 µg/mL of an antibody for detection (HRP conjugated anti-Fc IgG) was added. The reaction was allowed to proceed again at room temperature for 1 hour. Subsequently, the unbound antibody for detection was removed using PBST, and then TMB solution was added. The reaction was allowed to proceed for 10 minutes for development. Then, 2 N sulfuric acid solution was added to terminate the development, and the absorbance was measured at 450 nm to identify the antibodies that specifically bind to the CD300c antigen. The results are shown in Table 6 and FIG. 9.

**[Table 6]**

| CB301 antibody | EC50 (µg/mL) |
|---|---|
| CK1 | 0.056 |
| CK2 | 0.033 |
| CK3 | 0.793 |
| CL4 | 0.031 |
| CL5 | 0.032 |
| CL6 | 0.148 |
| CL7 | 0.047 |
| CL8 | 49.7 |
| CL9 | 0.094 |
| CL10 | 0.039 |
| SK11 | 0.052 |
| SK12 | 0.067 |
| SK13 | 0.044 |
| SK14 | 0.065 |
| SK15 | 14.74 |
| SK16 | 2.42 |
| SK17 | 0.054 |
| SL18 | 0.17 |

As shown in Table 6, as a result of measuring the EC50 (effective concentration of drug that causes 50% of the maximum response) values of the anti-CD300c monoclonal antibodies, it was identified that the remaining all 14 clones except for 4 clones (CK3, CL8, SK15, SK16) exhibited high binding affinity of 0.2 µg/mL or lower. In addition, as illustrated in FIG. 9, it was found that the anti-CD300c monoclonal antibodies of the present disclosure were bound to the CD300c antigen with high binding affinity even in the sigmoid curves for the results of the binding ELISA.

### Experimental Example 2.2. Identification of Recognition of Cellular Antigen by Anti-CD300c Monoclonal Antibody

In order to identify whether the anti-CD300c monoclonal antibody (CL7) recognizes a cellular antigen, FACS binding was performed.

CD300c was overexpressed in 293T cells (ATCC) and THP-1 cells (ATCC), and then the respective cells were dispensed into respective microcentrifuge tubes at 2×10⁵ cells per tube. Then, the cells were allowed to react with the anti-CD300c monoclonal antibody, which was serially diluted 3-fold starting from 10 µg/mL, in a CO₂ incubator for 30 min. Washing was performed twice with FACS buffer. Then, the cells were allowed to react with FITC-conjugated anti-human IgG (H+L), which was diluted 1:100 in FACS buffer, in a CO₂ incubator for 30 min. Washing was performed twice with FACS buffer. Subsequently, FITC signals were measured with a CytoFLEX instrument manufactured by Beckman Coulter, Inc., and then the MFI values were obtained using CytExpert program. Using the obtained MFI values, sigmoidal curves were drawn by a sigmaplot program to calculate the EC50 (an effective concentration of drug that causes 50% of the maximum response) values. As a result, an EC50 of 2.7 nM was obtained for the 293T cells and an EC50 of 2.6 nM was obtained for the THP-1 cells.

As illustrated in FIG. 10, in the sigmoidal curve for the result of FACS binding, the anti-CD300c monoclonal antibody produced in Example 1 bound, with strong binding affinity, to CD300c overexpressed on the surface of THP-1 and 293T cells. Therefore, it was identified that the anti-CD300c monoclonal antibody bound to CD300c in an antigen-specific manner.

### Experimental Example 2.3. Determination of Binding Affinity of Anti-CD300c Monoclonal Antibody to CD300c Antigen (I): Binding ELISA

CD300c antigen (250 ug/mL) was diluted to a concentration of 800 ng/mL in coating buffer (0.1 M sodium carbonate, pH 9.0), put into a 96-well microplate at 100 µL per well, and incubated at 4°C overnight. The next day, washing with 200 µL of PBST was performed three times. Then, 200 µL of blocking buffer (5% skim milk) was added thereto, and blocking was performed at room temperature for 1 hour. The anti-CD300c monoclonal antibody CL7 was diluted to 200 µg/mL in PBS, and its concentration was checked by measurement using Nanodrop (product name: NanoDrop One/One^{c}, manufactured by Thermo Fisher Scientific). Subsequently, CL7 was diluted in quadruplicate with PBS starting from 10 µg/mL, added at 100 µL each, and then the reaction was allowed to proceed at room temperature for 1 hour. After the reaction, washing with 200 µL of PBST was performed three times. Secondary antibody (conjugated anti-Fc IgG) was diluted 1:10,000 in blocking buffer, added at 100 µL, and then reaction was allowed to proceed at room temperature for 1 hour. Then, washing with 200 µL of PBST was performed three times. Subsequently, TMB and hydrogen peroxide were mixed 1:1, added at 100 µL per well, and then the reaction was allowed to proceed at room temperature for 7 to 9 minutes. Then, 50 µL of 1 N sulfuric acid was added to stop development, and measurement was performed at 450 nm using a microplate reader (product name: Varioskan LUX) to obtain binding affinity results.

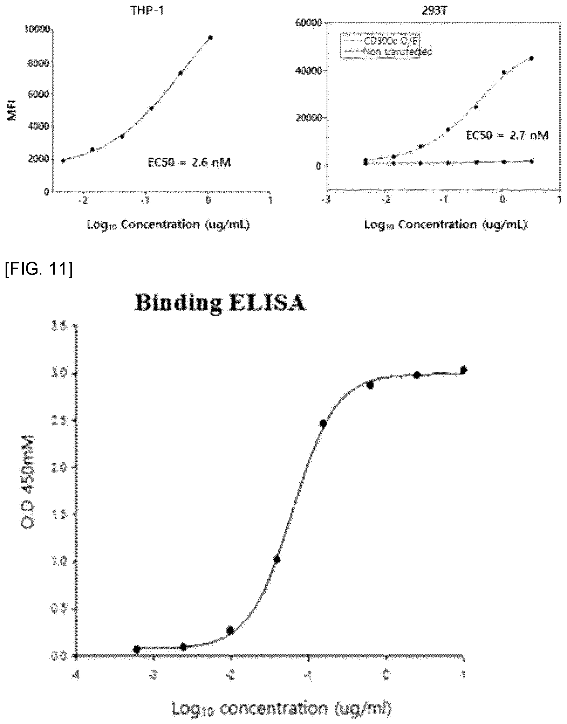

As a result, as illustrated in FIG. 11, it was identified that the anti-CD300c monoclonal antibody bound to CD300c in a concentration-dependent manner, indicating that the anti-CD300c monoclonal antibody has excellent binding affinity and specificity to the antigen CD300c.

### Experimental Example 2.4. Determination of Binding Affinity of Anti-CD300c Monoclonal Antibody to CD300c Antigen (II): Surface Plasmon Resonance (SPR)

In order to identify the binding affinity between the antigen CD300c and the anti-CD300c monoclonal antibody CL7, a surface plasmon resonance experiment was performed.

To immobilize CD300c on a CMS chip, 5 µg/mL of CD300c was diluted in 10 mM acetate buffer (pH 5.5). Then, the flow rates were all set equally to 10 mL/min, and the target RU was respectively set to 300 RU. Activation was carried out with a mixture of 0.2 M EDC and 0.05 M NHS and blocked with 1 M ethanolamine. Immobilization was performed so that the final RU of CD300c could become 399.2 RU. Then, CL7 was diluted in PBSP to concentrations of 0, 0.195, 0.39, 0.78, 1.56, 3.125, and 6.25 µg/mL, respectively. A kinetics/affinity test was performed with an association time of 240 seconds, a dissociation time of 900 seconds, and a flow rate of 30 µL/min. Subsequently, 50 mM NaOH was flowed at a rate of 30 µL/min for 30 seconds to regenerate the surface.

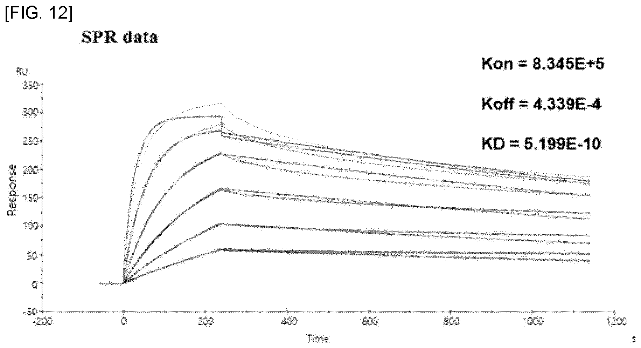

As a result, as illustrated in FIG. 12, the KD value was analyzed as 5.199E-10 M, and the binding affinity of the anti-CD300c monoclonal antibody was identified as 0.52 nM that is at a subnanomol level. This means that the anti-CD300c monoclonal antibody exhibits high binding affinity to the antigen.

### Experimental Example 2.5. Identification of Binding Specificity of Anti-CD300c Monoclonal Antibody to CD300c Antigen (I)

Binding ELISA was performed to identify that the anti-CD300c monoclonal antibody CL7 specifically binds only to CD300c without binding to other B7 family proteins. More specifically, each of CD300c antigen, CD300a antigen, or seven B7 family protein antigens (PD-L1 [B7-H1] (Sino Biological), ICOS Ligand[B7-H2] (Sino Biological), CD276[B7-H3] (Sino Biological), B7-H4 (Sino Biological), CD80[B7-1] (Sino Biological), CD86[B7-2] (Sino Biological), CD273[PD-L2] (Sino Biological)) in a coating buffer (0.1 M sodium carbonate, pH 9.0) was coated on an ELISA plate at a concentration of 8 µg/mL per well, and then incubation was performed at 2°C to 8°C overnight so that the antigen could bind to the plate. The unbound antigen was removed by performing washing with PBST three times, and then 300 mL of blocking buffer (5% skim milk in PBST) was added to each well. Subsequently, blocking was performed at room temperature for 1 hour, and then washing was performed again with PBST. Thereafter, CL7 was diluted in quadruplicate with PBS, and subjected to reaction with the antigen at room temperature for 1 hour for antigen binding. 1 hour later, washing with PBST was performed three times. Then, a secondary antibody (HRP-conjugated anti-Fc IgG) diluted to 4 µg/mL in blocking buffer was added thereto, and the reaction was allowed to proceed at room temperature for 1 hour. Subsequently, the unbound antibody for detection was removed using PBST, and then a TMB solution was added thereto. The reaction was allowed to proceed for 10 minutes for development. Then, a 2 N sulfuric acid solution was added to terminate the development, and the absorbance was measured at 450 nm to identify the antibody that specifically binds to the CD300c antigen.

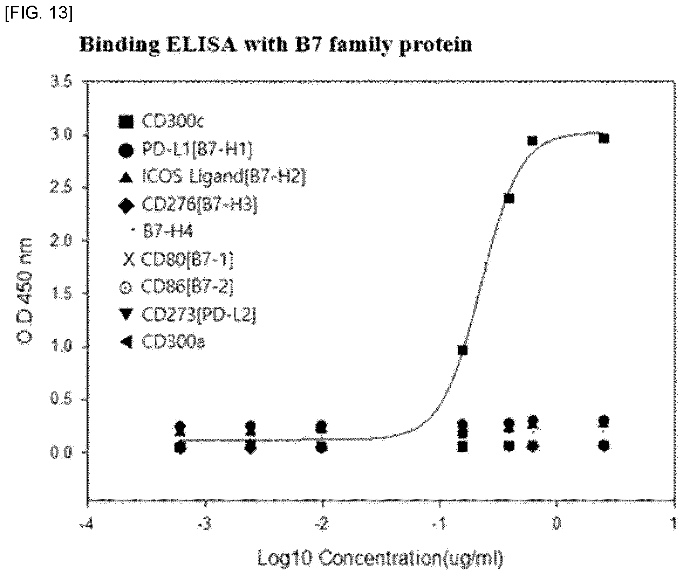

As a result, as illustrated in FIG. 13, it was identified that the anti-CD300c monoclonal antibody specifically recognized only CD300c without binding to other similar proteins.

### Experimental Example 2.6. Identification of Binding Specificity of Anti-CD300c Monoclonal Antibody to CD300c Antigen (II)

In order to identify specificity of the anti-CD300c monoclonal antibody CL7 to the CD300c antigen, it was further checked whether CL7 exhibits cross-reactivity to the CD300a antigen that has been known to antagonize the CD300c antigen and has a similar protein sequence thereto. More specifically, treatment with the CD300a antigen (from Sino Biological) was performed at concentrations of 0.039 µg/mL, 0.63 µg/mL, and 10 µg/mL, and binding ELISA was performed in the same manner as in Experimental Example 2.1.

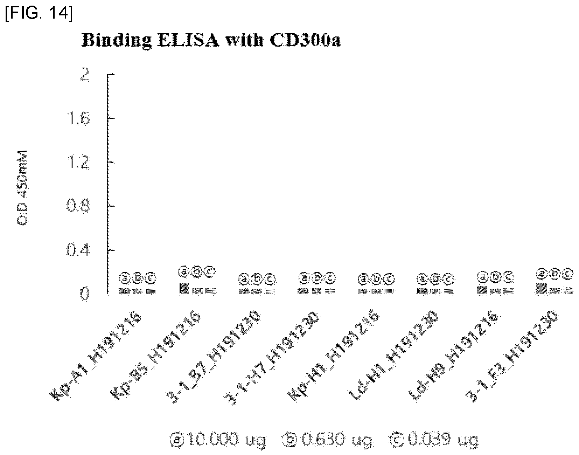

As a result, as illustrated in FIG. 14, it was found that the anti-CD300c monoclonal antibody did not bind to antigens other than CD300c and showed high binding specificity only to the CD300c antigen.

### Experimental Example 2.7. Comparison of Overall Survival of Various Cancer Patients Depending on CD300c Expression Level

Using data of patients with kidney cancer (530 patients), pancreatic cancer (177 patients), and liver cancer (370 patients) obtained from The Cancer Genome Atlas (TCGA) database, comparison of overall survival depending on the CD300c expression level was performed. First, the respective cancer patients were classified depending on the high or low degree of CD300c expression level. This high or low degree of level was determined by comparison with the average CD300c expression level for each cancer type. For the renal cancer patients, 394 patients had low CD300c expression levels and 136 patients had high CD300c expression levels. For the pancreatic cancer patients, 57 patients had low CD300c expression levels and 120 patients had high CD300c expression levels. Meanwhile, for the liver cancer patients, 192 patients had low CD300c expression levels, and 178 patients had high CD300c expression levels. The overall survival depending on the CD300c expression level of each cancer patient was analyzed by the Kaplan-Meier method. Subsequently, comparison of the survival between the patient group with a high CD300c expression level and the patient group with a low CD300c expression level was performed by log-rank test.

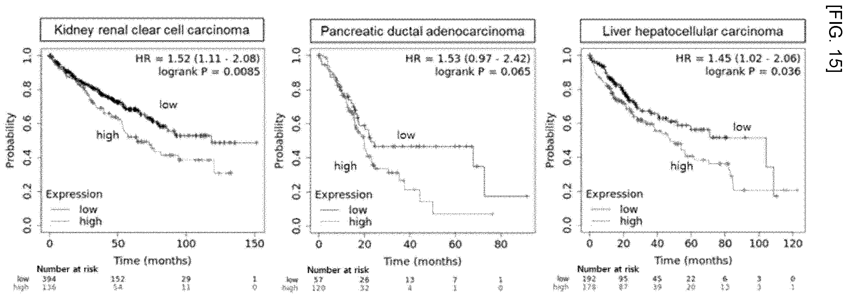

As a result, as illustrated in FIG. 15, it was identified that the patients with a high CD300c expression level had shorter survival than the patients with a low CD300c expression level, and these indicated significant results in a case where the P value was taken into account. These results indicate that expression of CD300c is highly correlated with the survival of cancer patients, and also that a cancer therapeutic effect or survival increase effect can be expected in a case where expression or activity of CD300c is inhibited.

### III. Anticancer Effect of Anti-CD300c Monoclonal Antibody

### Experimental Example 3. Identification of Anticancer Effect Caused by Administration of Anti-CD300c Monoclonal Antibody

### Experimental Example 3.1. Identification of T Cell Activation Effect

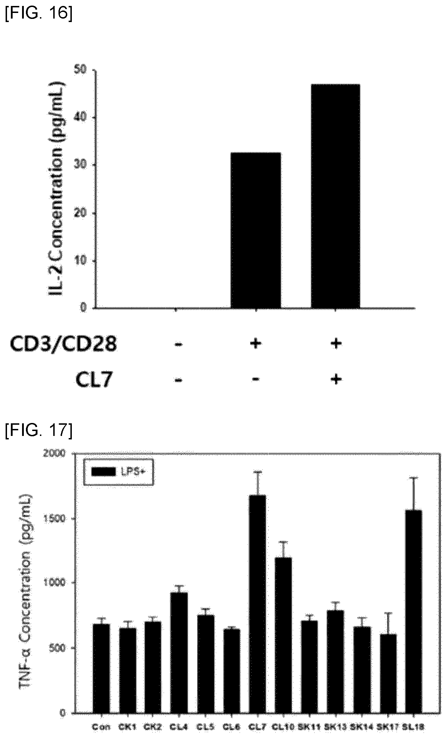

To identify whether the anti-CD300c monoclonal antibody produced in Example 1 can exhibit an anticancer effect by activating T cells, the production level of interleukin-2 (IL-2) was identified in a case where human T cells are subjected to treatment with the anti-CD300c monoclonal antibody. IL-2 is an immune factor that helps growth, proliferation, and differentiation of T cells, and the increased production level of IL-2 means activation of T cells due to an increase in stimulation that induces increased differentiation, proliferation, and growth of T cells. Specifically, each of the anti-CD3 monoclonal antibody and the anti-CD28 monoclonal antibody was added to a 96-well plate at a concentration of 2 µg/well and fixed for 24 hours. Then, co-treatment with 1 ×10⁵ cells/well of Jurkat T cells (human T lymphocyte cell line) and 10 µg/well of the anti-CD300c monoclonal antibody were performed. Subsequently, the production level of IL-2 was measured using an ELISA kit (IL-2 Quantikine kit, R&D Systems), and then compared with the control group that had not been treated with the anti-CD300c monoclonal antibody. The results are illustrated in FIG. 16.

As illustrated in FIG. 16, it was identified that the production level of IL-2 increased in a case where Jurkat T cells activated by treatment with the anti-CD3 monoclonal antibody and the anti-CD28 monoclonal antibody were treated with the anti-CD300c monoclonal antibody. From these results, it was found that the anti-CD300c monoclonal antibody was able to activate T cells, indicating that the anti-CD300c monoclonal antibody is able to induce anticancer immune action to inhibit growth of cancer tissue.

### Experimental Example 3.2. Identification of Promotion of Differentiation into M1 Macrophages (I): Measurement of Production Level of Differentiation Marker (TNF-α)

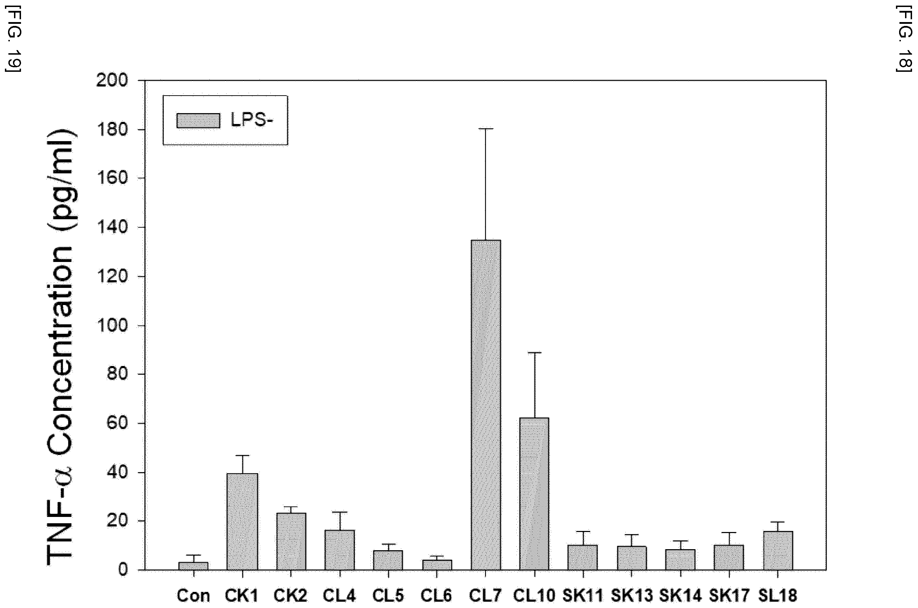

In order to identify whether the anti-CD300c monoclonal antibody selected in Example 1 is able to promote differentiation of monocytes into M1 macrophages, THP-1 (human monocyte cell line) at 1.5×10⁴ cells/well was dispensed onto a 96-well plate, and treatment with 10 µg/mL of the anti-CD300c monoclonal antibody and/or 100 ng/mL of LPS was performed. The reaction was allowed to proceed for 48 hours, and then the production level of tumor necrosis factor-α (TNF-α), which is a differentiation marker of M1 macrophages, was measured using an ELISA kit (Human TNF-α Quantikine kit, R&D Systems). The results are illustrated in FIGS. 17 and 18.

As illustrated in FIG. 17, it was identified that the anti-CD300c monoclonal antibodies, CL4, CL7, CL10, and SL18, exhibited an increase in production level of TNF-α which is about 2 or more times higher than that of the control group (Con) treated with LPS alone.

In addition, as illustrated in FIG. 18, it was identified that all the experimental groups treated with the anti-CD300c monoclonal antibody alone without LPS treatment exhibited an increase in production level of TNF-α as compared with the control group (Con) treated with LPS alone.

### Experimental Example 3.3. Identification of Increased Capacity for Causing Differentiation into M1 Macrophages Depending on Concentrations of Anti-CD300c Monoclonal Antibody

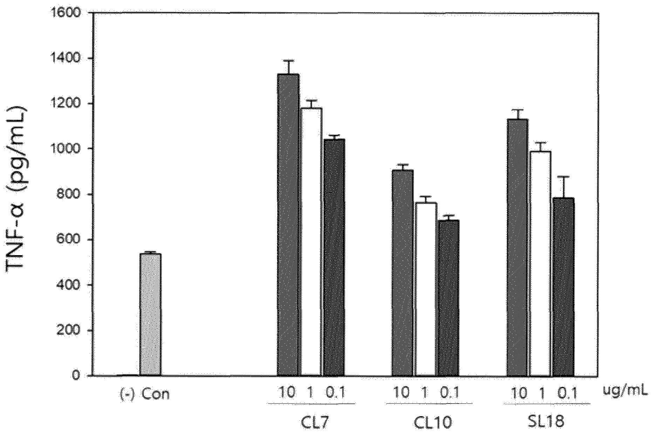

In order to identify whether induction of differentiation into M1 macrophages by the anti-CD300c monoclonal antibody increases with concentrations of the anti-CD300c monoclonal antibody, the production level of TNF-α was identified in the same manner as in Example 3.2. Treatment with the anti-CD300c monoclonal antibody was performed at concentrations of 10 µg/mL, 1 µg/mL, and 0.1 µg/mL. The results are illustrated in FIG. 19. As illustrated in FIG. 19, it was identified that the production level of TNF-α increased as the treatment concentration of the anti-CD300c monoclonal antibody (CL7, CL10, or SL18) increased.

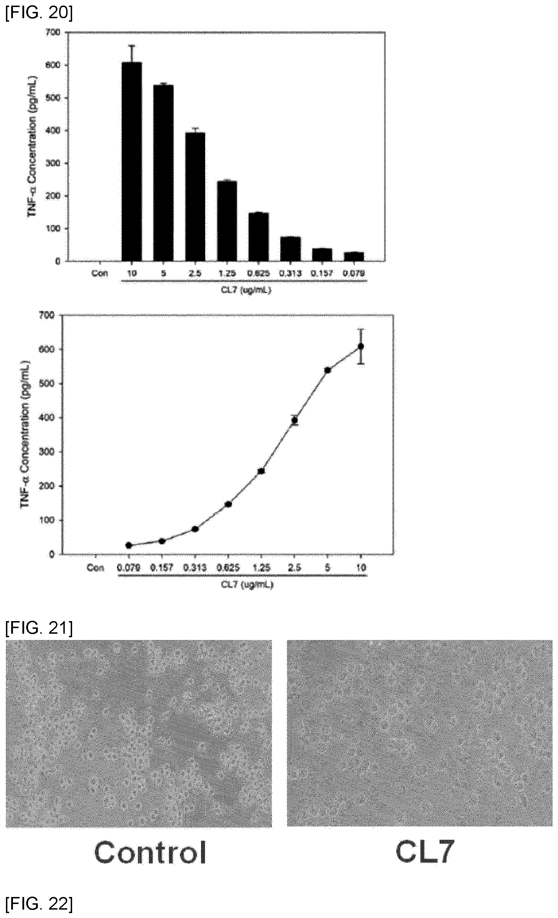

In order to identify results with further divided concentrations, treatment with the anti-CD300c monoclonal antibody (CL7) was performed at concentrations of 10 µg/mL, 5 µg/mL, 2.5 µg/mL, 1.25 µg/mL, 0.625 µg/mL, 0.313 µg/mL, 0.157 µg/mL, and 0.079 µg/mL, and the production level of TNF-α was identified. The results are illustrated in FIG. 20. As illustrated in FIG. 20, it was identified that the production level of TNF-α increased in a concentration-dependent manner with respect to the anti-CD300c monoclonal antibody.

### Experimental Example 3.4. Identification of Promotion of Differentiation into M1 Macrophages (II): Observation of Cell Morphology

In order to identify, through cell morphology, the differentiation pattern into M1 macrophages in a case where monocytes are treated with the anti-CD300c monoclonal antibody, THP-1 was treated with 10 µg/mL of the anti-CD300c monoclonal antibody, cultured for 48 hours, and then the shape of the cells was observed under a microscope. The results are illustrated in FIG. 21.

As illustrated in FIG. 21, it was identified that for the experimental group (CL7) treated with the anti-CD300c monoclonal antibody, the shape of THP-1 cells was changed from suspension cells to circular adherent cells that are in the form of M1 macrophages. From these results, it was identified that differentiation of monocytes into M1 macrophages was promoted by treatment with the anti-CD300c monoclonal antibody.

### Experimental Example 3.5. Reidentification of Promotion of Differentiation into M1 Macrophages

In order to identify again whether the anti-CD300c monoclonal antibody CL7 promotes differentiation of human monocytes into M1 macrophages, the secretion levels of TNF-α, interleukin-1β (IL-1β), and interleukin-8 (IL-8) were measured using an ELISA kit. More specifically, THP-1 at 1.5×10⁴ cells/well was dispensed onto a 96-well plate, and treatment with 10 µg/mL of the anti-CD300c monoclonal antibody was performed. The reaction was allowed to proceed for 48 hours, and then the production levels of TNF-α, IL-1β, and IL-8, which are markers for differentiation into M1 macrophages, were measured using an ELISA kit (Human TNF-α Quantikine kit, R&D Systems). The results are illustrated in FIG. 22.

As illustrated in FIG. 22, it was identified that all three types of markers for differentiation into M1 macrophages increased in the experimental group (CL7) treated with the anti-CD300c monoclonal antibody, as compared with the control group (Con) not treated with the anti-CD300c monoclonal antibody.

### Experimental Example 3.6. Identification of Capacity for Causing Repolarization of M2 Macrophages into M1 Macrophages

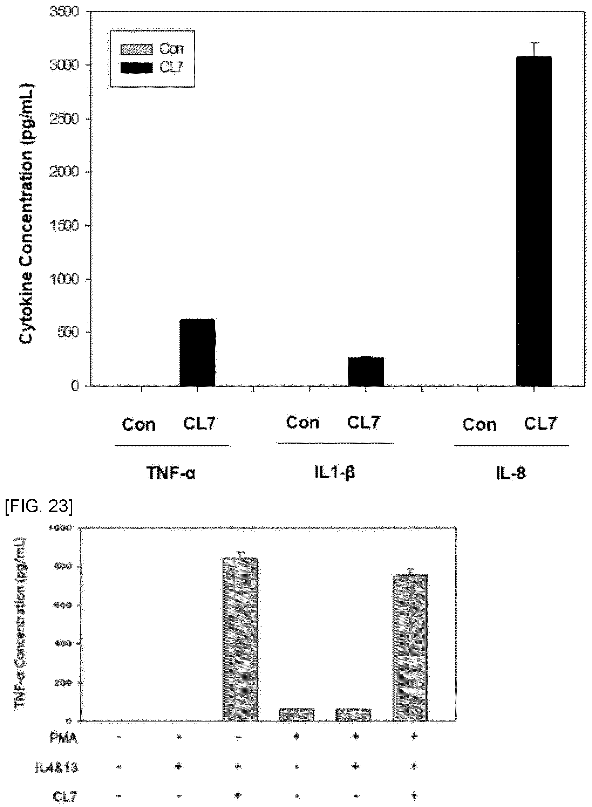

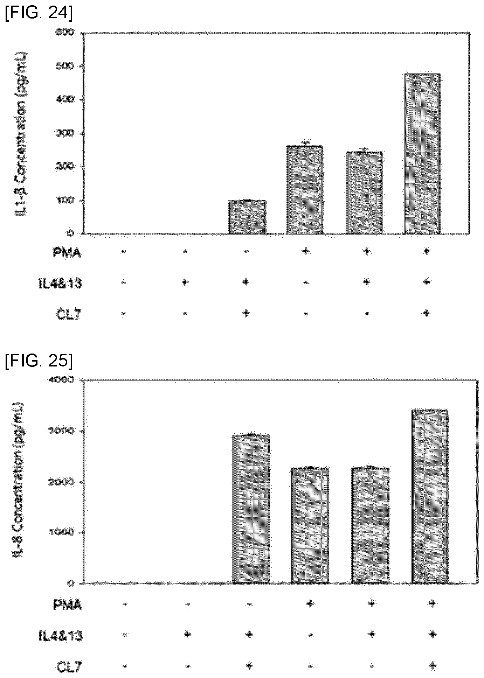

In order to identify whether the anti-CD300c monoclonal antibody is able to redifferentiate(repolarization) M2 macrophages into M1 macrophages, THP-1 at 1.5×10⁴ cells was dispensed onto a 96-well plate, and pre-treated for 6 hours by treatment with 320 nM of PMA. Then, treatment with 20 ng/mL of interleukin-4 (IL-4) and interleukin-13 (IL-13), and with 10 µg/mL of the anti-CD300c monoclonal antibody was performed, and the reaction was allowed to proceed for 18 hours. The production levels of TNF-α, IL-1β, and IL-8 were identified using an ELISA kit. The results are illustrated in FIGS. 23 to 25.

As illustrated in FIGS. 23 to 25, it was identified that among the experimental groups not pre-treated with PMA, the experimental group co-treated with IL-4 & IL-13 and the anti-CD300c monoclonal antibody exhibited increased production levels of TNF-α, IL-1β, and IL-8; and among the experimental groups pre-treated with PMA, the experimental group co-treated with IL-4 & IL-13 and the anti-CD300c monoclonal antibody similarly exhibited increased production levels of TNF-α, IL-1β, and IL-8. From these results, it was found that the anti-CD300c monoclonal antibody was able to effectively redifferentiate(repolarization) M2 macrophages into M1 macrophages.

### Experimental Example 3.7. Identification of Capacity for Causing Differentiation and Repolarization into M1 Macrophages

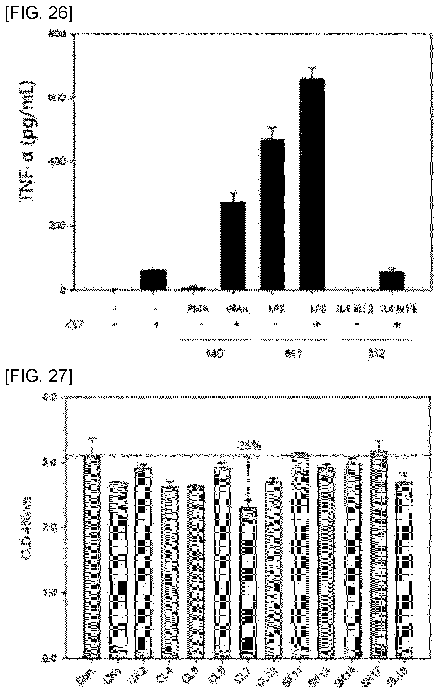

In order to identify the anti-CD300c monoclonal antibody's capacity for causing differentiation and redifferentiation(repolarization) into M1 macrophages, THP-1 at 1.5×10⁴ cells was dispensed onto a 96-well plate, pre-treated with 10 µg/mL of the anti-CD300c monoclonal antibody for 48 hours, and treated with 100 ng/mL of PMA, 100 ng/mL of LPS, and 20 ng/mL of IL-4 and IL-13. The reaction was allowed to proceed for 24 hours. The production level of TNF-α was identified using an ELISA kit. The results are illustrated in FIG. 26.

As illustrated in FIG. 26, it was identified that all experimental groups pre-treated with the anti-CD300c monoclonal antibody exhibited a significant increase in production level of TNF-α, as compared with the M0 macrophage control group treated with PMA alone, the M1 macrophage control group treated with LPS alone, and the M2 macrophage control group treated with IL-4 and IL-13 alone. From these results, it was found that the anti-CD300c monoclonal antibody had excellent capacity to differentiate M0 macrophages into M1 macrophages, to differentiate THP-1 into M1 macrophages, and to redifferentiate(repolarization) M2 macrophages into M1 macrophages.

### Experimental Example 4. Identification of Inter-Species Cross-Reactivity of Anti-CD300c Monoclonal Antibody by Observation of Anticancer Effect

### Experimental Example 4.1. Identification of Human Cancer Cell Growth inhibitory Effect

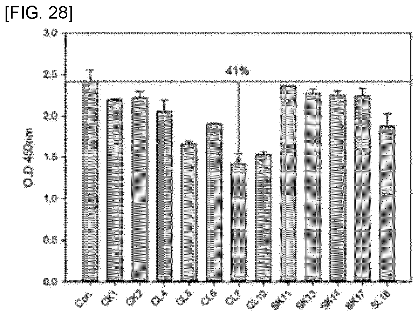

In order to identify an effect of the CD300c-targeting monoclonal antibody on growth of cancer cells, cell proliferation assay was performed using A549 (human lung cancer cell line). More specifically, the cells were dispensed onto a 96-well plate at 2×10⁴ cells under a condition of 0% fetal bovine serum (FBS), and at 6×10³ cells under a condition of 0.1% fetal bovine serum. Then, treatment with 10 µg/mL of anti-CD300c monoclonal antibody was performed and incubation was performed for 5 days. Treatment with CCK-8 (DOJINDO) was performed and the absorbance was measured at OD450 nm to identify a cancer cell growth inhibitory effect of the anti-CD300c monoclonal antibody. The results are illustrated in FIGS. 27 and 28.

As illustrated in FIG. 27, it was identified that all anti-CD300c monoclonal antibodies except for SK11 and SK17 had an effect of inhibiting proliferation of cancer cells under a condition of 0% FBS.

As illustrated in FIG. 28, it was identified that all anti-CD300c monoclonal antibodies used in the experiment had an effect of inhibiting proliferation of cancer cells under a condition of 0.1% FBS.

### Experimental Example 4.2. Identification of Cancer Cell Growth inhibitory Effects of Anti-CD300c Monoclonal Antibody Depending on its Concentrations

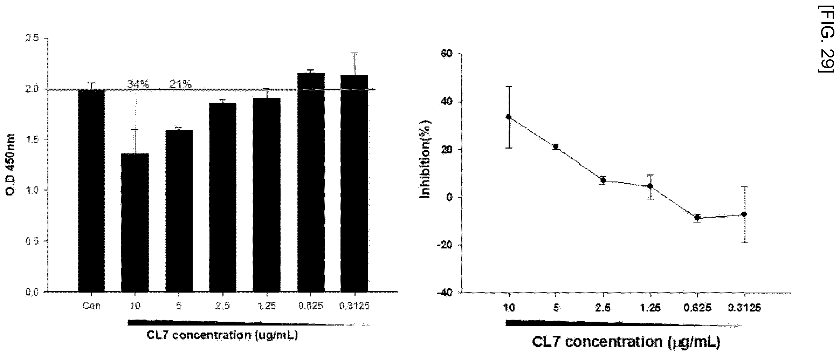

In order to identify cancer cell growth inhibitory effects of the anti-CD300c monoclonal antibody depending on its concentrations, A549 cells were dispensed onto a 96-well plate at 2×10⁴ cells under a condition of 0% fetal bovine serum (FBS). Treatment with 10 µg/mL of the anti-CD300c monoclonal antibody was performed and incubation was performed for 5 days. Subsequently, treatment with CCK-8 (DOJINDO) was performed and reaction was allowed to proceed for 3 hours. Then, the absorbance was measured at OD450 nm to identify cancer cell growth inhibitory effects of the anti-CD300c monoclonal antibody. The results are illustrated in FIG. 29.

As illustrated in FIG. 29, it was identified that growth of cancer cells was inhibited as the concentration of the anti-CD300c monoclonal antibody increased.

### Experimental Example 4.3. Identification of Increased Capacity for Causing Differentiation into M1 Macrophages in Mice

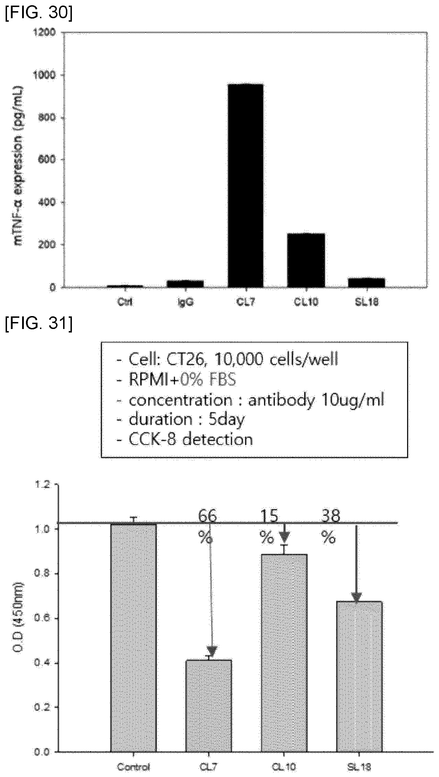

In order to identify whether the anti-CD300c monoclonal antibody is able to promote differentiation from mouse macrophages to M1 macrophages, mouse macrophages (Raw264.7) were dispensed onto a 96-well plate at a concentration of 1×10⁴ cells/well, treatment with 10 µg/mL of the anti-CD300c monoclonal antibody was performed, and incubation was performed. The production level of TNF-α was checked with an ELISA kit. The results are illustrated in FIG. 30.

As illustrated in FIG. 30, it was identified that the production level of TNF-α increased in the experimental groups treated with the anti-CD300c monoclonal antibody. From these results, it can be seen that the anti-CD300c monoclonal antibody acts equally in humans as well as mice, indicating the cross-reactivity of the anti-CD300c monoclonal antibody which promotes differentiation into M1 macrophages.

### Experimental Example 4.4. Identification of Cancer Cell Growth inhibitory Effects in Mice

In order to identify whether the anti-CD300c monoclonal antibodies CL7, CL10, and SL18 exhibit anticancer effects, CT26 (mouse colorectal cancer cell line) was dispensed onto a 96-well plate at a concentration of 1×10⁴ cells/well, treatment with 10 µg/mL of each monoclonal antibody was performed, and incubation was performed for 5 days. Then, cell proliferation assay was performed by detection of CCK-8.

As illustrated in FIG. 31, the anti-CD300c monoclonal antibodies exerted cancer cell proliferation inhibitory effects which were 66% (CL7), 15% (CL10), and 38% (SL18), respectively, higher than that of the control group. From these results, it was identified that the anti-CD300c monoclonal antibodies exhibited cancer therapeutic effects in mice. Thus, it can be seen that the anti-CD300c monoclonal antibody acts equally in mice as well as humans, indicating the cross-reactivity of the anti-CD300c monoclonal antibody which results in an anticancer effect.

### Experimental Example 5. Comparison of Anticancer Effects In Vitro Between Anti-CD300c Monoclonal Antibody and Conventional Cancer Immunotherapy

The manufacturers of the respective immunotherapies used in the experimental examples below are as follows: IMFINZI^{®} (AstraZeneca) and KEYTRUDA@ (Merck Sharp & Dohme).

### Experimental Example 5.1. Comparison of Capacity for Causing Differentiation into M1 Macrophages Between Anti-CD300c Monoclonal Antibody and Conventional Cancer Immunotherapy: Measurement of Production Levels of Three Differentiation Markers (TNF-α, IL-1β, and IL-8)

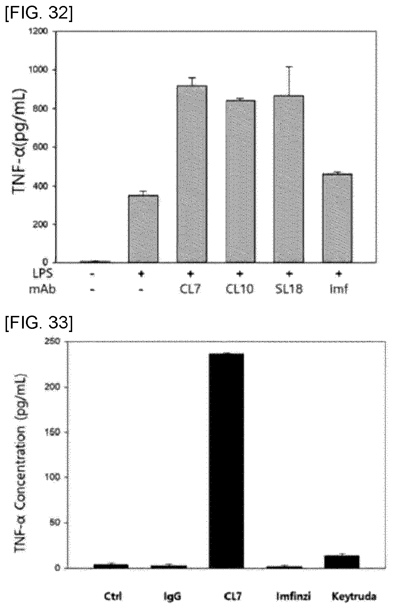

In order to compare capacity for causing differentiation into M1 macrophages between the anti-CD300c monoclonal antibodies and a conventional cancer immunotherapy, the production level of TNF-α was identified using an ELISA kit in the same manner as in Example 3.2. As the conventional cancer therapy, IMFINZI^{®} was used at a concentration of 10 µg/mL. The results are illustrated in FIG. 32.

As illustrated in FIG. 32, it was identified that the anti-CD300c monoclonal antibody resulted in a remarkably increased production level of TNF-α as compared with the control group treated with IMFINZI^{®} (Imf), which is known as a cancer immunotherapy, alone. From these results, it was found that the anti-CD300c monoclonal antibody resulted in remarkably increased capacity for causing differentiation into M1 macrophages as compared with the conventionally known cancer immunotherapy.

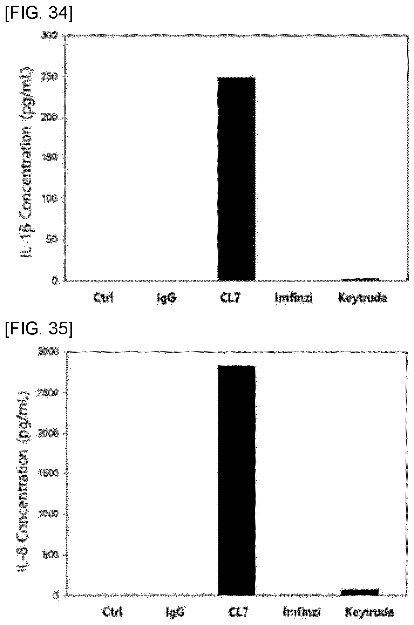

For comparison with other cancer immunotherapies, each of IMFINZI^{®}, which is an anti-PD-L1 immunotherapy, KEYTRUDA@, which is an anti-PD-1 immunotherapy, and an isotype control (immunoglobulin G) antibody was used at a concentration of 10 µg/mL, and the production levels of TNF-α, IL-1β, and IL-8 were identified using an ELISA kit. The results are illustrated in FIGS. 33 to 35.

As illustrated in FIGS. 33 to 35, it was identified that the anti-CD300c monoclonal antibody resulted in remarkably increased production levels of TNF-α, IL-1β, and IL-8 as compared with IMFINZI^{®}, KEYTRUDA@, and the IgG antibody. From these results, it was found that the anti-CD300c monoclonal antibody was able to result in remarkably increased promotion of differentiation into M1 macrophages as compared with the conventional cancer immunotherapies.

### Experimental Example 5.2. Comparison of Capacity for Causing Differentiation from M0 Macrophages into M1 Macrophages Between Anti-CD300c Monoclonal Antibody and Conventional Cancer Immunotherapy

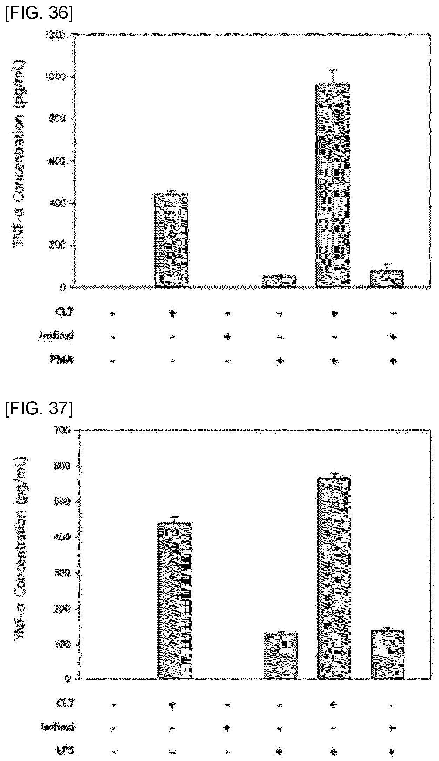

In order to compare capacity for causing differentiation from M0 macrophages into M1 macrophages between the anti-CD300c monoclonal antibodies and a cancer immunotherapy, THP-1 at 1.5×10⁴ cells/well was dispensed onto a 96-well plate, and treatment with 10 µg/mL of the anti-CD300c monoclonal antibody, 10 µg/mL of IMFINZI^{®}, and/or 200 nM of phorbol-12-myristate-13-acetate (PMA) was performed. The reaction was allowed to proceed for 48 hours, and then the production levels of TNF-α were measured using an ELISA kit. The results are illustrated in FIG. 36.

As illustrated in FIG. 36, it was identified that TNF-α was not produced in the comparative group treated with IMFINZI^{®}, which is a cancer immunotherapy, alone, and the production level of TNF-α increased in the experimental group treated with the anti-CD300c monoclonal antibody alone. In addition, it was identified that even in a case where THP-1 cells differentiated into M0 macrophages by treatment with PMA, the experimental group treated with the anti-CD300c monoclonal antibody exhibited a remarkably increased production level of TNF-α as compared with the experimental group treated with IMFINZI^{®}. From these results, it was found that the anti-CD300c monoclonal antibody promoted differentiation from M0 macrophages into M1 macrophages as compared with a conventional cancer immunotherapy.

### Experimental Example 5.3. Comparison of Capacity for Causing Differentiation into M1 Macrophages Between Anti-CD300c Monoclonal Antibody and Conventional Cancer Immunotherapy

In order to compare capacity for causing differentiation into M1 macrophages between the anti-CD300c monoclonal antibodies and conventional cancer immunotherapies, the production level of TNF-α was identified in the same manner as in Example 3.2. The results are illustrated in FIG. 37.

As illustrated in FIG. 37, it was identified that in a case where monocytes differentiated into M1 macrophages by treatment with LPS, the experimental group co-treated with IMFINZI^{®} and LPS did not exhibit a significant difference in production level of TNF-α, whereas the experimental group co-treated with the anti-CD300c monoclonal antibody and LPS exhibited a significant increase in production level of TNF-α as compared with the experimental group treated with the anti-CD300c monoclonal antibody alone.

### Experimental Example 5.4. Comparison of Cancer Cell Growth inhibitory Effects Between Anti-CD300c Monoclonal Antibody and Conventional Cancer Immunotherapy

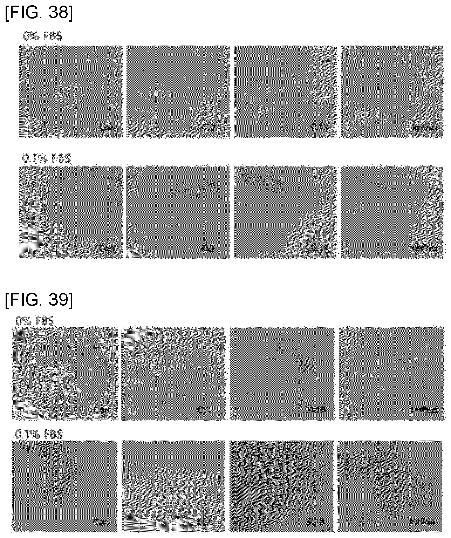

In order to compare cancer cell growth inhibitory effects of the anti-CD300c monoclonal antibody and a conventional cancer immunotherapy, cell growth inhibitory effects were identified using A549 (human lung cancer cell line) and MDA-MB-231 (human breast cancer cell line). More specifically, the respective cells were dispensed onto a 96-well plate at 2×10⁴ cells under a condition of 0% fetal bovine serum (FBS), and at 6×10³ cells under a condition of 0.1 % fetal bovine serum. Subsequently, treatment with 10 µg/mL of the anti-CD300c monoclonal antibody was performed and incubation was performed for 5 days. Then, observation was made under an optical microscope. The results are illustrated in FIGS. 38 and 39.

As illustrated in FIG. 38, it was identified that the anti-CD300c monoclonal antibody more effectively inhibited proliferation of cancer cells than IMFINZI^{®}, which is a cancer immunotherapy, in the A549 cell line.

As illustrated in FIG. 39, it was identified that the anti-CD300c monoclonal antibody more effectively inhibited proliferation of cancer cells than IMFINZI^{®}, which is a cancer immunotherapy, in the MDA-MB-231 cell line.

### Experimental Example 6. Identification of Anticancer Effects In Vivo of Anti-CD300c Monoclonal Antibody

### Experimental Example 6.1. Identification of Increase in Tumor-Associated Macrophages (TAMs)

In order to identify effects *in vivo* of the anti-CD300c monoclonal antibody CL7 on tumor-associated macrophages, a colorectal cancer cell line (CT26) at 2×10⁵ cells was transplanted by subcutaneous injection into 8-week-old BALB/c mice to prepare a syngeneic mouse tumor model. Breeding and experiments for animals were all conducted in a specific pathogen free (SPF) facility. 12 days after transplantation of the colorectal cancer cell line, the mice with a tumor size of 50 mm³ to 100 mm³ were respectively injected with the anti-CD300c monoclonal antibody, and injected with an equal amount of phosphate buffered saline (PBS) as a control group. The mice were intraperitoneally injected with 25 mg/kg of each material twice a week for two weeks over a total of 4 times. On day 25 after injection, the mice were sacrificed, and tumor tissues were collected from each of 6 mice in the CL7 25 mg/kg administration group in which the highest antitumor effect was observed as compared with the control group. The collected tumor tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with CD16/32 antibody (from Invitrogen), and the cells were stained with a staining solution for checking cell viability (from Invitrogen), and antibodies (from Abcam) to the total macrophage marker F4/80 and the M1 macrophage marker iNOS. Then, the data were read with CytoFLEX flow cytometer and analyzed with FlowJo software.

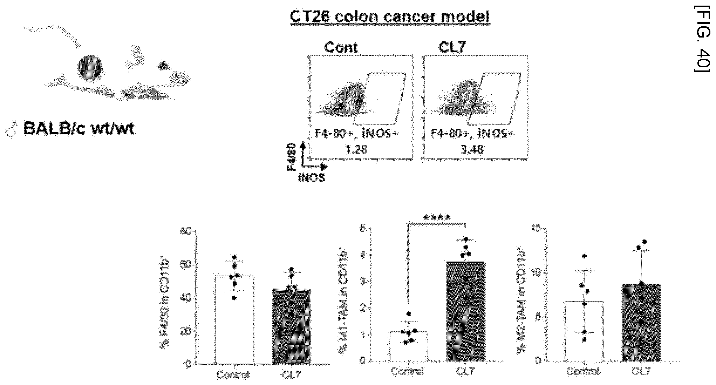

As a result, as illustrated in FIG. 40, it was identified that when treated with the anti-CD300c monoclonal antibody alone, the expression level of the M1 type tumor-associated macrophages increased in mouse cancer tissues. This means that administration of the anti-CD300c monoclonal antibody causes increased tumor-associated macrophages in cancer tissues, thereby inhibiting cancer growth.

### Experimental Example 6.2. Identification of Increase in Cytotoxic T Cells

In order to identify effects *in vivo* of the anti-CD300c monoclonal antibody CL7 on CD8+ T cells, a syngeneic mouse tumor model was prepared as in Experimental Example 6.1 and administered with the anti-CD300c monoclonal antibody at the same concentration. On day 25 after injection, the mice were sacrificed, and tumor tissues were collected from each of 6 mice in the CL7 25 mg/kg administration group in which the highest antitumor effect was observed as compared with the control group. The collected tumor tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with CD16/32 antibody (from Invitrogen), and the cells were stained with a staining solution for checking cell viability and CD8+ antibody (from Abcam) and CD4+ antibody (from Abcam). Then, the data were read with CytoFLEX flow cytometer and analyzed with FlowJo software.

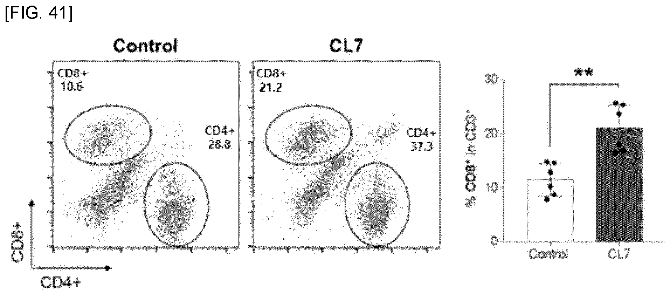

As a result, as illustrated in FIG. 41, it was identified that the number of CD8+ T cells in the tumor increased when treated with the anti-CD300C monoclonal antibody alone. This means that administration of the anti-CD300c monoclonal antibody causes increased intratumoral cytotoxic T cells, thereby exhibiting a cancer therapeutic effect.

### Experimental Example 6.3. Identification of Tumor-Specific Increase in Cytotoxic T Cells

In order to identify whether the anti-CD300c monoclonal antibody CL7 increases the number of CD8+ T cells in a tumor-specific manner, a syngeneic mouse tumor model was prepared as in Experimental Example 6.1 and administered with the anti-CD300c monoclonal antibody at the same concentration. On day 25 after injection, the mice were sacrificed, and tumor tissues were collected from each of 6 mice in the CL7 25 mg/kg administration group in which the highest antitumor effect was observed as compared with the control group. The collected tumor tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with CD16/32 antibody (from Invitrogen), and the cells were stained with a staining solution for checking cell viability and AH1 tetramer antibody (from Abcam). Then, the data were read with CytoFLEX flow cytometer and analyzed with FlowJo software.

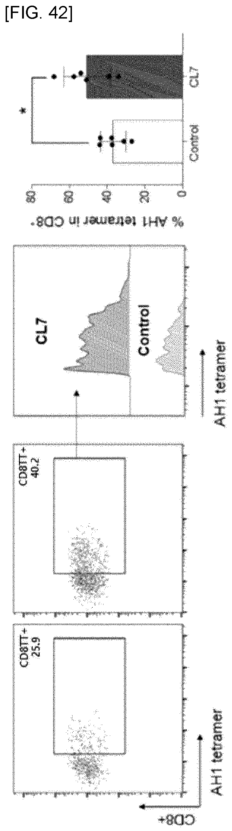

As a result, as illustrated in FIG. 42, it was identified that when treated with the anti-CD300c monoclonal antibody alone, the expression of AH1-tetramer, which is a CT26 tumor marker factor, increased in CD8+ T cells, indicating that the number of CD8+ T cells increased in a tumor (CT26)-specific manner. This means that administration of anti-CD300c monoclonal antibody causes increased CD8+ T cells to target and inhibit CT26 cancer cells.

### Experimental Example 6.4. Identification of Increased Activity of Cytotoxic T Cells

In order to identify effects *in vivo* of the anti-CD300c monoclonal antibody CL7 on CD8+ T cells, a syngeneic mouse tumor model was prepared as in Experimental Example 6.1 and administered with the anti-CD300c monoclonal antibody at the same concentration. On day 25 after injection, the mice were sacrificed, and spleens were collected from each of 6 mice in the CL7 25 mg/kg administration group in which the highest antitumor effect was observed as compared with the control group. Subsequently, the results were identified by measuring IFN-g through an ELISPOT assay. Specifically, the Mouse IFN-g ELISpot kit was purchased from R&D Systems (#EL485), and IFN-g was measured according to the kit's protocol.

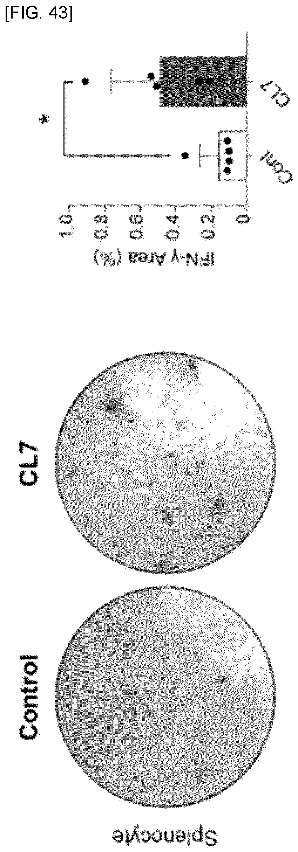

As a result, as illustrated in FIG. 43, it was identified that the expression of IFN-g increased when treated with the anti-CD300C monoclonal antibody alone. This means that administration of the anti-CD300C monoclonal antibody alone not only resulted in an increase in number of CD8+ T cells (see FIG. 41), but also an increase in activity of CD8+ T cells, by which cancer growth is inhibited in various ways to exhibit a cancer therapeutic effect.

### Experimental Example 6.5. Identification of Increase in Cytotoxic T Cells Relative to Regulatory T Cells

In order to identify effects *in vivo* of the anti-CD300c monoclonal antibody CL7 on an increase in cytotoxic T cells relative to regulatory T cells, a syngeneic mouse tumor model was prepared as in Experimental Example 6.1 and administered with the anti-CD300c monoclonal antibody at the same concentration. On day 25 after injection, the mice were sacrificed, and tumor tissues were collected from each of 6 mice in the CL7 25 mg/kg administration group in which the highest antitumor effect was observed as compared with the control group. The collected tumor tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with CD16/32 antibody (from Invitrogen), and the cells were stained with a staining solution for checking cell viability, antibodies to the Treg marker proteins CD25 (from Sino Biological) and Foxp3 (from Abcam), CD3+ antibody, and CD8+ antibody. Then, the data were read with CytoFLEX flow cytometer and analyzed with FlowJo software.

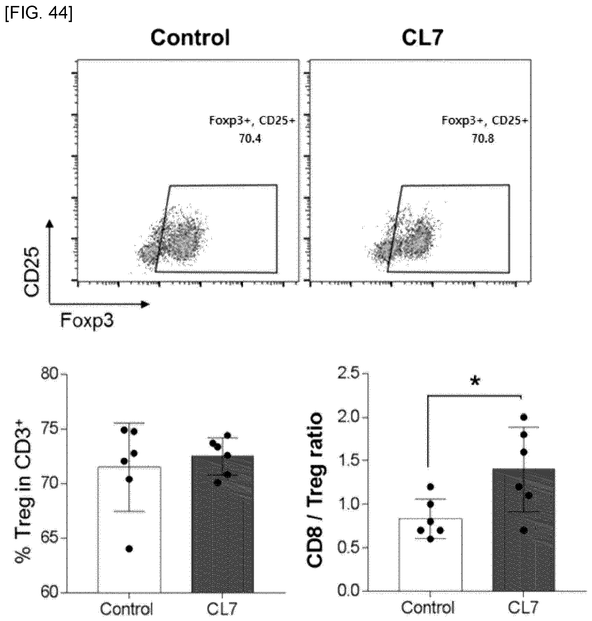

As a result, as illustrated in FIG. 44, it was identified that CD8+ T cells increased relative to Treg T cells in a case of being treated with the anti-CD300C monoclonal antibody alone. This means that the increased number of CD8+ T cells, which is caused by administration of the anti-CD300C monoclonal antibody, further inhibits cancer growth.

### Experimental Example 6.6. Identification of Effects on Cytotoxic T Cells, Regulatory T Cells, and Tumor-Associated Macrophages

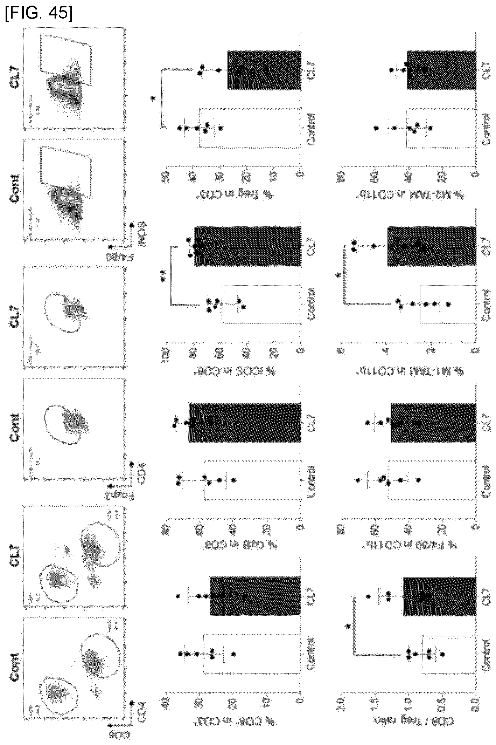

In order to identify effects of the anti-CD300c monoclonal antibody CL7 on cytotoxic T cells, regulatory T cells, and tumor-associated macrophages, the following experiment was performed. A syngeneic mouse tumor model was prepared as in Experimental Example 6.1. 12 days after transplantation of the colorectal cancer cell line, the mice with a tumor size of 50 mm³ to 100 mm³ were respectively injected with the anti-CD300c monoclonal antibody, and injected with an equal amount of phosphate buffered saline (PBS) as a control group. The mice were intraperitoneally injected with 25 mg/kg of each material twice a week for two weeks over a total of 4 times. On day 25 after injection, the mice were sacrificed, and tumor tissues were collected from each of 6 mice in the CL7 25 mg/kg administration group in which the highest antitumor effect was observed as compared with the control group. The collected tumor tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with a CD16/32 (Invitrogen) antibody, and the cells were stained with a staining solution for checking cell viability; and CD8+ antibody, which is a CD8+ T cell marker, and CD4+ antibody, Foxp3 antibody, which is a Treg cell marker, and CD4+ antibody, or antibodies (from Abcam) to the total macrophage marker F4/80 and the M1 macrophage marker iNOS. Then, the data were read with CytoFLEX flow cytometer and analyzed with FlowJo software. The results are illustrated in FIG. 45.

As illustrated in FIG. 45, it was identified that the anti-CD300c monoclonal antibody (CL7) significantly increased activated CD8+ T cells, inhibited regulatory T cells, and repolarized tumor-associated macrophages towards M1 phenotype.

### Experimental Example 6.7. Identification of Cancer Growth inhibitory Effects In Vivo

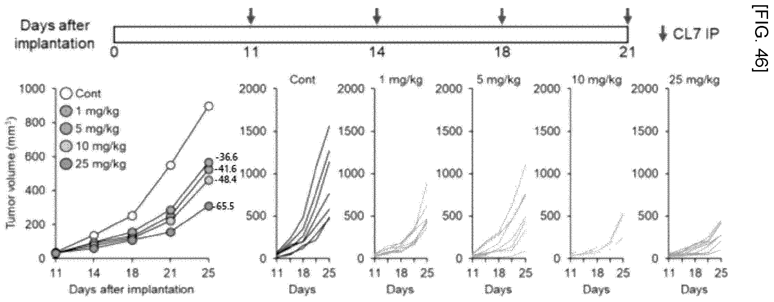

In order to identify anticancer effects *in vivo* of the anti-CD300c monoclonal antibody CL7, a colorectal cancer cell line (CT26) at 2×10⁵ cells was transplanted by subcutaneous injection into 8-week-old BALB/c mice to prepare a syngeneic mouse tumor model. Breeding and experiments for animals were all conducted in a SPF facility. On day 11 (D11) after transplantation of the colon cancer cell line, the mice with a tumor size of 50 mm³ to 100 mm³ were administered with 1 mg/kg, 5 mg/kg, 10 mg/kg, or 25 mg/kg of the anti-CD300c monoclonal antibody, respectively, and administered with an equal amount of phosphate buffered saline (PBS) as a control group. Specifically, the mice were intraperitoneally injected with each dose twice a week for two weeks (a total of 4 times on D11, D14, D18, and D21). The tumor volume was measured for 25 days. The results are illustrated in FIG. 46.

As illustrated in FIG. 46, it was identified that the anti-CD300c monoclonal antibody CL7 resulted in delayed growth of CT26 colorectal cancer in a dose-dependent manner.

### IV. Changes in Expression of Biomarkers Caused by Administration of Anti-CD300c Monoclonal Antibody

### Example 2. Changes in Expression of Immune Cell-Related Markers and Tumor Microenvironment-Related Markers Caused by Administration of Anti-CD300c Monoclonal Antibody

### Example 2.1. Nanostring Immune Profiling

In order to identify changes in expression of immune cell- and tumor microenvironment-related markers in a case where a solid cancer model is administered with the anti-CD300c monoclonal antibody (CL7) produced in Example 1, a colorectal cancer cell line (CT26) at 2×10⁵ cells was transplanted by subcutaneous injection into 8-week-old BALB/c mice to prepare a syngeneic mouse tumor model. Breeding and experiments for animals were all conducted in a SPF facility. 12 days after transplantation of the colorectal cancer cell line, the mice with a tumor size of 50 mm³ to 100 mm³ were respectively administered with the anti-CD300c monoclonal antibody, and administered with an equal amount of phosphate buffered saline (PBS) as a control group. The mice were intraperitoneally injected with 25 mg/kg of each material twice a week for two weeks over a total of 4 times. On day 25 after injection, the mice were euthanized to prepare tumor tissues. RNA was extracted and purified therefrom, and changes in dendritic cell markers, macrophage markers, tumor microenvironment (TME) markers, Th1 response markers, or Th2 response markers were identified through Nanostring Immune profiling.

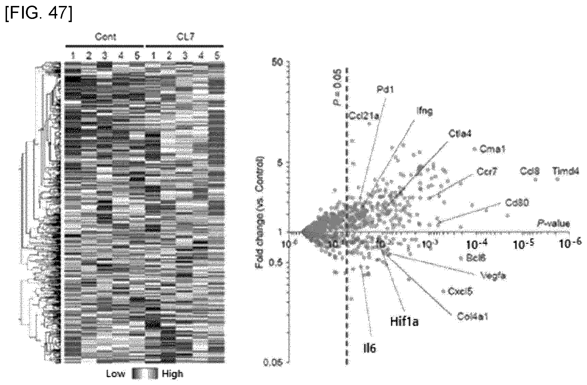

The Nanostring Immune profiling results are illustrated in FIG. 47. From these results, it was identified that administration of the anti-CD300c monoclonal antibody extensively reprogrammed the tumor immune microenvironment.

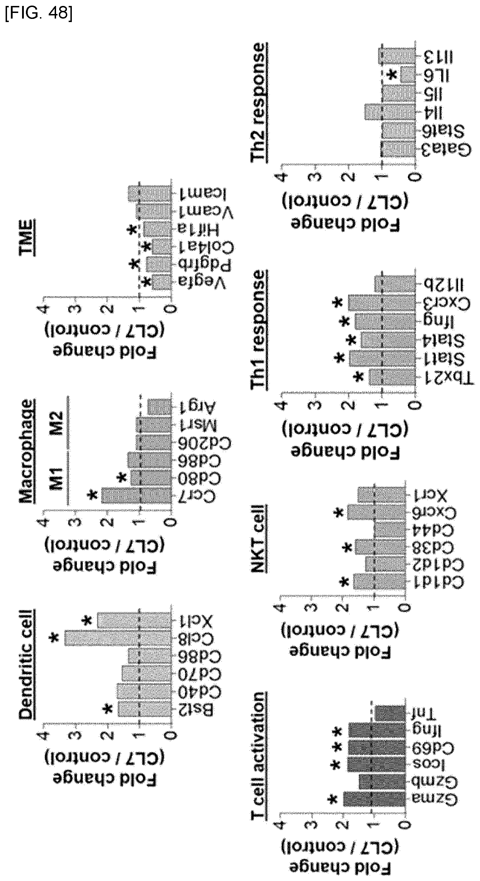

In addition, FIG. 48 illustrates the results obtained by observing, relative to the control group, changes in dendritic cell markers, macrophage markers, tumor microenvironment markers, Th1 response markers, or Th2 response markers which were caused by administration of the anti-CD300c monoclonal antibody. As illustrated in FIG. 48, it was identified that administration of the anti-CD300c monoclonal antibody resulted in significantly increased the expression of Bst2, CCL8, and Xcl1 which are dendritic cell markers; significantly increased the expression of CCR7 and CD80 which are M1 macrophage markers; decreased the expression of vegfa, pdgfrb, Col4a1, and Hif1a which help cancer growth in the tumor microenvironment; and increased the expression of Tbx21, Stat1, Stat4, Ifn-g, and Cxcr3 which are markers that can identify the Th1 response.

### Example 2.2. Changes in Expression of Immune Checkpoint Markers

Based on the Nanostring Immune profiling results obtained in Example 2.1, it was identified which immune checkpoint markers show a significant difference in expression as compared with the control group in a case where a syngeneic mouse tumor model is administered with the anti-CD300c monoclonal antibody.

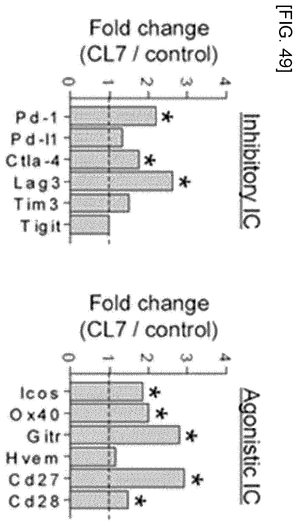

The results are illustrated in FIG. 49. It was identified that administration of the anti-CD300c monoclonal antibody resulted in increased expression of PD-1, CTLA-4, and Lag3 which are inhibitory immune checkpoints (ICs), and increased expression of ICOS, OX40, Gitr, Cd27, and Cd28 which are agonistic ICs.

These results are of considerable significance in that they can provide useful information on which immune checkpoint-related immunotherapy should be selected in a case where the anti-CD300c monoclonal antibody and an additional immunotherapy are co-administered to obtain further enhanced anticancer efficacy.

### V. Combination of Anti-CD300c Monoclonal Antibody and Immunotherapy

### Example 3. Co-Administration of Anti-CD300c Monoclonal Antibody (CL7) and Immunotherapy

The anti-CD300c monoclonal antibody (CL7) produced in Example 1 was used in combination with other cancer immunotherapies, for example, the anti-PD-L1 antibodies IMFINZI^{®} and OPDIVO^{®}, and the anti-PD-1 antibody KEYTRUDA@, an anti-CD47 antibody (αCD47), and an anti-CTLA-4 antibody. Then, the results were obtained.

The manufacturers of the respective immunotherapies are as follows: IMFINZI^{®} (AstraZeneca); OPDIVO^{®} and the anti-CTLA-4 antibody (Bristol Myers Squibb Company); KEYTRUDA@ (Merck Sharp & Dohme); and the anti-CD47 antibody (Abcam).

### Experimental Example 7. Identification of (Synergistically) Increased Macrophage Activity Caused by Combination

### Experimental Example 7.1. Identification of Increased M1 Macrophages

In order to identify, through cell morphology, the pattern of differentiation into M1 macrophages in a case where monocytes are co-treated with the anti-CD300c monoclonal antibody CL7 produced in Example 1 and a cancer immunotherapy such as IMFINZI^{®} that is an anti-PD-L1 antibody, KEYTRUDA^{®} that is an anti-PD-1 antibody, and an anti-CD47 antibody (αCD47), THP-1 (human monocyte cell line) was treated with the anti-CD300c monoclonal antibody and the cancer immunotherapy, respectively, at 10 µg/mL alone or in combination. Incubation was performed for 48 hours, and then the cell shape was observed under a microscope.

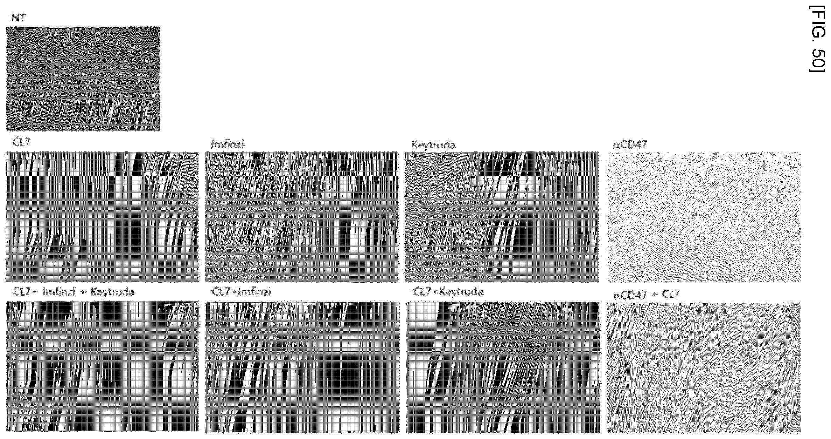

As a result, as illustrated in FIG. 50, it was identified that the THP-1 cells changed their shape from suspension cells to circular adherent cells that are in the shape of M1 macrophages, in a case where co-treatment with the anti-CD300c monoclonal antibody and the cancer immunotherapy was performed as compared with a case where treatment with the cancer immunotherapy alone was performed. From these results, it was identified that differentiation of monocytes into M1 macrophages was further promoted by co-treatment with the anti-CD300c monoclonal antibody and the cancer immunotherapy.

### Experimental Example 7.2. Identification of Increased M1 Macrophage Markers

In order to identify whether induction of differentiation of monocytes into M1 macrophages increases in a case where monocytes are co-treated with the anti-CD300c monoclonal antibody CL7 produced in Example 1 and a cancer immunotherapy such as IMFINZI^{®} that is an anti-PD-L1 antibody, OPDIVO^{®} that is an anti-PD-1 antibody, KEYTRUDA^{®} that is an anti-PD-1 antibody, an anti-CD47 antibody (αCD47), and an anti-CTLA-4 antibody, THP-1 was dispensed onto a 96-well plate at 1.5×10⁴ cells/well and treated with the anti-CD300c monoclonal antibody and the cancer immunotherapy, respectively, at 10 µg/mL alone or in combination. The reaction was allowed to proceed for 48 hours, and the production levels of tumor necrosis factor-α (TNF-α), IL-1b, and IL-8, which are differentiation markers of M1 macrophages, were measured using an ELISA kit (Human TNF-α Quantikine kit, R&D Systems).

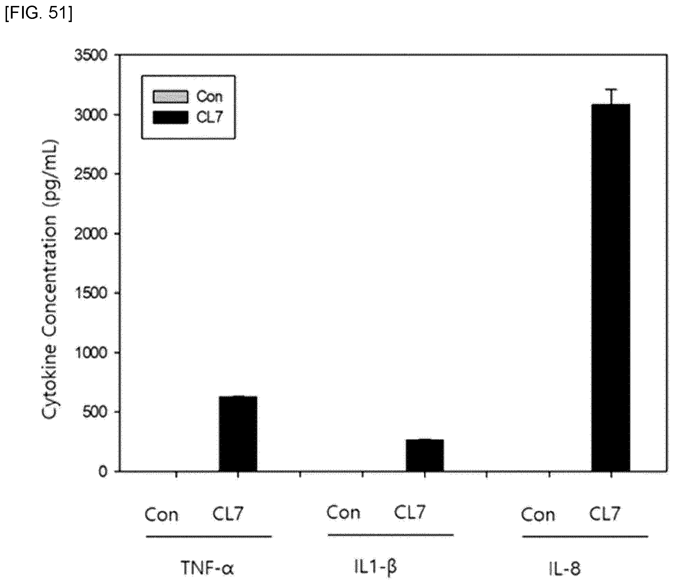

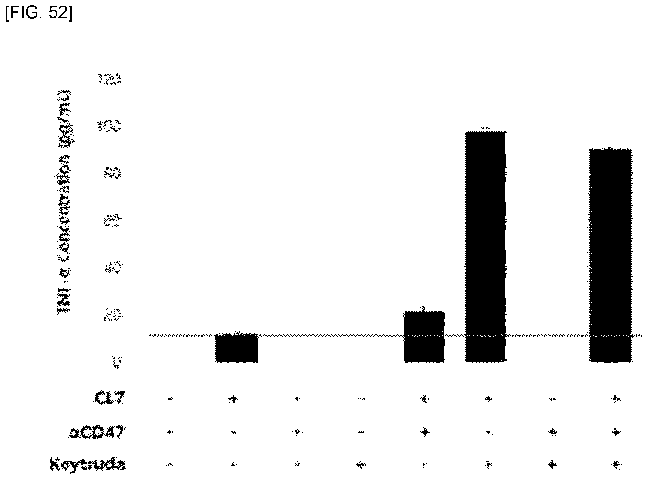

As a result, as illustrated in FIG. 51, it was identified that the production levels of all three differentiation markers increased in a case where treatment with the anti-CD300c monoclonal antibody alone was performed, in which the production level of IL-8 particularly significantly increased. In addition, as illustrated in FIG. 52, it was identified that the production levels of TNF-α, which is a differentiation marker of M1 macrophages, further increased in a case where co-treatment with the anti-CD300c monoclonal antibody and at least one of IMFINZI^{®}, OPDIVO^{®}, KEYTRUDA@, and αCD47 was performed as compared with a case where treatment with the anti-CD300c monoclonal antibody alone was performed. From these results, it was identified that more monocytes were differentiated into M1 macrophages in a case where co-treatment with the anti-CD300c monoclonal antibody and the anti-PD-1 antibody and/or the anti-CD47 antibody than a case where treatment with the anti-CD300c monoclonal antibody alone was performed.

### Experimental Example 7.3. Identification of Decreased M2 Macrophage Markers

In order to identify whether induction of differentiation of monocytes into M2 macrophages decreases in a case where monocytes are co-treated with the anti-CD300c monoclonal antibody and a cancer immunotherapy such as IMFINZI^{®}, OPDIVO^{®}, KEYTRUDA@, anti-CTLA-4, or αCD47, THP-1 was dispensed onto a 96-well plate at 1.5×10⁴ cells/well and pre-treated with 320 nM PMA for 6 hours. Then, treatment with the anti-CD300c monoclonal antibody and the immunotherapy, respectively, at 10 µg/mL alone or in combination was performed together with treatment with interleukin-4 (IL-4) and interleukin-13 (IL-13) at 20 ng/mL. The reaction was allowed to proceed for 48 hours. Then, the production levels of IL-10 and IL-12, which are differentiation markers of M2 macrophages, were measured using an ELISA kit (R&D Systems).

As a result, it was identified that the production levels of IL-10 and IL-12 further decreased by 30% or higher in a case where co-treatment with the anti-CD300c monoclonal antibody and at least one of IMFINZI^{®}, OPDIVO^{®}, KEYTRUDA@, and αCD47 was performed as compared with a case where treatment with the anti-CD300c monoclonal antibody alone was performed.

### Experimental Example 7.4. Identification of Increased Capacity for Causing Differentiation into M1 Macrophages

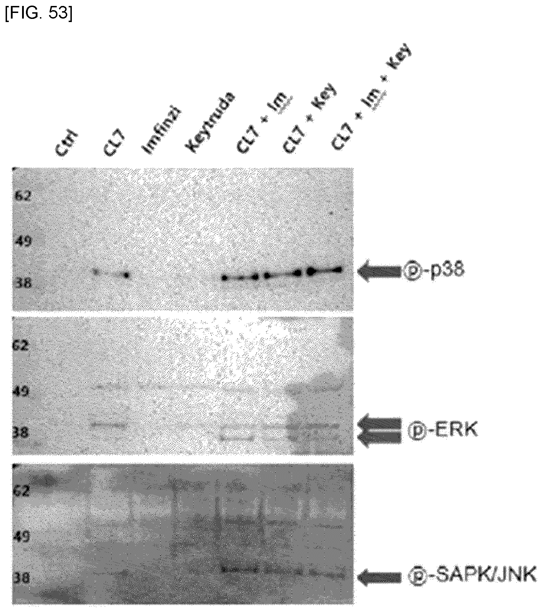

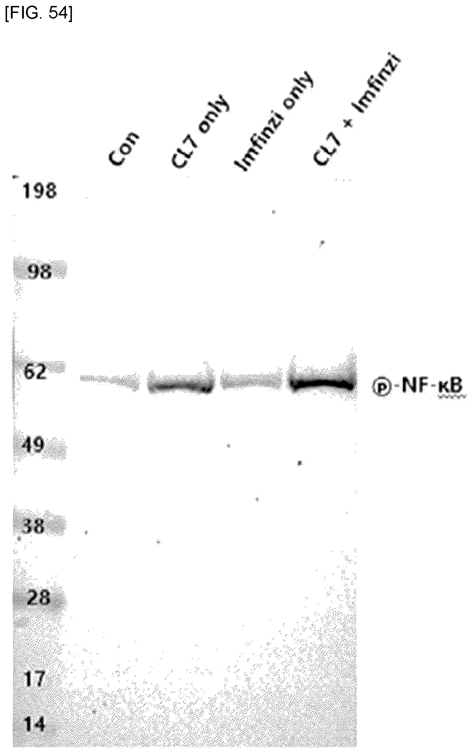

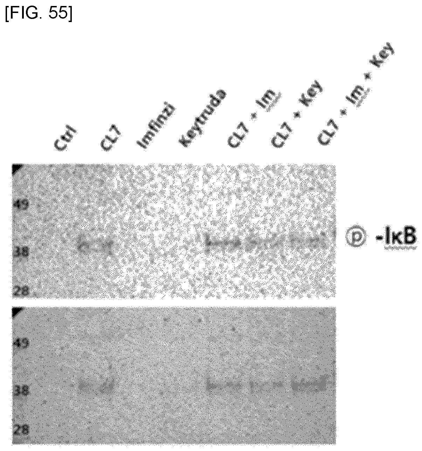

In order to identify whether differentiation of monocytes into M1 macrophages increases in a case where the monocytes are co-treated with the anti-CD300c monoclonal antibody CL7 and a cancer immunotherapy such as an anti-PD-1 antibody, an anti-PD-L1 antibody, an anti-CTLA-4 antibody, and an anti-CD47 antibody, signal transduction of mitogen-activated protein kinase (MAPK), IκB, and NF-kB, which are representative signals of M1 macrophage differentiation, was checked. Specifically, THP-1 was dispensed onto a 6-well plate at 8.8×10⁵ cells/well, and treated with 10 µg/mL of the anti-CD300c monoclonal antibody, 10 µg/mL of IMFINZI^{®} and/or 10 µg/mL of KEYTRUDA@. For a control group, treatment with the same amount of phosphate buffer solution (PBS) was performed. Incubation was performed for 48 hours. Then, Western blotting was conducted to identify phosphorylated SAPK/JNK, phosphorylated ERK, phosphorylated p38 for MAPK signal, phosphorylated NF-kB for NF-kB signal, and phosphorylated IkB for IkB signal. The results are illustrated in FIGS. 53 to 55.

FIGS. 53, 54, and 55 illustrate the results obtained by identifying signal transduction of MAPK, NF-Kb, and IkB, respectively. It was identified that the levels of phosphorylated MAPK, IkB, and NF-kB increased in a case where THP-1 was co-treated with the anti-CD300c monoclonal antibody and a cancer immunotherapy such as an anti-PD-1 antibody, an anti-PD-L1 antibody, an anti-CTLA-4 antibody, and an anti-CD47 antibody as compared with a case where THP-1 was treated with the anti-CD300c monoclonal antibody alone. From these results, it was identified that cell signaling representing differentiation into M1 macrophages increased in a case where THP-1 was co-treated with the anti-CD300c monoclonal antibody and a cancer immunotherapy such as an anti-PD-1 antibody, an anti-PD-L1 antibody, an anti-CTLA-4 antibody, and an anti-CD47 antibody as compared with a case where THP-1 was treated with the anti-CD300c monoclonal antibody alone.

### Experimental Example 8. Identification of (Synergistically) Increased Cancer Cell Inhibitory Effects Caused by Combination (In vitro)

### Experimental Example 8.1. Identification of Apoptosis Signals

It was identified whether apoptosis signals increase in a case where co-treatment with the anti-CD300c monoclonal antibody CL7 and a cancer immunotherapy such as an anti-PD-1 antibody, an anti-PD-L1 antibody, an anti-CTLA-4 antibody, and an anti-CD47 antibody is performed. Specifically, A549 was dispensed onto a 6-well plate at 8×10⁵ cells/well, and treated with 10 µg/mL of the anti-CD300c monoclonal antibody, and 10 µg/mL of IMFINZI^{®}, KEYTRUDA@, OPDIVO^{®}, and an anti-CD47 antibody alone or in combination. Incubation was performed for 48 hours, and then Western blotting was conducted to identify apoptosis signals or cell cycle signals. Cleaved caspase-9, caspase-3, caspase-2, and caspase-8 were identified as markers for the apoptosis signals, and cyclin D1, CDK2, p27kip1, CDK6, cyclin D3, P21 Waf1, Cip1, and the like were identified as markers for the cell cycle signals.

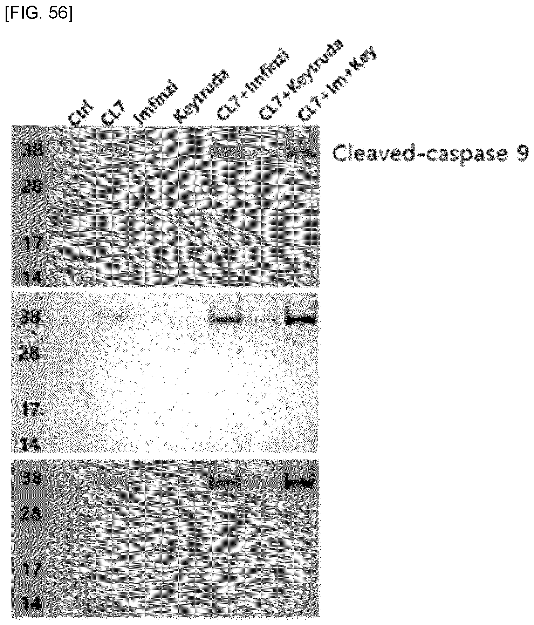

As illustrated in FIG. 56, the apoptosis signals increased in a case where co-treatment with the anti-CD300c monoclonal antibody and IMFINZI^{®} that is an anti-PD-1 antibody was performed as compared with a case where treatment with the anti-CD300c monoclonal antibody alone was performed; and the levels of cleaved-caspase9 and p21 increased and the level of cyclin D1 decreased in a case where co-treatment with the anti-CD300c monoclonal antibody and an immunotherapy such as an anti-PD-1 antibody, an anti-PD-L1 antibody, an anti-CTLA-4 antibody, and an anti-CD47 antibody was performed. From these results, it was identified that apoptosis of cancer cells was better induced in a case where co-treatment with the anti-CD300c monoclonal antibody and an immunotherapy such as anti-PD-1 antibody, anti-PD-L1 antibody, anti-CTLA-4 antibody, and anti-CD47 antibody was performed than a case where treatment with the anti-CD300c monoclonal antibody alone was performed.

### Experimental Example 8.2. Identification of Growth Inhibitory Effects on Cancer Cell Lines

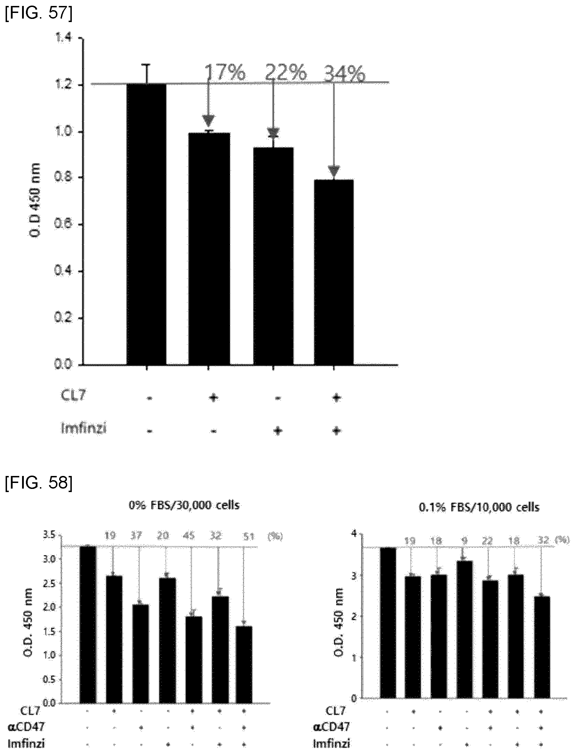

In order to identify cancer cell growth inhibitory effects caused by co-administration of the anti-CD300c monoclonal antibody CL7 and a cancer immunotherapy, comparison of cancer cell growth inhibitory effects was performed using A549 (human lung cancer cell line) and MDA-MB-231 (human breast cancer cell line). Specifically, the cells were dispensed onto a 96-well plate at 2×10⁴ cells (A549) or 3×10⁴cells (MDA-MB-231) under a condition without fetal bovine serum (FBS), and at 6×10³ cells (A549) or 1×10⁴ cells (MDA-MB-231) under a condition of 0.1% fetal bovine serum. Subsequently, the cells were treated with the anti-CD300c monoclonal antibody and IMFINZI^{®} at 10 µg/mL alone or in combination, and incubation was performed for 5 days. For a control group, treatment with the same amount of phosphate buffer solution (PBS) was performed. Then, treatment with CCK-8 (DOJINDO) was performed, and the absorbance was measured at OD 450 nm. The results are illustrated in FIG. 57 (A549) and FIG. 58 (MDA-MB-231).

As illustrated in FIG. 57, it was identified for the A549 cell line that under a condition without FBS, as compared with the control, a 17% higher cell growth inhibitory effect was observed in a case where treatment with the anti-CD300c monoclonal antibody alone was performed, and a 34% higher cell growth inhibitory effect was observed in a case where co-treatment with the anti-CD300c monoclonal antibody and IMFINZI^{®} was performed.

As illustrated in FIG. 58, it was identified for the MDA-MB-231 cell line that under a condition of 0.1% FBS, as compared with the control, a 19% higher cell growth inhibitory effect was observed in a case where treatment with the anti-CD300c monoclonal antibody alone was performed; a 45% higher cell growth inhibitory effect was observed in a case where co-treatment with the anti-CD300c monoclonal antibody and the anti-CD47 antibody was performed; and a 51% higher cell growth inhibitory effect was observed in a case where co-treatment with the anti-CD300c monoclonal antibody, the anti-CD47 antibody, and IMFINZI^{®} was performed. Under a condition of 0.1% FBS, as compared with the control, a 19% higher cell growth inhibitory effect was observed in a case where treatment with the anti-CD300c monoclonal antibody alone was performed; a 22% higher cell growth inhibitory effect was observed in a case where co-treatment with the anti-CD300c monoclonal antibody and the anti-CD47 antibody was performed; and a 32% higher cell growth inhibitory effect was observed in a case where co-treatment with the anti-CD300c monoclonal antibody, the anti-CD47 antibody, and IMFINZI^{®} was performed.

From these results, it was identified that growth of the cancer cells was further inhibited in a case where co-treatment with the anti-CD300c monoclonal antibody and an immunotherapy such as an anti-PD-1 antibody, an anti-PD-L1 antibody, an anti-CTLA-4 antibody, and an anti-CD47 antibody was performed as compared with a case where treatment with the anti-CD300c monoclonal antibody alone was performed.

### Experimental Example 9. Identification of (Synergistically) Increased Anticancer Effects In Vivo Caused by Combination (Colon Cancer Mouse Model)

### Experimental Example 9.1. Identification of Cancer Growth Inhibitory Effects In Vivo

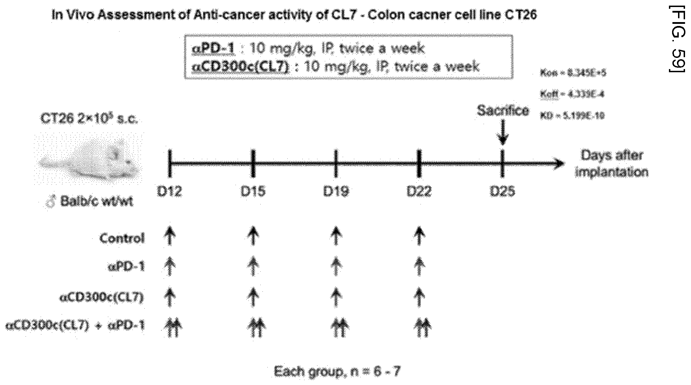

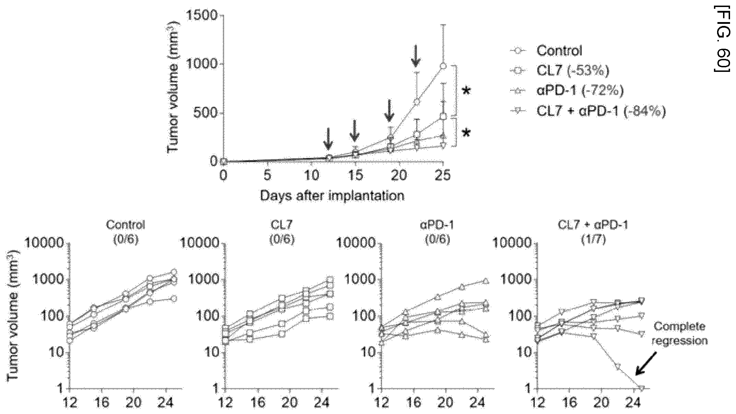

In order to identify anticancer effects *in vivo* of the anti-CD300c monoclonal antibody CL7, a colorectal cancer cell line (CT26) at 2×10⁵ cells was transplanted by subcutaneous injection into 8-week-old BALB/c mice to prepare a syngeneic mouse tumor model. Breeding and experiments for animals were all conducted in a SPF facility. On day 12 (D12) after transplantation of the colon cancer cell line, the mice with a tumor size of 50 mm³ to 100 mm³ were administered with the anti-CD300c monoclonal antibody and an anti-PD-1 antibody purchased from BioXcell alone or in combination, and administered with an equal amount of phosphate buffered saline (PBS) as a control group. A schematic experimental method is illustrated in FIG. 59. Specifically, the mice were intraperitoneally injected with the respective antibodies (CL7: 10 mg/kg; and anti-PD-1 antibody: 10 mg/kg) alone or in combination, twice a week for two weeks (a total of 4 times on D12, D15, D19, and D22). The tumor volume was measured for 25 days. The results are illustrated in FIG. 60.

As can be seen from FIG. 60, it was identified that although cancer growth was inhibited relative to the control group even in the experimental group administered with the anti-CD300c monoclonal antibody alone, cancer growth was more effectively inhibited in a case where co-treatment with the anti-CD300c monoclonal antibody and an immunotherapy such as an anti-PD-1 antibody was performed than a case where treatment with the anti-CD300c monoclonal antibody alone was performed.

### Experimental Example 9.2. Identification of Increased Tumor-Infiltrating Lymphocytes Under Tumor Microenvironment In Vivo

In order to identify effects of the anti-CD300c monoclonal antibody on tumor-infiltrating lymphocytes (TIL) in a tumor microenvironment (TME), on day 25 of the experiment performed in the same manner as in Experimental Example 9.1, the mice were euthanized, injected intravascularly with 1% paraformaldehyde (PFA), and then perfusion was performed to obtain cancer tissue. The obtained cancer tissue was fixed using 1% PFA, and sequentially dehydrated using 10%, 20%, and 30% sucrose solutions. The dehydrated cancer tissue was frozen in OCT compound (optimal cutting temperature compound), and then the cancer tissue was sectioned to a thickness of 50 µm using a cryotome. The tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh to make them single cells. To suppress non-specific reactions in the single cell suspension, a reaction with a CD16/32 antibody (Invitrogen) was allowed to proceed for 1 hour, cell viability was checked, and staining of CD8+ T cells and CD31+ tumor blood vessel cells, which are tumor-infiltrating lymphocyte markers, was performed.

As a result, it was identified that CD8+ T cells increased in the experimental group co-administered with the anti-CD300c monoclonal antibody and the anti-PD-1 antibody, the anti-PD-L1 antibody, the anti-CTLA-4 antibody, or the anti-CD47 antibody, as compared with the experimental group administered with the anti-CD300c monoclonal antibody alone. From these results, it was found that the number of tumor-infiltrating lymphocytes increased in a tumor microenvironment to exert an anticancer effect in a case where co-treatment with the anti-CD300c monoclonal antibody and the anti-PD-1 antibody, the anti-PD-L1 antibody, the anti-CTLA-4 antibody, or the anti-CD47 antibody was performed, as compared with a case where treatment with the anti-CD300c monoclonal antibody alone was performed.

### Experimental Example 9.3. Identification of Effect of Increasing M1 Macrophages In Vivo

In order to identify whether the anti-CD300c monoclonal antibody increases M1 macrophages in cancer tissue of a mouse model, the cancer tissue sections prepared in the same manner as in Experimental Example 9.2 were stained with antibodies to the M1 macrophage marker iNOS and the M2 macrophage marker CD206, and checked by FACS.

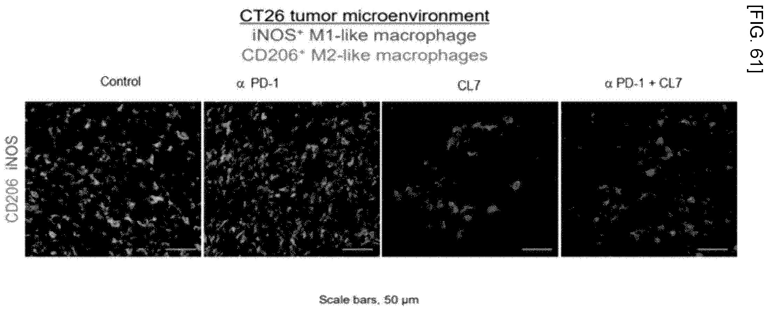

As a result, as illustrated in FIG. 61, it was identified that as compared with the control group, M1 macrophages partially increased in the experimental group treated with the anti-PD-1 antibody, and M1 macrophages remarkably increased, but M2 macrophages were hardly observed in the experimental group treated with the anti-CD300c monoclonal antibody. In addition, it was identified that M1 macrophages further increased in the experimental group co-administered with the anti-CD300c monoclonal antibody and the anti-PD-1 antibody. From these results, it was identified that differentiation into M1 macrophages could be effectively promoted in a case where co-treatment with the anti-CD300c monoclonal antibody and an immunotherapy, such as an anti-PD-1 antibody, an anti-PD-L1 antibody, an anti-CTLA-4 antibody, or an anti-CD47 antibody, was performed, than a case where treatment with the anti-CD300c monoclonal antibody alone was performed.

### Experimental Example 9.4. Identification of Effect of Promoting CD8+ T Cell Immunity In Vivo

In order to identify whether the anti-CD300c monoclonal antibody CL7 promotes CD8+ T cell immunity in a mouse tumor model, on day 25 of the experiment performed in the same manner as in Experimental Example 9.1, the mice were euthanized, injected intravascularly with 1% paraformaldehyde (PFA), and then perfusion was performed to obtain cancer tissue. The obtained cancer tissue was fixed using 1% PFA, and sequentially dehydrated using 10%, 20%, and 30% sucrose solutions. The dehydrated cancer tissue was frozen in OCT compound (optimal cutting temperature compound), and then the cancer tissue was sectioned to a thickness of 50 µm using a cryotome. Then, staining was performed on CD8+ and iNOS.

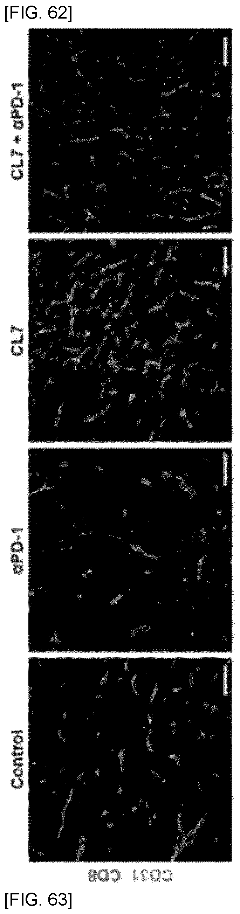

As illustrated in FIG. 62, it was identified that as compared with the control group, CD8+ T cells partially increased in the experimental group treated with the anti-PD-1 antibody, and CD8+ T cells remarkably increased in the experimental group treated with the anti-CD300c monoclonal antibody. In addition, it was identified that as compared with the group administered with the anti-PD-1 antibody alone, CD8+ T cells further increased in the experimental group co-administered with the anti-CD300c monoclonal antibody and the anti-PD-1 antibody. From these results, it was found that the anti-CD300c monoclonal antibody more effectively increased the number of CD8+ T cells when used in combination with a conventional cancer immunotherapy.

### Experimental Example 9.5. Identification of Effect of Increasing Immune Cell Activity In Vivo

In order to identify whether co-administration of the anti-CD300c monoclonal antibody and an immunotherapy increases activity of immune cells, spleens were obtained in the same manner as in Experimental Example 9.1 from mice co-administered with the anti-CD300c monoclonal antibody and an anti-PD-1 antibody, an anti-CTLA-4 antibody, an anti-KIR antibody, an anti-LAG3 antibody, an anti-CD137 antibody, an anti-OX40 antibody, an anti-CD276 antibody, an anti-CD27 antibody, an anti-GITR antibody, an anti-TIM3 antibody, an anti-41BB antibody, an anti-CD226 antibody, an anti-CD40 antibody, an anti-CD70 antibody, an anti-ICOS antibody, an anti-CD40L antibody, an anti-BTLA antibody, an anti-TCR antibody, an anti-TIGIT antibody, or an anti-CD47 antibody. The obtained spleens were FACS-stained with various markers that can detect T cell activity and NKT cell activity as described in Experimental Example 9.2, and checked through MFI.

As a result, it was identified that in a case where co-administration of the anti-CD300c monoclonal antibody and the above-mentioned immunotherapy was performed, Gzma, Icos, CD69, and Ifng, which are T cell activation markers, increased, and Cd11, CD38, and cxcr6, which are NKT cell activation markers, significantly increased. From these results, it was identified that T cells and NKT cells were further activated in a case where co-treatment with the anti-CD300c antibody and the immunotherapy, as compared with a case where treatment with the anti-CD300c antibody alone, was performed.

### Experimental Example 9.6. Identification of Effect of Inhibiting Treg Cells In Vivo

In order to identify changes in pattern of Treg cells that cause immune-suppressing responses in a case where co-administration of the anti-CD300c monoclonal antibody and an immunotherapy such as an anti-PD-1 antibody, an anti-PD-L1 antibody, an anti-CTLA-4 antibody, an anti-KIR antibody, an anti-LAG3 antibody, an anti-CD137 antibody, an anti-OX40 antibody, an anti-CD276 antibody, an anti-CD27 antibody, an anti-GITR antibody, an anti-TIM3 antibody, an anti-41BB antibody, an anti-CD226 antibody, an anti-CD40 antibody, an anti-CD70 antibody, an anti-ICOS antibody, an anti-CD40L antibody, an anti-BTLA antibody, an anti-TCR antibody, an anti-TIGIT antibody, or an anti-CD47 antibody was performed in a solid cancer model, T cells were extracted from the spleen tissue prepared in the same manner as in Experimental Example 9.2 and FACS staining was performed to check the number of Treg cells, which express FOXP3, among CD3+ T cells.

Given the proportion of Treg cells in CD3+ cells, it was identified that a remarkably decreased proportion of Treg cells was observed in a case where co-administration of the anti-CD300c monoclonal antibody and the immunotherapy was performed as compared with a case where administration of each immunotherapy alone was performed, wherein the remarkably decreased proportion of Treg cells indicates that activation of T cells attacking cancer cells was induced.

### Experimental Example 10. Identification of (Synergistically) Increased Anticancer Effects In Vivo Caused by Combination (Melanoma Mouse Model)

### Experimental Example 10.1. Identification of Cancer Growth inhibitory Effects In Vivo

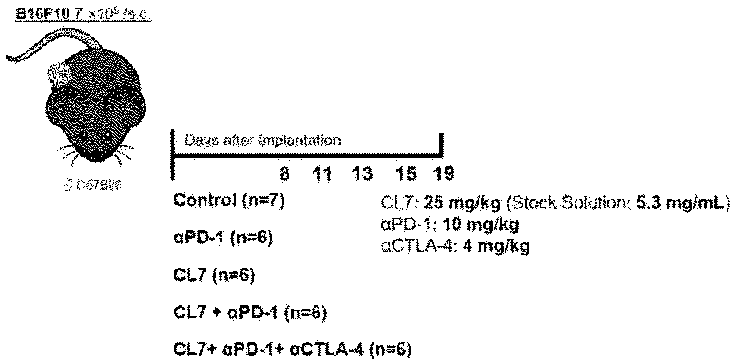

In order to identify whether the anti-CD300c monoclonal antibody CL7 is effective in other carcinomas in addition to a CT26 colorectal cancer mouse model, an additional experiment was conducted with a melanoma mouse model. B16F10 melanoma cells at 7×10⁵ cells were transplanted by subcutaneous injection into 8-week-old male C57BL/6 mice to prepare a syngeneic mouse tumor model. Animal breeding and experiments were all conducted in a SPF facility. On day 8 after transplantation of the melanoma cell line, the mice with a tumor size of 50 mm³ to 100 mm³ were intraperitoneally injected with 25 mg/kg of CL7, 10 mg/kg of α-PD-1, and 4 mg/kg of α-CTLA4. A schematic experimental method is illustrated in FIG. 63. More specifically, the mice were injected, twice a week for 2 weeks (over a total of 4 times), with each of the anti-CD300c monoclonal antibody, the anti-PD-1 antibody, the anti-CD300c monoclonal antibody+the anti-PD-1 antibody (Combo), and the anti-CD300c monoclonal antibody+the anti-PD-1 antibody+the anti-CTLA-4 antibody (Triple), and injected with phosphate buffered saline (PBS), whose amount is the same as the anti-CD300c monoclonal antibody, as a control group. The cancer size was measured for 20 days.

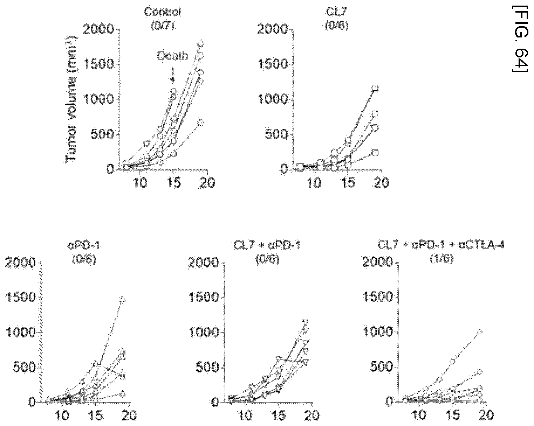

As a result, as illustrated in FIG. 64, it was identified that although cancer growth was inhibited even by administration of the anti-CD300c monoclonal antibody alone, cancer growth was more effectively inhibited in the group co-administered with the anti-CD300c monoclonal antibody and the anti-PD-1 antibody+the anti-CTLA-4 antibody.

### Experimental Example 10.2. Identification of Increased Cytotoxic T Cells

In order to identify effects *in vivo,* on CD8+ T cells, of combination of the anti-CD300c monoclonal antibody CL7 with an anti-PD-1 antibody and/or an anti-CTLA-4 antibody in a B16F10 melanoma model, a mouse tumor model was prepared as in Experimental Example 10.1 and injected with each test substance at the same concentration.

Tumor tissues were collected from 6 mice in each group. The collected tumor tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with CD16/32 antibody (from Invitrogen), and the cells were stained with a staining solution for checking cell viability (from Invitrogen), and a CD8+ antibody and a CD4+ antibody. Then, the data were read with CytoFLEX flow cytometer and analyzed with FlowJo software.

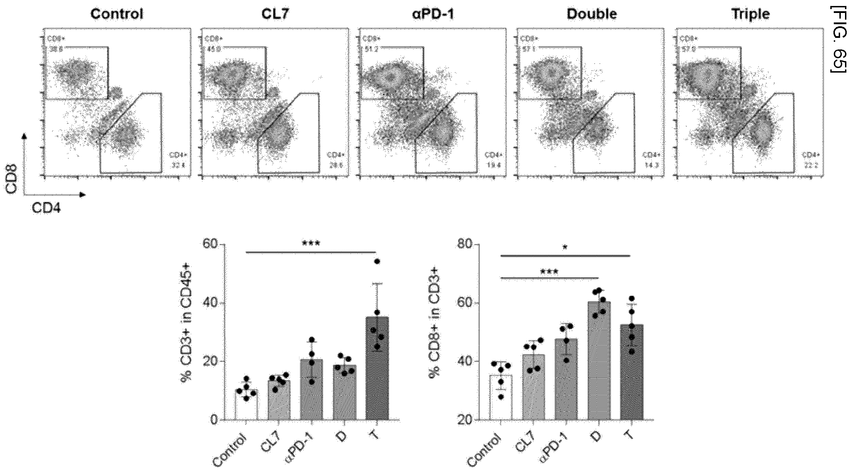

As a result, as illustrated in FIG. 65, it was identified that as in the CT26 carcinoma model (Experimental Example 6.2), the number of CD8+ T cells increased even in the B16F10 melanoma model upon co-administration of the anti-CD300c monoclonal antibody and (the) immunotherapy(ies) (groups D and T). Here, group D represents the combination of CL7 and the anti-PD-1 antibody, and group T represents the combination of CL7, the anti-PD-1 antibody, and the anti-CTLA-4 antibody.

### Experimental Example 10.3. Identification of Increased Cytotoxic T Cells Relative to Regulatory T Cells

In order to identify effects *in vivo,* on regulatory T cells, of combination of the anti-CD300c monoclonal antibody CL7 with an anti-PD-1 antibody and/or an anti-CTLA-4 antibody in a B16F10 melanoma model, a mouse tumor model was prepared as in Experimental Example 10.1 and injected with each test substance at the same concentration. The experimental groups were as follows: (i) a group administered with CL7, (ii) a group administered with the anti-PD-1 antibody, (iii) a group administered with CL7 and the anti-PD-1 antibody (group D), and (iv) a group administered with CL7, the anti-PD-1 antibody, and the anti-CTLA4 antibody (group T).

Tumor tissues were collected from 6 mice in each group. The collected tumor tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with CD16/32 antibody (from Invitrogen), and the cells were stained with a staining solution for checking cell viability, antibodies to the Treg marker proteins CD25 and Foxp3, a CD3+ antibody, and a CD8+ antibody. Then, the data were read with CytoFLEX flow cytometer and analyzed with FlowJo software.

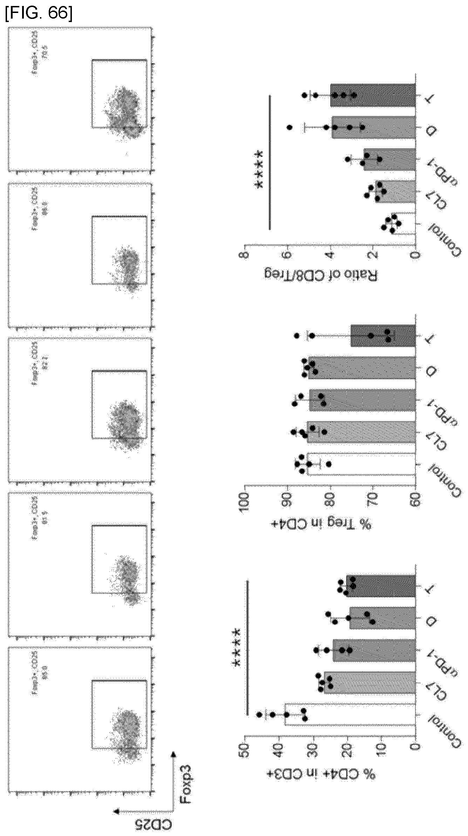

As a result, as illustrated in FIG. 66, it was identified that as in the CT26 carcinoma model (Experimental Example 6.5), the number of CD8+ T cells increased relative to regulatory T cells even in the B16F10 melanoma model upon co-administration of the anti-CD300c monoclonal antibody and (the) immunotherapy(ies) (groups D and T). Here, group D represents combination of CL7 and an anti-PD-1 antibody, and group T represents combination of CL7, the anti-PD-1 antibody, and the anti-CTLA-4 antibody.

### Experimental Example 10.4. Identification of Increased Tumor-Associated Macrophages (TAM)

In order to identify effects *in vivo,* on macrophages, of combination of the anti-CD300c monoclonal antibody CL7 with an anti-PD-1 antibody and/or an anti-CTLA-4 antibody in a B16F10 melanoma model, a mouse tumor model was prepared as in Experimental Example 10.1 and injected with each test substance at the same concentration.

Tumor tissues were collected from 6 mice in each group. The collected tumor tissue was incubated at 37°C for 1 hour in a mixed solution of collagenase D (20 mg/mL) and DNase I (2 mg/mL). Then, the resultant was filtered through a 70 µm cell strainer, red blood cells were subjected to lysis, and then the resultant was filtered again through a nylon mesh. Subsequently, the single cell suspension was blocked with a CD16/32 (Invitrogen) antibody, and the cells were stained with a staining solution for checking cell viability and antibodies against F4/80 and iNOS. Then, the data were read with CytoFLEX flow cytometer and analyzed with FlowJo software.

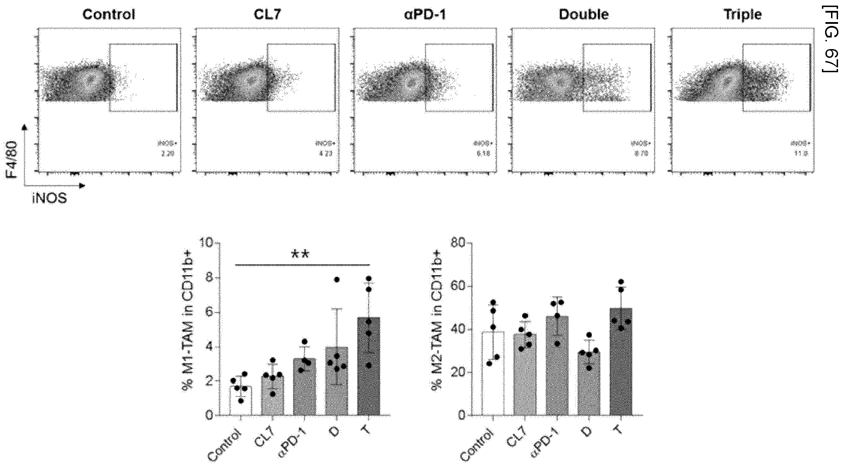

As a result, as illustrated in FIG. 67, it was identified that as in the CT26 carcinoma model (Experimental Example 6.1), the expression level of tumor-related M1-type macrophages increased even in the B16F10 melanoma model upon co-administration of the anti-CD300c monoclonal antibody and (the) immunotherapy(ies) (groups D and T). Here, group D represents combination of CL7 and an anti-PD-1 antibody, and group T represents combination of CL7, the anti-PD-1 antibody, and the anti-CTLA-4 antibody.

Taken together, the results illustrated in FIGS. 65, 66, and 67 have the following meaning. From the viewpoint that the anti-CD300c monoclonal antibody CL7 treats cancer in the B16F10 melanoma mouse model with the same mechanism as in the CT26 colorectal cancer mouse model (FIGS. 40, 41, and 42), it is predictable that co-administration of CL7 and the immunotherapy can exert the same effect in various carcinomas.

### Experimental Example 11. Identification of Complete Remission in Colorectal Cancer Mouse Model by Co-Administration

In order to identify anticancer effects *in vivo* caused by co-administration of the anti-CD300c monoclonal antibody CL7, and an anti-PD-1 antibody and/or an anti-CTLA-4 antibody, a colorectal cancer cell line (CT26) at 2×10⁵ cells was transplanted by subcutaneous injection into 8-week-old BALB/c mice to prepare a syngeneic mouse tumor model. Animal breeding and experiments were all conducted in a SPF facility. On day 11 (D11) after transplantation of the colon cancer cell line, the mice with a tumor size of 50 mm³ to 100 mm³ were administered with the anti-CD300c monoclonal antibody, and an anti-PD-1 antibody and the anti-CTLA-4 antibody, each of which was purchased from BioXcell, alone or in combination, and injected with phosphate buffered saline (PBS), whose amount is the same as CL7, as a control group. Specifically, on D11, D14 and D18, the mice were injected with each antibody alone or in combination by intraperitoneal injection (CL7: 25 mg/kg; anti-PD-1 antibody: 10 mg/kg; anti-CTLA-4 antibody: 4 mg/kg), and the tumor volume was measured.

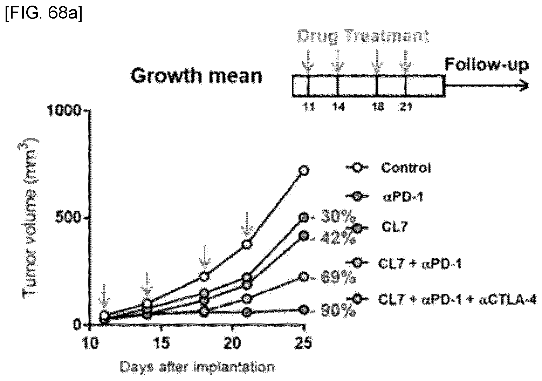

As a result, as illustrated in FIG. 68a , it was identified that although cancer growth was inhibited relative to the control group even in the experimental group administered with the anti-CD300c monoclonal antibody alone, cancer growth was further inhibited in a case where co-treatment with the anti-CD300c monoclonal antibody and an immunotherapy such as anti-PD-1 antibody and anti-CTLA-4 antibody was performed as compared with a case where treatment with the anti-CD300c monoclonal antibody alone was performed. In particular, triple co-administration of CL7+αPD-1+αCTLA-4 decreased the tumor size by 90%.

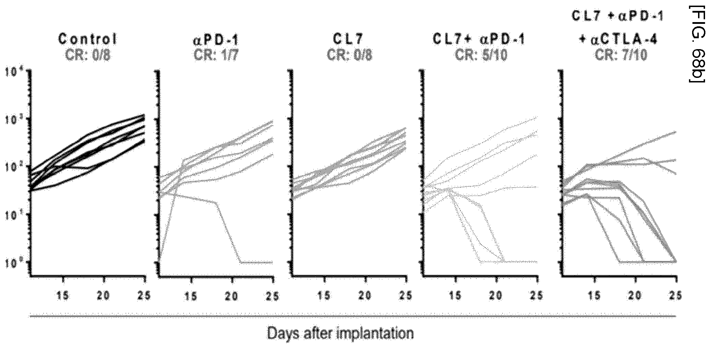

In addition, as illustrated in FIG. 68b, complete remission (CR) of 50% was achieved in a case where co-administration (the anti-CD300c monoclonal antibody and the anti-PD-1 antibody) was performed, and complete remission (CR) of 70% was achieved in a case where triple co-administration (the anti-CD300c monoclonal antibody+the anti-PD-1 antibody+the anti-CTLA-4 antibody) was performed, indicating that the co-administration resulted in an excellent anticancer effect.

### Experimental Example 12. Identification of Improved Long-Term Survival Caused by Co-Administration

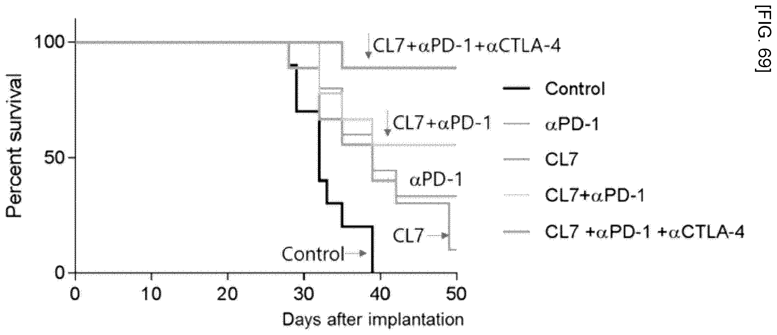

The long-term survival of the mice tested in Experimental Example 11 was checked. As a result, as illustrated in FIG. 69, it was identified that the long-term survival also increased in a case where co-treatment with the anti-CD300c monoclonal antibody and an immunotherapy such as anti-PD-1 antibody and anti-CTLA-4 antibody was performed as compared with a case where treatment with the anti-CD300c monoclonal antibody alone was performed.

### Experimental Example 13. Identification of Cancer Recurrence Prevention Effect Caused by Co-Administration

In order to identify cancer recurrence prevention effects *in vivo* caused by co-administration of the anti-CD300c monoclonal antibody CL7 and (an) immunotherapy(ies), a colorectal cancer cell line (CT26) at 2×10⁵ cells was transplanted by subcutaneous injection into 8-week-old BALB/c mice to prepare a syngeneic mouse tumor model, and then an experiment was conducted as described in Experimental Example 11 to obtain the mice that have undergone complete remission. The thus obtained mice were transplanted again with a colorectal cancer cell line (CT26) at 2×10⁵ cells, and observed for 30 days.

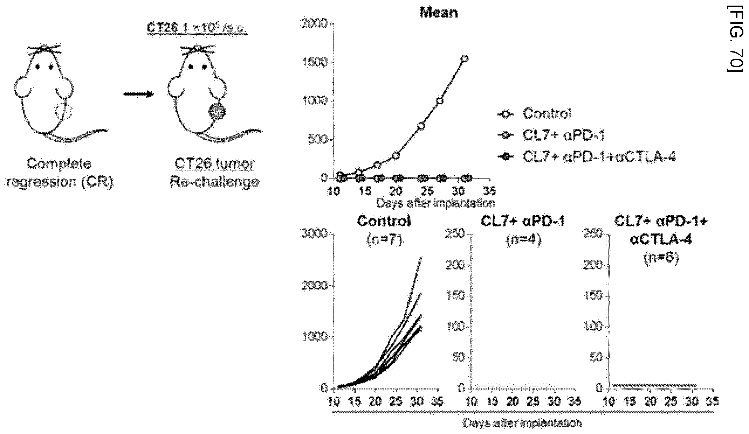

As a result, as illustrated in FIG. 70, it was identified that cancer recurrence or metastasis did not occur in the group in which complete remission was achieved by co-administration of the anti-CD300c monoclonal antibody and the immunotherapy. From these results, it was possible to predict that an individual, who had undergone complete remission of cancer by co-administration of the anti-CD300c monoclonal antibody and the anti-PD-1 antibody (CL7+αPD-1; Combi) or triple co-administration of the anti-CD300c monoclonal antibody, the anti-PD-1 antibody, and the anti-CTLA-4 antibody (CL7+αPD-1+αCTLA-4; Triple), had a systemic protective immune response through continuous immune memory, which could suppress recurrence or metastasis of cancer.

### Experimental Example 14. Identification of Immune Memory Effect Caused by Co-Administration

In order to analyze effector memory T cells in the mice that had undergone complete remission in Experimental Example 13, the mice were sacrificed and spleens were collected therefrom. Then, splenocytes were obtained therefrom and stained with antibodies (from Invitrogen) to CD44 and CD62L which are markers related to T cell activity. Then, the data were read with CytoFLEX flow cytometer and analyzed with FlowJo software.

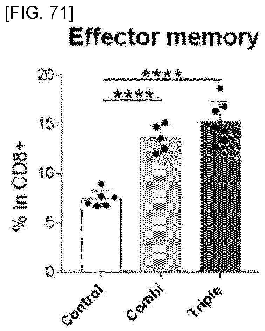

As a result, as illustrated in FIG. 71, it was identified that the effector memory T cells significantly increased in a case where co-treatment with the anti-CD300c monoclonal antibody and the immunotherapy was performed. This indicates that as predicted in Experimental Example 13, the mice having undergone complete remission have immune memory through increased effector memory T cells upon co-administration (Combi) of CL7 and the anti-PD-1 antibody or triple co-administration (Triple) of CL7, the anti-PD-1 antibody, and the anti-CTLA-4 antibody, and thus are able to suppress growth of additional cancer cells even if such cells occur.

### Example 4. Co-Administration of Anti-CD300c Monoclonal Antibody (CL10, SL18) and Immunotherapy

Each of the anti-CD300c monoclonal antibodies (CL10, SL18) prepared in Example 1 was used in combination with other immunotherapies, for example, IMFINZI^{®} that is an anti-PD-L1 antibody, and KEYTRUDA^{®} that is an anti-PD-1 antibody. The results were observed.

The manufacturers of the respective immunotherapies are as follows: IMFINZI^{®} (AstraZeneca); and KEYTRUDA@ (Merck Sharp & Dohme).

### Experimental Example 15. Identification of Increased Capacity for Causing Differentiation into M1 Macrophages

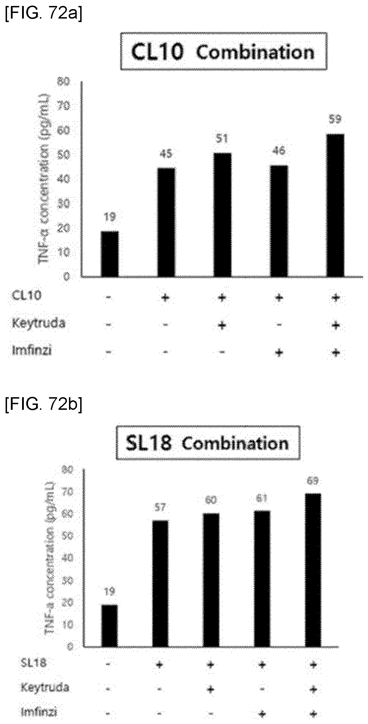

In order to identify whether anti-CD300c monoclonal antibody CL10 or SL18 can promote differentiation from macrophages to M1 macrophages, THP-1 cells at 1×10⁴ cells were dispensed onto a 96-well plate, and then treated with 10 µg/mL of CL10 or SL18. In addition, to identify effects caused by co-treatment with CL10 or SL18 and (an) immunotherapy(ies), the THP-1 cells were treated with the anti-CD300c monoclonal antibody in combination with IMFINZI^{®} and/or KEYTRUDA@ at 10 µg/mL. Subsequently, incubation was performed in a CO₂ incubator for 48 hours, and then the production level of TNF-α, which is a differentiation marker of M1 macrophages, was checked with an ELISA kit (Human TNF-α Quantikine kit, R&D Systems). The results are illustrated in FIG. 72a (CL10combination) and FIG. 72b (SL18 combination).

As illustrated in FIGS. 72a and 72b , it was identified that the expression level of TNF-α increased in a case where the THP-1 cells were co-treated with CL10 or SL18 and IMFINZI^{®} or KEYTRUDA@ as compared with a case where the THP-1 cells were treated with CL10 or SL18 alone. In particular, it was identified that the highest expression level of TNF-α was observed in a case where co-administration of CL10 or SL18 and the two antibodies, IMFINZI^{®} and KEYTRUDA@ was performed at once.

### Experimental Example 16. Identification of Cancer Cell Growth Inhibitory Effects

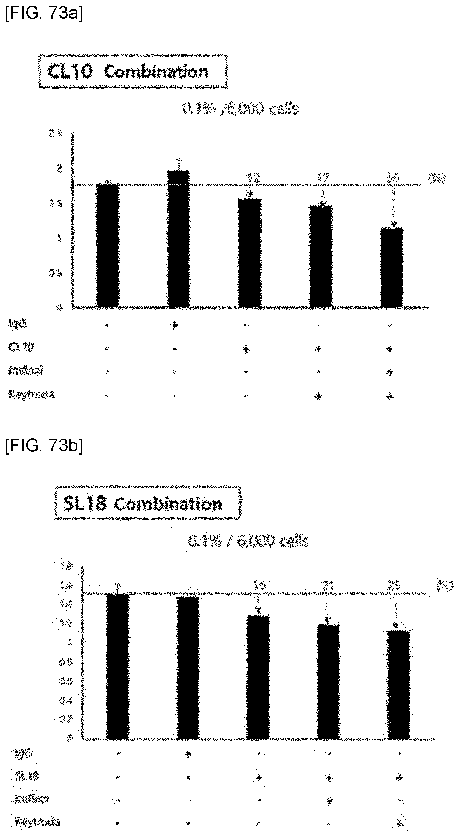

In order to identify cancer cell growth inhibitory effects caused by co-administration of the anti-CD300c monoclonal antibody CL10 or SL18 and an immunotherapy, cell growth inhibitory effects were compared using A549 (human lung cancer cell line) cells. Specifically, the cells were dispensed onto a 96-well plate at 2×10⁴cells under a condition of 0% FBS, and at 6×10³ cells under a condition of 0.1% FBS. Then, the cells were treated with CL10 or SL18 alone or co-treated with CL10 or SL18 and IMFINZI^{®} and/or KEYTRUDA@, each substance being used at a concentration of 10 µg/mL. Incubation was performed for 5 days. Subsequently, treatment with CCK-8 (DOJINDO) at 30 µL/well was performed. The absorbance was measured at OD 450 nm every hour while performing incubation in a CO₂ incubator for 4 hours. The results are illustrated in FIG. 73a (CL10 combination) and FIG. 73b (SL18 combination).

As illustrated in FIGS. 73a and 73b, it was identified that under a condition of 0.1% FBS, a further increased cancer cell proliferation inhibitory effect was observed in a case where the A549 cells were co-treated with CL10 or SL18 and IMFINZI^{®} or KEYTRUDA@ as compared with a case where the A549 cells were treated with CL10 or SL18 alone, and the highest cancer cell proliferation inhibitory effect was observed in a case where the A549 cells were co-treated with CL10 or SL18 and the two antibodies (IMFINZI^{®} and KEYTRUDA@) at once.

As can be seen from Experimental Example 15 and this Experimental Example, the remaining anti-CD300c monoclonal antibodies produced in Example 1, including CL10 and SL18, also exerted efficacy through the same mechanism of action as CL7, and had increased efficacy through combination with (an) immunotherapy(ies).

### Statistical Processing

For the results obtained through the experiments, one-way ANOVA followed by Bonferroni's post-hoc test was used to perform comparative analysis between the experimental groups. The difference between the groups was significant when the p-value was 0.05 or lower.

## Claims

1. An anti-CD300c monoclonal antibody or an antigen-binding fragment thereof, comprising:
(i) a heavy chain variable region that comprises CDR1 comprising an amino acid sequence selected from the group consisting of SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115, SEQ ID NO: 127, SEQ ID NO: 139, SEQ ID NO: 151, SEQ ID NO: 163, SEQ ID NO: 175, SEQ ID NO: 187, SEQ ID NO: 199, SEQ ID NO: 211, SEQ ID NO: 223, SEQ ID NO: 235, SEQ ID NO: 247, SEQ ID NO: 259, SEQ ID NO: 271, SEQ ID NO: 283, and SEQ ID NO: 295;
CDR2 comprising an amino acid sequence selected from the group consisting of SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116, SEQ ID NO: 128, SEQ ID NO: 140, SEQ ID NO: 152, SEQ ID NO: 164, SEQ ID NO: 176, SEQ ID NO: 188, SEQ ID NO: 200, SEQ ID NO: 212, SEQ ID NO: 224, SEQ ID NO: 236, SEQ ID NO: 248, SEQ ID NO: 260, SEQ ID NO: 272, SEQ ID NO: 284, and SEQ ID NO: 296; and
CDR3 comprising an amino acid sequence selected from the group consisting of SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117, SEQ ID NO: 129, SEQ ID NO: 141, SEQ ID NO: 153, SEQ ID NO: 165, SEQ ID NO: 177, SEQ ID NO: 189, SEQ ID NO: 201, SEQ ID NO: 213, SEQ ID NO: 225, SEQ ID NO: 237, SEQ ID NO: 249, SEQ ID NO: 261, SEQ ID NO: 273, SEQ ID NO: 285, and SEQ ID NO: 297; and
(ii) a light chain variable region that comprises CDR1 comprising an amino acid sequence selected from the group consisting of SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 142, SEQ ID NO: 154, SEQ ID NO: 166, SEQ ID NO: 178, SEQ ID NO: 190, SEQ ID NO: 202, SEQ ID NO: 214, SEQ ID NO: 226, SEQ ID NO: 238, SEQ ID NO: 250, SEQ ID NO: 262, SEQ ID NO: 274, SEQ ID NO: 286, and SEQ ID NO: 298;
CDR2 comprising an amino acid sequence selected from the group consisting of SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119, SEQ ID NO: 131, SEQ ID NO: 143, SEQ ID NO: 155, SEQ ID NO: 167, SEQ ID NO: 179, SEQ ID NO: 191, SEQ ID NO: 203, SEQ ID NO: 215, SEQ ID NO: 227, SEQ ID NO: 239, SEQ ID NO: 251, SEQ ID NO: 263, SEQ ID NO: 275, SEQ ID NO: 287, and SEQ ID NO: 299; and
CDR3 comprising an amino acid sequence selected from the group consisting of SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120, SEQ ID NO: 132, SEQ ID NO: 144, SEQ ID NO: 156, SEQ ID NO: 168, SEQ ID NO: 180, SEQ ID NO: 192, SEQ ID NO: 204, SEQ ID NO: 216, SEQ ID NO: 228, SEQ ID NO: 240, SEQ ID NO: 252, SEQ ID NO: 264, SEQ ID NO: 276, SEQ ID NO: 288, and SEQ ID NO: 300.

2. The anti-CD300c monoclonal antibody or an antigen-binding fragment thereof of claim 1,
wherein (i) the heavy chain variable region comprises:
CDR1 comprising an amino acid sequence selected from the group consisting of SEQ ID NO: 7, SEQ ID NO: 19, SEQ ID NO: 43, SEQ ID NO: 55, SEQ ID NO: 67, SEQ ID NO: 79, SEQ ID NO: 103, SEQ ID NO: 115, SEQ ID NO: 127, SEQ ID NO: 139, SEQ ID NO: 151, SEQ ID NO: 163, SEQ ID NO: 199, and SEQ ID NO: 211;
CDR2 comprising an amino acid sequence selected from the group consisting of SEQ ID NO: 8, SEQ ID NO: 20, SEQ ID NO: 44, SEQ ID NO: 56, SEQ ID NO: 68, SEQ ID NO: 80, SEQ ID NO: 104, SEQ ID NO: 116, SEQ ID NO: 128, SEQ ID NO: 140, SEQ ID NO: 152, SEQ ID NO: 164, SEQ ID NO: 200, and SEQ ID NO: 212; and
CDR3 comprising an amino acid sequence selected from the group consisting of SEQ ID NO: 9, SEQ ID NO: 21, SEQ ID NO: 45, SEQ ID NO: 57, SEQ ID NO: 69, SEQ ID NO: 81, SEQ ID NO: 105, SEQ ID NO: 117, SEQ ID NO: 129, SEQ ID NO: 141, SEQ ID NO: 153, SEQ ID NO: 165, SEQ ID NO: 201, and SEQ ID NO: 213; and
(ii) the light chain variable region comprises:
CDR1 comprising an amino acid sequence selected from the group consisting of SEQ ID NO: 10, SEQ ID NO: 22, SEQ ID NO: 46, SEQ ID NO: 58, SEQ ID NO: 70, SEQ ID NO: 82, SEQ ID NO: 106, SEQ ID NO: 118, SEQ ID NO: 130, SEQ ID NO: 142, SEQ ID NO: 154, SEQ ID NO: 166, SEQ ID NO: 202, and SEQ ID NO: 214;
CDR2 comprising an amino acid sequence selected from the group consisting of SEQ ID NO: 11, SEQ ID NO: 23, SEQ ID NO: 47, SEQ ID NO: 59, SEQ ID NO: 71, SEQ ID NO: 83, SEQ ID NO: 107, SEQ ID NO: 119, SEQ ID NO: 131, SEQ ID NO: 143, SEQ ID NO: 155, SEQ ID NO: 167, SEQ ID NO: 203, and SEQ ID NO: 215; and
CDR3 comprising an amino acid sequence selected from the group consisting of SEQ ID NO: 12, SEQ ID NO: 24, SEQ ID NO: 48, SEQ ID NO: 60, SEQ ID NO: 72, SEQ ID NO: 84, SEQ ID NO: 108, SEQ ID NO: 120, SEQ ID NO: 132, SEQ ID NO: 144, SEQ ID NO: 156, SEQ ID NO: 168, SEQ ID NO: 204, and SEQ ID NO: 216.

3. The anti-CD300c monoclonal antibody or an antigen-binding fragment thereof of claim 1,
wherein (i) the heavy chain variable region comprises:
CDR1 comprising an amino acid sequence selected from the group consisting of SEQ ID NO: 43, SEQ ID NO: 79, SEQ ID NO: 115, and SEQ ID NO: 211;
CDR2 comprising an amino acid sequence selected from the group consisting of SEQ ID NO: 44, SEQ ID NO: 80, SEQ ID NO: 116, and SEQ ID NO: 212; and
CDR3 comprising an amino acid sequence selected from the group consisting of SEQ ID NO: 45, SEQ ID NO: 81, SEQ ID NO: 117, and SEQ ID NO: 213; and
(ii) the light chain variable region comprises:
CDR1 comprising an amino acid sequence selected from the group consisting of SEQ ID NO: 46, SEQ ID NO: 82, SEQ ID NO: 118, and SEQ ID NO: 214;
CDR2 comprising an amino acid sequence selected from the group consisting of SEQ ID NO: 47, SEQ ID NO: 83, SEQ ID NO: 119, and SEQ ID NO: 215; and
CDR3 comprising an amino acid sequence selected from the group consisting of SEQ ID NO: 48, SEQ ID NO: 84, SEQ ID NO: 120, and SEQ ID NO: 216.

4. The anti-CD300c monoclonal antibody or an antigen-binding fragment thereof of claim 1, wherein:
the heavy chain variable region comprises CDR1 comprising the amino acid sequence represented by SEQ ID NO: 43, CDR2 comprising the amino acid sequence represented by SEQ ID NO: 44, and CDR3 comprising the amino acid sequence represented by SEQ ID NO: 45; and
the light chain variable region comprises CDR1 comprising the amino acid sequence represented by SEQ ID NO: 46, CDR2 comprising the amino acid sequence represented by SEQ ID NO: 47, and CDR3 comprising the amino acid sequence represented by SEQ ID NO: 48.

5. The anti-CD300c monoclonal antibody or an antigen-binding fragment thereof of claim 1, wherein:
the heavy chain variable region comprises CDR1 comprising the amino acid sequence represented by SEQ ID NO: 79, CDR2 comprising the amino acid sequence represented by SEQ ID NO: 80, and CDR3 comprising the amino acid sequence represented by SEQ ID NO: 81, and
the light chain variable region comprises CDR1 comprising the amino acid sequence represented by SEQ ID NO: 82, CDR2 comprising the amino acid sequence represented by SEQ ID NO: 83, and CDR3 comprising the amino acid sequence represented by SEQ ID NO: 84.

6. The anti-CD300c monoclonal antibody or an antigen-binding fragment thereof of claim 1, wherein:
the heavy chain variable region comprises CDR1 comprising the amino acid sequence represented by SEQ ID NO: 115, CDR2 comprising the amino acid sequence represented by SEQ ID NO: 116, and CDR3 comprising the amino acid sequence represented by SEQ ID NO: 117, and
the light chain variable region comprises CDR1 comprising the amino acid sequence represented by SEQ ID NO: 118, CDR2 comprising the amino acid sequence represented by SEQ ID NO: 119, and CDR3 comprising the amino acid sequence represented by SEQ ID NO: 120.

7. The anti-CD300c monoclonal antibody or an antigen-binding fragment thereof of claim 1, wherein:
the heavy chain variable region comprises CDR1 comprising the amino acid sequence represented by SEQ ID NO: 211, CDR2 comprising the amino acid sequence represented by SEQ ID NO: 212, and CDR3 comprising the amino acid sequence represented by SEQ ID NO: 213, and
the light chain variable region comprises CDR1 comprising the amino acid sequence represented by SEQ ID NO: 214, CDR2 comprising the amino acid sequence represented by SEQ ID NO: 215, and CDR3 comprising the amino acid sequence represented by SEQ ID NO: 216.

8. The anti-CD300c monoclonal antibody or an antigen-binding fragment thereof of claim 1, wherein:
the heavy chain variable region comprises an amino acid sequence selected from the group consisting of SEQ ID Nos: 303, 307, 311, 315, 319, 323, 327, 331, 335, 339, 343, 347, 351, 355, 359, 363, 367, 371, 375, 379, 383, 387, 391, 395, and 399; and
the light chain variable region comprises an amino acid sequence selected from the group consisting of SEQ ID Nos: 304, 308, 312, 316, 320, 324, 328, 332, 336, 340, 344, 348, 352, 356, 360, 364, 368, 372, 376, 380, 384, 388, 392, 396, and 400.

9. The anti-CD300c monoclonal antibody or an antigen-binding fragment thereof of claim 1, wherein:
the heavy chain variable region comprises an amino acid sequence selected from the group consisting of SEQ ID Nos: 315, 327, 339, and 371; and
the light chain variable region comprises an amino acid sequence selected from the group consisting of SEQ ID Nos: 316, 328, 340, and 372.

10. The anti-CD300c monoclonal antibody or an antigen-binding fragment thereof of claim 1, wherein the anti-CD300c monoclonal antibody or an antigen-binding fragment thereof has inter-species cross-reactivity.

11. The anti-CD300c monoclonal antibody or an antigen-binding fragment thereof of claim 10, wherein the anti-CD300c monoclonal antibody or an antigen-binding fragment thereof has cross-reactivity to both a human and a mouse CD300c antigens.

12. An anti-CD300c monoclonal antibody or an antigen-binding fragment thereof, comprising:
a heavy chain variable region that comprises CDR1 to CDR3 comprising amino acid sequences, respectively, represented by Formulas (1) to (3), and
a light chain variable region that comprises CDR1 to CDR3 comprising amino acid sequences, respectively, represented by Formulas (4) to (6),
FTFX1X2X3X4MX5WVR (1) in the above formula,
X1 = G or S
X2 = S, R, or D
X3 = N or Y
X4 = Y, A, G, or H
X5 = S or H
X1ISX2SGX3X4TYYAX5 (2) in the above formula,
X1 = T or A
X2 = G or S
X3 = T or G
X4 = S or Y
X5 = D or E
YCAX1X2X3X4X5X6X7X8X9W (3) in the above formula,
X1 = R or S
X2 = G or S
X3 = M, S, Y, or I
X4 = W, Q, G, or R
X5 = G or L
X6 = M, I, or P
X7 = D, F, or L
X8 = V or D
X9 = I, Y, or not present
CX1X2X3X4X5X6X7X8X9X10X11VX12W (4) in the above formula,
X1 = T or S
X2 = G or R
X3 = K, N, or S
X4 = H, N, or S
X5 = R, I, or G
X6 = H, G, or I
X7 = T, I, or S
X8 = R, A, K, or not present
X9 = R, S, G, or not present
X10 = N or not present
X11 = Y or not present
X12 = N, H, or Q
X1X2X3X4RPSGVX5 (5) in the above formula,
X1 = L, S, R, or E
X2 = D, K, or N
X3 = S or N
X4 = E, N, Q, or K
X5 = P or R
YCX1X2X3X4X5X6X7X8X9X10VF (6) in the above formula,
X1 = Q, A, or S
X2 = S or A
X3 = Y or W
X4 = D or A
X5 = S, D, or G
X6 = S, N, or T
X7 = S, L, N, or K
X8 = V, S, N, or G
X9 = G, L, V, or not present
X10 = P or not present.

13. A pharmaceutical composition for preventing or treating cancer comprising, as an active ingredient, the anti-CD300c monoclonal antibody or an antigen-binding fragment thereof according to any one of claims 1 to 12.

14. The pharmaceutical composition of claim 13, wherein the cancer comprises any one or more selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer.

15. The pharmaceutical composition of claim 13, wherein the cancer is a solid cancer.

16. The pharmaceutical composition of claim 13, wherein the cancer comprises one or more selected from the group consisting of colorectal cancer, lung cancer, melanoma, and breast cancer.

17. The pharmaceutical composition of claim 13, wherein the pharmaceutical composition inhibits proliferation, survival, metastasis, recurrence, or therapy resistance of cancer.

18. The pharmaceutical composition of claim 13, further comprising one or more cancer immunotherapy.

19. The pharmaceutical composition of claim 18, wherein the cancer immunotherapy comprises any one or more selected from the group consisting of anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT.

20. The pharmaceutical composition of claim 18, wherein the cancer immunotherapy comprises one or more selected from the group consisting of anti-PD-1, anti-PD-L1, anti-CTLA-4, and anti-CD47 antibodies.

21. The pharmaceutical composition of claim 18, wherein the cancer immunotherapy comprises one or more selected from the group consisting of durvalumab, pembrolizumab, nivolumab, αCD47, and ipilimumab.

22. The pharmaceutical composition of claim 18, wherein the anti-CD300c antibody or an antigen-binding fragment thereof and the cancer immunotherapy are respectively formulated and administered simultaneously or separately in a sequential manner.

23. A method for preventing or treating cancer comprising administering, to a subject in need of prevention or treatment of cancer, the anti-CD300c monoclonal antibody or an antigen-binding fragment thereof according to any one of claims 1 to 12.

24. The method of claim 23, further comprising administering one or more cancer immunotherapies.

25. The method of claim 23, further comprising determining an expression level of a CD300c protein based on the subject's biological sample or data, prior to the administration of the anti-CD300c antibody or an antigen-binding fragment thereof.

26. The method of claim 25, further comprising determining therapeutic responsiveness of the anti-CD300c antibody or an antigen-binding fragment thereof based on the determined expression level of a marker.

27. The method of claim 23, further comprising determining an expression level of one or more markers selected from the following markers, using a biological sample or data from the subject to which the anti-CD300c monoclonal antibody or an antigen-binding fragment thereof has been administered:
Bst2, Cd40, Cd70, Cd86, Ccl8, Xcl1, Ccr7, Cd80, Cd206, Msr1, Arg1, Vegfa, Pdgfrb, Col4a1, Hif1a, Vcam1, Icam1, Gzma, Gzmb, Icos, Cd69, Ifng, Tnf, Cd1d1, Cd1d2, Cd38, Cxcr6, Xcr1, Tbx21, Stat1, Stat4, Cxcr3, IL-12b, IL-4, IL-6, IL-13, PD-1, PD-L1, CTLA-4, Lag3, Tim3, Icos, Ox40, Gitr, Hvem, CD27, CD28, Cma1, Timd4, Bcl6, Cxcl5, and Ccl21a.

28. The method of claim 27, further comprising selecting one or more other cancer immunotherapies to be used in combination with the anti-CD300c antibody or an antigen-binding fragment thereof, based on the determined expression level of the marker.

29. The method of claim 28, wherein the marker comprises one or more selected from the group consisting of PD-1, PD-L1, CTLA-4, Lag3, Tim3, Icos, Ox40, Gitr, Hvem, CD27, and CD28.

30. The method of claim 27, wherein the marker comprises one or more selected from the group consisting of vegfa, pdgfrb, Col4a1, Hif1a, Bst2, CCL8, Xcl1, CCR7, CD80, Tbx21, Stat1, Stat4, Ifng, Cxcr3, IL-6, Gzma, Icos, Cd69, Cd1d1, Cd38, Cxcr6, Ox40, Gitr, CD27, and CD28.

31. The method of claim 30, further comprising determining that the therapeutic responsiveness of the anti-CD300c antibody or an antigen-binding fragment thereof is good or excellent in a case where an expression level of one or more markers selected from the group consisting of vegfa, pdgfrb, Col4a1, Hif1a, and IL-6 is statistically significantly decreased as compared with a subject having not received the anti-CD300c antibody or an antigen-binding fragment thereof.

32. The method of claim 30, further comprising determining that the therapeutic responsiveness of the anti-CD300c monoclonal antibody or an antigen-binding fragment thereof is good or excellent in a case where an expression level of one or more markers selected from the group consisting of Bst2, CCL8, Xcl1, CCR7, CD80, Tbx21, Stat1, Stat4, Ifng, Cxcr3, Gzma, Icos, Cd69, Cd1d1, Cd38, Cxcr6, Ox40, Gitr, CD27, and CD28 is statistically significantly increased as compared with a subject having not received the anti-CD300c antibody or an antigen-binding fragment thereof.

33. A kit for preventing or treating cancer, comprising:
a composition that comprises the anti-CD300c monoclonal antibody or an antigen-binding fragment thereof according to any one of claims 1 to 12; and
an instruction that directs use of the antibody or an antigen-binding fragment thereof.

34. The kit of claim 33, wherein the instruction comprises an instruction that directs combined use of the antibody or an antigen-binding fragment thereof and one or more additional cancer therapies.

35. The kit of claim 33, wherein the instruction comprises an instruction that directs measurement of an expression level of a CD300c protein using a biological sample or data obtained from the subject prior to administration of the antibody or an antigen-binding fragment thereof.

36. A method for providing information for prevention or treatment of cancer, comprising determining an expression level of a CD300c protein based on a biological sample or data obtained from a subject in need of prevention or treatment of cancer.

37. The method of claim 36, wherein the information for prevention or treatment of cancer comprises information on any one or more of therapeutic responsiveness of a therapeutic agent associated with a CD300c protein (for example, an anti-CD300c antibody or an antigen-binding fragment thereof), selection of a therapeutic agent, selection of a subject to be treated, prognosis of a subject, and survival of a subject.

38. A kit for providing information for prevention or treatment of cancer, comprising a material for measuring an expression level of a CD300c protein using a biological sample or data obtained from a subject in need of prevention or treatment of cancer.

39. An isolated nucleic acid molecule, encoding the anti-CD300c monoclonal antibody or an antigen-binding fragment thereof according to any one of claims 1 to 12.

40. An expression vector, comprising the nucleic acid molecule according to claim 39.

41. A host cell, comprising the nucleic acid molecule according to claim 39.

42. A method for producing an anti-CD300c monoclonal antibody or an antigen-binding fragment thereof, comprising culturing the host cell of claim 41.
